(19) Europäisches Patentamt
European Patent Office
Office européen des brevets

(11) Publication number: 0 349 204
A2

# (12) EUROPEAN PATENT APPLICATION

(21) Application number: 89306341.2

(22) Date of filing: 23.06.89

(51) Int. Cl.[4]: B01J 14/00 , C12P 1/00

A request for correction in the description and the figures has been filed pursuant to Rule 88 EPC. A decision on the request will be taken during the proceedings before the Examining Division (Guidelines for Examination in the EPO, A-V, 2.2).

(30) Priority: 28.06.88 US 212606

(43) Date of publication of application:
03.01.90 Bulletin 90/01

(84) Designated Contracting States:
AT BE CH DE ES FR GB GR IT LI LU NL SE

(71) Applicant: BEND RESEARCH, INC.
64550 Research Road
Bend Oregon, 97701(US)

(72) Inventor: van Eikeren, Paul
1922 N.W. Seventh Street
Bend Oregon 97701(US)

(74) Representative: Skerrett, John Norton Haigh et al
H.N. & W.S. Skerrett Charles House 148/9
Great Charles Street
Birmingham B3 3HT(GB)

## (54) Use and regeneration of reagents using coupled reactions and permselective barriers.

(57) A process is disclosed for the use and regeneration of valuable reagents, including coenzymes, using permselective barrier-assisted coupled reactions, most involving redox couples.

EP 0 349 204 A2

# USE AND REGENERATION OF REAGENTS USING COUPLED REACTIONS AND PERMSELECTIVE BARRIERS

## BACKGROUND OF THE INVENTION

This invention relates to the use and regeneration of valuable chemical reagents by a novel permselective barrier-assisted process using coupled reactions.

Mediated transport of electrons across membranes is known. See, for example, the article by Nambu, et al. in 23 J. Polym Sci.: Polym. Ltrs. Ed. 409 (1985), which discloses electron transport across a polypeptide/viologen membrane, the transport being mediated by a viologen structure covalently bound to the membrane polymer, with redox reactions taking place at the two interfaces of the membrane with ion-containing solutions. See also the report of Danesi in 29 J. Memb. Sci. 287 (1986), which discloses electron transport across a redox supported liquid membrane comprising a microporous polypropylene film containing solution of hydroquinone in the pores of the membrane, the hydroquinone functioning as an electron carrier, redox reactions again taking place at the interfaces of the membrane with reactant-containing solutions.

There has been considerable attention focused in recent years on regeneration of the naturally-occuring electron carriers comprising the nicotimade cofactors nicotinamide adenine dinucleotide (NAD+ representing the oxidized form and NADH the reduced form) and nicotinamide adenine dinucleotide phosphate (NADP+ representing the oxidized form and NADPH the reduced form). (For ease of reference, the symbols NAD(P)+ and NAD(P)H shall be used to respectively designate the oxidized and reduced forms of both the phosphorylated and non-phosphorylated cofactors). A good summary of such work is found in the extensive review by Chenault, et al. in 14 Appl. Biochem, Biotech. 147 (1987), which reviews the significant work in the areas of synthesis of those expensive cofactors, methods of regenerating them in situ, and process considerations relating to their use in synthesis of other biochemicals. Wichmann, et al., in 23 Biotech. Bioeng. 2789 (1981) disclose the regeneration of nicotinamide coenzymes in a reactor comprising a reactant stream separated from the product stream by an ultrafiltration (UF) membrane which, under pressure-driven convective flow, retains the much larger catalyst and coenzyme molecules while allowing passage of the smaller product molecules. The reactant stream contains reactant, catalyst, coenzyme and sacrificial reducing agent all mixed together.

Similar systems are disclosed in papers by Davies, et al. in 370 Biochim. Biophys. Acta 329 (1974), by Coughlin, et al. in 17 Biotech. Bioeng. 515 (1975), and by Fink, et al. in 17 Biotech. Bioeng. 1029 (1975).

In the Fink, et al. paper, nicotinamide coenzymes are shown as permeating the membrane and so must be recovered from the product stream and separately regenerated and recycled to the feed stream. The Coughlin, et al. reactor uses an electrolytic cell in conjunction with the UF membrane to electrochemically regenerate cofactor NAD. Electrochemical regeneration of nicotinamide cofactors is reviewed in a short article by Allen, et al. in 3 Trends in Biotechnology 145 (1985).

All of the foregoing regeneration schemes suffer from one or more of the drawbacks of having too narrow an application, being unduly complex in terms of both method and apparatus, having unfavorable thermodynamics, being limited to the regeneration of NAD alone, or producing byproducts that unduly complicate process engineering. What is needed therefore is a reagent regeneration method that is simple in design, inexpensive, reliable and capable of producing quantitative results with a minimum amount of work-up, product separation, and engineering. These needs and others that will become apparent are provided by the present invention, which is summarized and described in detail below.

## SUMMARY OF THE INVENTION

The present invention provides a novel method for using and regenerating valuable chemically highly reactive group transfer reagents. The basic method utilizes a permselective barrier to segregate reactions that are advantageously coupled. More specifically, the present invention indirectly couples strong chemical reactions with weak ones, the strong reactions driving the weak ones, to permit discrete product formation by using and regenerating small amounts of highly valuable reagents.

In its simplest form, the present invention may be embodied in a reactor bifurcated into two reaction centers by a permselective membrane that provides a barrier to segregate chemical species and reactions

that are desireably segregated, the membrane at the same time being selectively permeable to certain of the involved chemical species so as to permit beneficial coupling of chemical reactions on either side of the membrane barrier. By carrying out simultaneous chemical reactions on both sides of the membrane barrier, such reactions being susceptible of being coupled by the permeation through the membrane of certain species, the chemical potentials of the permeating species may be manipulated so as to drive the separated reactions under dramatically different reaction conditions without any mixing of the segregated reactants and without the need for product separation processing steps.

Thus, although the term "bifurcated" is used frequently herein with reference to the present invention, it is to be understood that the term is used principally for convenience of reference to and description of the simplest form of the invention, and that the invention in its broadest scope is intended to include any number of permselective barriers that segregate and indirectly couple chemical reactions in the manner described herein.

In general, there are three broad aspects to the present invention, having regard to categories of the reaction schemes involved: (1) processes for the use and regeneration of the chemically active form of reagents useful in group transfer reactions; (2) processes for the use and regeneration of the coenzymes NAD(P)H and $NAD(P)^+$ using strong sacrificial redox agents and membrane-permeating redox couples; and (3) processes similar to those in category (2) but involving trapping one member of a redox couple.

## BRIEF DESCRIPTION OF THE DRAWINGS

FIG. 1 is a schematic depiction of the group transfer reaction aspect of the invention.

FIG. 2 is a schematic depiction of the scheme for the use and regeneration of the coenzymes NAD-(P)H using a sacrificial reducing agent.

FIG. 3 is a schematic depiction of the scheme for the use and regeneration of the coenzymes $NAD(P)^+$ using a sacrificial oxidizing agent.

FIG. 4 is a schematic depiction of the scheme for the use and regeneration of the coenzymes NAD-(P)H involving trapping the membrane-permeating species.

FIG. 5 is a schematic depiction of the scheme for the use and regeneration of the coenzymes $NAD(P)^+$ also involving trapping the membrane-permeating species.

FIG. 6 is a perspective view of exemplary apparatus for use in the present invention.

FIGS. 7-12 are schematic depictions of exemplary FIG. 1-type group transfer reaction use and regeneration schemes.

FIGS. 13-17 are schematic depictions of exemplary FIG. 2-type NAD(P)H use and regeneration schemes.

FIGS. 18-20 are schematic depictions of exemplary FIG. 3-type $NAD(P)^+$ use and regeneration schemes.

FIGS. 21-24 are schematic depictions of exemplary embodiments of FIG. 4-type NAD(P)H use and regeneration schemes.

FIGS. 25-27 are schematic depictions of exemplary embodiments of FIG. 5-type $NAD(P)^+$ use and regeneration schemes.

FIG. 28 is a schematic depiction of the use of multiple permselective barriers in a pervaporation mode of the process of the present invention.

FIGS. 29-36 are graphs showing the results of various use and regeneration schemes of the present invention.

## DETAILED DESCRIPTION OF THE INVENTION

According to the present invention there are provided a number of reaction schemes useful for the use and regeneration of a wide variety of reagents, including the coenzymes $NAD(P)^+$ and NAD(P)H. The five basic schemes depicted in FIGS. 1-5 will first be addressed, with specific embodiments to follow.

Referring to FIG. 1, there is shown a reactor bifurcated into a first side and a second by a permselective barrier that is selectively permeable to both the chemically active and the chemically inactive forms of a reagent. As seen there, the chemically active form of the reagent is allowed to react on the first side of the barrier with a first reactant in a group transfer reaction, whereby a chemically active group is transferred from the chemically active form of the reagent to the first reactant so as to obtain a desired first

product and the chemically inactive form of the reagent. This first group transfer reaction may be catalyzed or uncatalyzed. The effect of the first group transfer reaction is to raise the chemical potential of the inactive form of the reagent on the first side of the barrier and to lower the chemical potential of the active form of the reagent on that same side. "Chemical potential" is used in its conventional physical chemistry sense that relates the potential chemical activity of a chemical moiety to its relative concentration by the formula

$\mu = \mu + RT1nC$

where $\mu$ is the chemical potential of the solute, $\mu$ is the chemical potential of the solute in the standard state of 1M concentration, and C is a dimensionless value corresponding to the concentration of the solute relative to its concentration in the standard state.

Simultaneously with the aforementioned reaction on the first side of the barrier of FIG. 1, the chemically inactive form of the same reagant involved in the first group transfer reaction reacts with a second reactant in a second group transfer reaction, catalyzed or uncatalyzed, on the second side of the barrier to form a second product and to convert the chemically inactive form of the same reagent to its chemically active form by transfer of the same chemically active group from the second reactant. This group transfer reaction on the second side raises the chemical potential of the chemically active form of the reagent on the second side and at the same time lowers the chemical potential of the chemically inactive form of the reagent. In this manner, a predetermined differential in chemical potentials of the chemically active and inactive forms of the reagent on either side of the permselective barrier may be set up, which differential in effect provides a driving force for the two forms of the reagent to permeate or diffuse through the permselective barrier in the directions noted in FIG. 1.

Thus, the two reactions,

1st react + active reag→1st prod + inactive reag

2nd react + inactive reag→2nd prod + active reag

though segregated from each other on the first side and second side of the permselective barrier, are effectively coupled by utilizing the chemically active and inactive forms of the reagent as a link. It will be appreciated that the net effect of the reaction scheme shown in FIG. 1 is to regenerate the chemically active form of the reagent while simultaneously using the chemically active form of that reagent to make a desired product (the "first product" in FIG. 1) and to use its chemically inactive form to make another desired product (the "second product" in FIG. 1).

Referring to FIG. 2, there is shown a reactor bifurcated into a first side and a second side by a permselective barrier that is selectively permeable to both the oxidized and reduced forms of a chemical arbitrarily designated as "chemical A." As seen in the schematic, the coenzymes NAD(P)H may be oxidized on the first side of the barrier by the oxidized form of a reactant arbitrarily designated as "chemical B" in the presence of hydrogen ions and a first catalyst so as to form $NAD(P)^+$ and a desired product comprising the reduced form of chemical B. At the same time on the first side of the barrier, the reduced form of chemical A is used to reduce $NAD(P)^+$ in the presence of a second catalyst to form NAD(P)H and the oxidized form of chemical A, which has the effect of raising the chemical potential of the oxidized form of chemical A on the first side of the barrier and lowering the chemical potential of the reduced form of chemical A on the same side.

Simultaneously with the aforementioned first side reactions of FIG. 2, a sacrificial reducing agent is used to reduce the oxidized form of chemical A on the second side of the barrier, in the presence of an optional third catalyst so as to form the reduced form of chemical A, which has the effect of raising the chemical potential of the reduced form of chemical A on the second side and lowering the chemical potential of the oxidized form of chemical A on that same side. The sacrificial reducing agent in this second side reaction is a stronger reducing agent than the reduced form of chemical A so that the thermodynamics of the reaction favor the formation of reduced chemical A.

In this fashion, a predetermined differential in chemical potentials of the oxidized and reduced forms of chemical A on either side of the permselective barrier may be set up, which differential in effect provides a driving force for the two forms of chemical A to permeate or diffuse through the permselective barrier in the directions noted in FIG. 2. In the scheme depicted in FIG. 2, chemical A comprises a redox couple and is a substrate for the second catalyst, and the thermodynamics of the process strongly favor the formation of the reduced form of chemical B and the oxidized form of the sacrificial reducing agent over the oxidized form of chemical B and the reduced form of the sacrificial reducing agent.

Thus, the reactions

$$NAD(P)H + H + \text{ox chem B} \xrightarrow{\text{catalyst 1}} NAD(P)^{+} + \text{red chem B}$$

$$\text{red chem A} + NAD(P)^{+} \xrightarrow{\text{catalyst 2}} NAD(P)H + H^{+} + \text{ox chem A}$$

are coupled with each other and coupled to and segregated from the reaction

ox chem A + sac red agent→red chem A

the coupling between the reactions on either side of the barrier taking place by the link comprising the barrier-permeating oxidized and reduced forms of chemical A. It is thus apparent that the strong second side reaction thermodynamically drives the weaker first side coupled reactions. It may also be seen that the net effect of the reaction scheme shown in FIG. 2 is to regenerate the coenzymes NAD(P)H while simultaneously using them to produce at least one desired product comprising the reduced form of chemical B (a second potentially useful product comprising the oxidized sacrificial reducing agent is also formed).

It should be noted that the barrier, while permitting the coupling of the first and second side reactions, prevents the second side strong sacrificial redox pair of reactants from otherwise indiscriminate reaction with the first side reactants and catalysts. Another advantage of the present invention is that, by preventing indiscriminate reactions that would otherwise take place from direct contact, the reactions on the first side can be carried out at optimal conditions without regard to incompatabilities to reaction conditions present on the second side. Further, by removing oxidized chemical A from the first side to the second side, its concentration in the region of the two catalyzed first side reactions is reduced, which has the effect of minimizing its inhibition of the catalytic action of such catalysts and so enhance the overall efficiency of those reactions. Finally, the barrier segregates spent sacrificial reducing agent (on the second side) from the desired end product reduced chemical B (on the first side), which eliminates the need for product separation processing steps.

Referring to FIG. 3, the redox converse of the scheme shown in FIG. 2 is depicted. Specifically, there is shown a reactor bifurcated into a first side and a second side by a permselective barrier that is selectively permeable to both the oxidized and reduced forms of a chemical again arbitrarily designated as "chemical A." As seen in FIG. 3, the coenzymes $NAD(P)^{+}$ may be reduced on the first side of the barrier by the reduced form of a second chemical arbitrarily designated as "chemical B" in the presence of a first catalyst so as to form NAD(P)H and a desired product comprising the oxidized form of chemical B. At the same time on the first side of the barrier, the oxidized form of chemical A is used to oxidize NAD(P)H in the presence of hydrogen ions and a second catalyst to form $NAD(P)^{+}$ and the reduced form of chemical A, thereby raising the chemical potential of the reduced form of chemical A on the first side of the barrier and lowering the chemical potential of the oxidized form of chemical A on the same side.

Simultaneously with such first side reactions of FIG. 3, a sacrificial oxidizing agent is used to oxidize the reduced form of chemical A on the second side of the barrier, in the presence of an optional third catalyst so as to form the oxidized form of chemical A, thereby raising the chemical potential of the oxidized form of chemical A on the second side and lowering the chemical potential of the reduced form of chemical A on that same side. The sacrificial oxidizing agent in this second side reaction is a stronger oxidizing agent than the oxidized form of chemical A so that the thermodynamics of the reaction favor the formation of oxidized chemical A.

In this manner, a predetermined differential in chemical potentials of the oxidized and reduced forms of chemical A on either side of the permselective barrier may be set up, which differential effectively provides a driving force for the two forms of chemical A to permeate the permselective barrier in the directions noted in FIG. 3. In the scheme depicted, chemical A comprises a redox couple and is a substrate for the second catalyst, and the thermodynamics of the process strongly favor the formation of the oxidized form of chemical B and the reduced form of the sacrificial oxidizing agent over the reduced form of chemical B and the oxidized form of the sacrificial oxidizing agent.

Thus, the reactions,

$$NAD(P)^+ + \text{red chem B} \xrightarrow{\text{catalyst 1}} NAD(P)H + H^+ + \text{ox chem B}$$

$$\text{ox chem A} + NAD(P)H + H^+ \xrightarrow[\text{catalyst 2}]{} NAD(P)^+ + \text{red chem A}$$

are coupled with each other and coupled to and segregated from the reaction
red chem A + sac ox agent→ox chem A
the coupling of the first side reactions and the second side reaction taking place by the link comprising the barrier-permeating oxidized and reduced forms of chemical A. Thus, the strong second side reaction involving a sacrificial oxidizing agent thermodynamically drives the weaker first side coupled reactions. It will also be understood that the net effect of the reaction scheme of FIG. 3 is to regenerate the coenzymes $NAD(P)^+$ while simultaneously using them to produce at least one desired product comprising the oxidized form of chemical B (a second potentially useful product comprising the reduced form of sacrificial oxidizing agent is also formed). As in the case of the FIG. 2-type reaction scheme, the barrier prevents the second side strong sacrificial redox pair of reactants from otherwise indiscriminate reaction with the first side reactants and catalysts. Also, by the removal of reduced chemical A from the first side to the second side, its concentration in the region of the catalyst 2-catalyzed reaction is reduced, thereby substantially enhancing the efficiency of that reaction, which in turn enhances the efficiency of the catalyst 1-catalyzed reaction coupled to it. Also, as noted in connection with FIG. 2, product separation from spent sacrificial oxidizing agent is achieved without extra processing steps.

Referring to FIG. 4, there is depicted another variant of a scheme for the use and regeneration of the coenzymes NAD(P)H. A reactor is again shown as bifurcated into first and second sides by a permselective barrier that is selectively permeable to the oxidized form of a chemical arbitrarily designated as "chemical A." The coenzymes NAD(P)H may be oxidized on the first side of the barrier by the oxidized form of a second chemical arbitrarily designated as "chemical B" in the presence of hydrogen ions and a first catalyst to form $NAD(P)^+$ and a desired product comprising the reduced form of chemical B. At the same time on the first side of the barrier, $NAD(P)^+$ is reduced with the reduced form of chemical A in the presence of a second catalyst to form NAD(P)H and the oxidized form of chemical A so as to raise the chemical potential of the oxidized form of chemical A on the first side of the barrier.

Simultaneously with the first side reactions of FIG. 4, the oxidized form of chemical A is chemically or physically trapped on the second side of the barrier so as to lower the chemical potential of the oxidized form of chemical A on the second side of the barrier. In this fashion, a predetermined difference in the chemical potentials of the oxidized form of chemical A on either side of the permselective barrier is created, the difference in the chemical potentials effectively providing a driving force for the oxidized form of chemical A to permeate the permselective barrier in the direction noted in FIG. 4.

The term "trapping" means the capturing of the chemical species comprising the oxidized form of chemical A, and is intended to include such mechanisms as irreversibly reacting the oxidized form of chemical A with an agent such as a complexing agent or a polymeric agent, condensation, absorption, and adsorption. In the scheme depicted in FIG. 4, it will be apparent that the reactions are limited by the concentration of reduced chemical A on the first side of the barrier and so, to the extent it is desired to continue the reaction, the reduced form of chemical A must be added to the first side of the reactor.

Thus, the reactions

$$\text{ox chem B} + NAD(P)H + H^+ \xrightarrow[\text{catalyst 1}]{} NAD(P)^+ + \text{red chem B}$$

$$\text{red chem A} + NAD(P)^+ \xrightarrow{\text{catalyst 2}} NAD(P)H + \text{ox chem A}$$

are coupled with each other and coupled to and segregated from the reaction

$$\text{ox chem A} \xrightarrow{\text{trapping}} \text{trapped ox chem A}$$

the coupling of the first side reactions and the second side reaction taking place by the link comprising the barrier-permeating oxidized form of chemical A. It will be apparent that the net effect of the reaction scheme of FIG. 4 is to regenerate the coenzymes NAD(P)H while at the same time using them to produce the products comprising the trapped oxidized form of chemical A and the reduced form of chemical B.

Referring to FIG. 5, there is depicted the redox converse of FIG. 4, FIG. 5 depicting a scheme for the use and regeneration of the coenzymes $NAD(P)^+$. A reactor is again shown as bifurcated into first and second sides by a permselective barrier that is selectively permeable to the reduced form of a chemical arbitrarily designated as "chemical A". The coenzymes $NAD(P)^+$ may be reduced on the first side of the barrier by the reduced form of a second chemical arbitrarily designated as "chemical B" in the presence of a first catalyst so as to form NAD(P)H and the oxidized form of chemical B. At the same time on the first side of the barrier, NAD(P)H is oxidized to $NAD(P)^+$ by the oxidized form of chemical A in the presence of a second catalyst, at the same time forming the reduced form of chemical A. This second reaction raises the chemical potential of the reduced form of chemical A on the first side of the barrier.

Simultaneously with the first side reactions, the reduced form of chemical A is chemically or physically trapped on the second side of the barrier, thereby lowering the chemical potential of the reduced form of chemical A on the second side of the barrier. In this fashion, a predetermined differential in the chemical potentials of the reduced form of chemical A on either side of the permselective barrier is created, which has the effect of providing a driving force for the reduced form of chemical A to permeate the permselective barrier in the direction noted in FIG. 5.

Thus, the reactions

$$\text{red chem B} + NAD(P)^+ \xrightarrow{\text{catalyst 1}} NAD(P)H + \text{ox chem B}$$

$$\text{ox chem A} + NAD(P)H \xrightarrow{\text{catalyst 2}} NAD(P) + \text{red chem A}$$

are coupled with each other and coupled to and segregated from the reaction

$$\text{red chem A} \xrightarrow{\text{trapping}} \text{trapped red chem A}$$

the coupling taking place by the link comprising the barrier-permeating reduced form of chemical A. It will be apparent that the reactions depicted in the scheme of FIG. 5 are limited by the concentration of the oxidized form of chemical A and so, to the extent it is desired to continue the reactions, it will be necessary to replenish that chemical species. It will be appreciated that the net effect of the reaction scheme of FIG. 5 is to use and regenerate the coenzymes $NAD(P)^+$ while producing products comprising the trapped reduced form of chemical A and the oxidized form of chemical B.

In each of the reaction schemes depicted in FIGS. 1-5, the reactions take place in a reactor confining a fluid environment, such as liquid, gel or gas. The preferred environment is liquid, so that all of the reactions take place in solution. The form of the permselective barrier may be a supported gas membrane, a supported liquid membrane, a cast polymer membrane, a thin-film composite membrane, a liquid-swollen polymer membrane, or a series of membrane contactors, also known as perstraction, the last-mentioned form being of the general configuration and operation set forth in 20 J. Memb. Sci. 125 (1984). It will further be appreciated by one skilled in the art that, instead of a single permselective barrier in each of the foregoing five types of schemes, there could be n permselective barriers, where n is any integer, coupling any number of compatible reactions to produce any number of desireable products. Categories of permselective barriers include the following, which are coded for convenience of reference and which codes are used in the Tables herein:

A: supported-gas membrane;

B: supported-liquid membrane consisting of a strongly hydrophobic liquid (e.g., dodecane, alkyl benzenes, perfluoroalkanes) embedded in the pores of a hydrophobic microporous membrane;

C: supported-liquid membrane consisting of a strongly hydrophilic liquid (e.g., water, ethylene glycol, propane 1,3-diol polyethylene glycols) embedded in the pores of a hydrophilic microporous membrane;

D: supported-liquid membrane consisting of a mixture of neutral modifier (e.g., tri-n-octyl phosphine oxide, primary alkyl alcohols, secondary alkyl alcohols, glymes) and a hydrophobic liquid diluent embedded in the pores of a hydrophobic microporous membrane;

E: supported-liquid membrane consisting of a mixture of liquid anion exchanger (e.g., primary alkyl amines, secondary alkyl amines, tertiary alkyl amines, tetraalkyl ammonium) and a hydrophobic liquid diluent embedded in the pores of a hydrophobic microporous membrane;

F: supported-liquid membrane consisting of a mixture of liquid cation exchanger (e.g., alkane carboxylic acids, alkyl sulfonic acids, benzene sulfonic acids, alkyl phosphoric acids) and a hydrophobic liquid diluent embedded in the pores of a hydrophobic microporous membrane;

G: supported-liquid membrane consisting of a mixture of a specific complexing agent (e.g., acetyl acetone, acetyl acetone oxime) and a hydrophobic liquid diluent embedded in the pores of a hydrophobic microporous membrane;

H: dense polymer membrane or thin-film-composite polymer membrane;

I: solvent-swollen dense polymer membrane or solvent-swollen thin-film-composite polymer membrane;

J: anion-exchange polymer membrane;

K: cation-exchange polymer membrane; and

L: size-exclusion membrane (e.g., dialysis membrane, ultrafiltration membrane).

Table A contains specific examples of each membrane type, together with reported specifications, while Table B contains the structures and names of complex organic reagents and classes of such reagents used in the process and apparatus of the present invention.

As mentioned above, the permselective barrier has the dual functions of preventing the solutions on the two sides of the barrier from making direct contact and mixing so as to prevent direct chemical reaction between the components on either side, while at the same time providing a means to selectively transport

TABLE A

| Membrane Type | Examples | thickness (µm) | porosity (%) | pore size (µm) | Supported Liquid (If Applicable) |
|---|---|---|---|---|---|
| A | Celgard 2500 flat sheet (PP) (Celanese, Charlotte, NC) | 25 | 45 | 0.03 | not applicable |
| | Celgard 2400 flat sheet (PP) | 25 | 38 | 0.02 | not applicable |
| | Celgard X-20 hollow fiber (PP) | 27 | 35 | 0.03 | not applicable |
| | Celgard X-10 hollow fiber (PP) | 29 | 40 | 0.03 | not applicable |
| | Goretex flat sheet (PTFE) (W.L. Gore & Assoc.) | 40 | 75 | 2.6 | not applicable |
| | Goretex flat sheet (PTFE) | 75 | 50 | 0.02 | not applicable |
| | Goretex flat sheet (PTFE) | 25 | 80 | 0.20 | not applicable |
| | Kynar hollow fiber (PVDF) (Bend Research, Bend, OR) | 50 | 75 | 0.20 | not applicable |
| | 60TW hollow fiber (PP) (Mitsubishi Rayon America, NY) | 53 | 72 | 0.20-0.50 | not applicable |
| | Microdyn hollow fiber (PP) (Enka AG, W. Germany) | 40 | ~70 | 0.20 | not applicable |
| B | Celgard 2400 | Same as above | | | perfluorocarbon, dodecane, or alkyl benzene |
| | Celgard X-20 | Same as above | | | methyl amyl ketone or amyl acetate |
| | Synpore (Nitrocellulose) (Chechoslovakia) | 100 | na | 0.03 | isobutyl laurate or methyl oleate |
| C | Whatman #1 filter paper (Whatman Lab., Hillsboro, OR) | na | na | na | Cyclodextrin in aqueous buffer |
| | Regenerated Cellulose | | | | |
| | Sartorius SM 11607 (Sartorius GmbH, W. Germany) | 90 | na | 0.20 | Ethylene glycol in water |
| | Enka Cuprophan 250 M (Enka AG, W. Germany) | 17.5 | na | na | Propane 1,3-diol in water |
| | Anapore flat sheet (alumina) (Anotec Separations, New York) | 45 | 90 | 0.20 | Ethylene glycol in water |

TABLE A

| Membrane Type | Examples | thickness (µm) | porosity (%) | pore size (µm) | Supported Liquid (If Applicable) |
|---|---|---|---|---|---|
| D | Celgard 2400 | | Same as above | | Tributyl phosphate in p-nitrophenyl ether |
| | Celgard 2400 | | Same as above | | Chiral crown ether in p-nitro-phenyl ether |
| | Celgard 2500 | | Same as above | | 2,5-di(tert-pentyl) hydroquinone in decaline and tributyl phosphate |
| | NFT-5200 (Teflon) (Nitto electric, Japan) | 75 | na | 0.30 | trioctyl phosphine oxide in kerosene |
| | Ultipore NM (Nylon 6,6) (Pall Trinity, Cortland, NY) | 130 | na | na | Co(MeOSalen) and DMAP in DMSO and butyrolactone |
| E | Celgard 2400 | | Same as above | | Didodecylamine in phenyl ether |
| | No support (extraction only) | na | na | na | Trioctylmethylammonium chloride in xylene |
| | No support (U tube) | na | na | na | Tricaprylmethylammonium chloride in toluene |
| | Celgard 2400 | | Same as above | | Tri(C8-C10)amine in dodecane |
| | Enka TMD 805 hollow fiber (PP) (Enka AG, W. Germany) | 45 | 75 | 0.20 | Trilaurylamine in dodecane |
| F | No support (U tube) | na | na | na | Dinonylnapthalene sulfonic acid in toluene |
| | Celgard 2500 | | Same as above | | Bis(2-ethylhexyl)phosphoric acid in dodecane |
| | Celgard X-20 | | Same as above | | Stearic acid in dodecane |
| G | Kynar hollow fiber (PVDF) | | Same as above | | Acetyl acetone in dodecane |
| | Ultipore NM (Nylon 6,6) | | Same as above | | Acetyl acetone oxime in dodecane |
| H | Poly(dimethyl)siloxane (UOP Fluid Systems, San Diego, CA) | ~2 | na | na | not applicable |
| | Petrothane NA 344-55 (LDPE) | 75 | na | na | not applicable |

TABLE A

| Membrane Type | Examples | thickness (μm) | porosity (%) | pore size (μm) | Supported Liquid (If Applicable) |
|---|---|---|---|---|---|
| | (Luetzow Industries) | | | | |
| | Asymmetric cellulose acetate (Hydronautics, Inc.) | NA | na | na | not applicable |
| | Silicon rubber (Fuji Systems, Inc., Japan) | 120 | na | na | not applicable |
| | FT-30 polyamide thin-film (Filmtec, Minneapolis, MN) | ~0.1 | na | na | not applicable |
| | Silicone/carbonate copolymer (General Electric, Schenectady, NY) | 25 | na | na | not applicable |
| | Poly(etheramide) copolymer (Rikan Chemical, Birdsboro, PA) | 25 | na | na | not applicable |
| | Poly(urethane) [ester base] (Lord Co., Erie, PA) | 75 | na | na | not applicable |
| | Poly(vinylidene chloride) (Dow Chemical, Midland, MI) | 25 | na | na | not applicable |
| | Poly(ethylene) (Cellocraft, Portland, OR) | 30 | na | na | not applicable |
| I | Perfluorosulfonic acid memb. | 170 | na | na | Swollen with ethylene diamine |
| | Calcium alginate flat sheet | 4200 | na | na | Swollen with water |
| | Collagen film | 25 | na | na | Swollen with lactic acid solution |
| | Silicon rubber | Same as above | | | Swollen with perfluorodecalin |
| | Poly(vinylidene chloride) | Same as above | | | Swollen with dinonyl phthalate |
| J | Selemion AMV anion exchange (Asahi Glass, Co., Japan) | na | na | na | not applicable |
| | Neosepter ACH-45T an. exchange (Tokuyama Soda, Co., Japan) | 160 | na | na | not applicable |
| | Uplex CA-2 anion exchange (Asahi Chemical Industry, Co., Japan) | na | na | na | not applicable |
| | AR-111A anion exchange (Ionics, Inc., Cambridge, MA) | 600 | na | na | not applicable |

TABLE A

| Membrane Type | Examples | thickness (µm) | porosity (%) | pore size (µm) | Supported Liquid (If Applicable) |
|---|---|---|---|---|---|
| K | Selemion CMV cation exchange (Asahi Glass, Co., Japan) | na | na | na | not applicable |
| | Neosepter CL-25T cat. exchange (Tokuyama Soda, Co., Japan) | 160 | na | na | not applicable |
| | Uplex CA-1 cation exchange (Asahi Chemical Industry, Co., Japan) | na | na | na | not applicable |
| | CR-61 cation exchange (Ionics, Inc., Cambridge, MA) | 600 | na | na | not applicable |
| | N-901 cation exchange (Dupont Co., Wilmington, DE) | 400 | na | na | not applicable |
| L | Cellulose Acet. Dialysis Tube (Spectrum Medical Industries) | na | na | MWCO=13000 | not applicable |
| | MRD 2370 (Polyester) Mitsubishi Rayon Co. (Tokyo, Japan) | 80 | 53 | na | not applicable |
| | Polysulfone (Abcor-Dorr, W. Germany) (Alfa-Laval, W. Germany) (Amicon) (DDS, Denmark) | na | na | na | not applicable |
| | Cellulose Acetate (Abcor-Dorr) (DDS) (Patterson Candy, Ltd., England) (Nitto, Japan) | na | na | na | not applicable |

NOTES: All specifications are as reported by manufacturer and "na" is an abbreviation for "not available."

Table B

| Designation | Structure | Name |
|---|---|---|
| I | 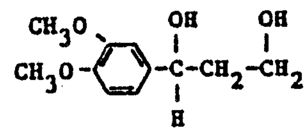 | 3-(3',4'-dimethoxy-phenyl)-3-keto-propan-1-ol |
| II | 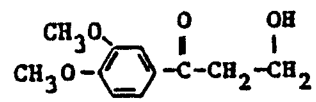 | 3-(3',4'-dimethoxy-phenyl)-3-hydroxy-propan-1-ol |
| III | 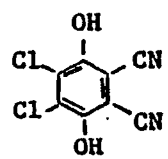 | 2,3-dicyano-5,6-dichloro-dihydroquinone |
| IV | 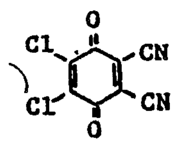 | 2,3-dicyano-5,6-dichloro-quinone |
| V | 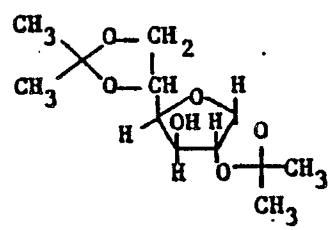 | di-O-isopropylidene-D-glucofuranose |

Table B (cont.)

| Designation | Structure | Name |
|---|---|---|
| VI | 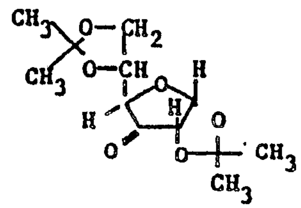 | 3-keto-di-O-isopropylidine-D-glucofuranose |
| VII | 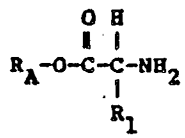 | $AminoAcid_1$-PEG ester |
| VIII | 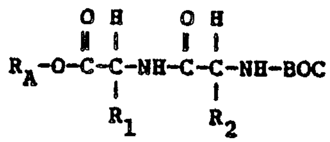 | BOC-$AminoAcid_2$-$AminoAcid_1$-PEG ester |
| IX | 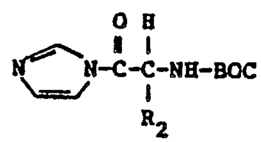 | BOC-$AminoAcid_2$-imidazole |
| X | 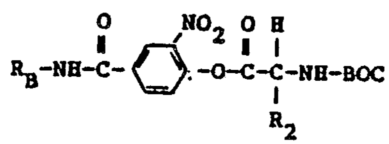 | BOC-$AminoAcid_2$-N-(4'-hydroxy-3'nitro benzoyl)-PEI ester |

Table B (conc.)

| Designation | Structure | Name |
|---|---|---|
| XI | 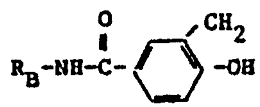 | N-(4'-hydroxy-3'-nitro benzoyl)-PEI |
| XII | 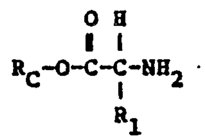 | AminoAcid'$_1$ phenylalkyl ester |
| XIII | 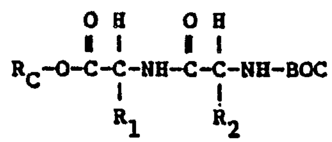 | BOC-AminoAcid$_1$-AminoAcid$_2$-phenylalkyl ester |
| XIV | 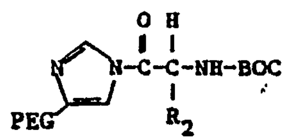 | BOC-AminoAcid$_2$-PEG imidazole |
| XV | 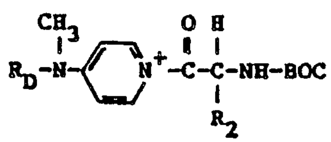 | BOC-AminoAcid$_2$-4-(N-PEG-N-methyl) aminopyridinium cation |

Table B (cont.)

| Designation | Structure | Name |
| --- | --- | --- |
| XVI | 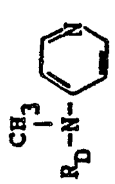 | 4-(N-PEG-N-methyl)aminopyridine |

NOTES:

PEG = polyethylene glycol
BOC = Benzyloxycarbonyl
PEI = polyethylene imine
n = integer from 1 to 100
$R_A$ = $HO-(CH_2-CH_2-O)_n-CH_2CH_2O-$ or

$HO-(CH_2-CH_2-O)_n-CH_2CH_2-O-$ 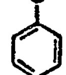 $-CH_2-$

$R_B$ = $NH_2-(CH_2CH_2-NH)_n-CH_2-CH_2-$

$R_C$ = $CH_3(CH_2)_n-$ 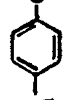 $-CH_2-$

$R_D$ = $CH_3-NH-(CH_2CH_2-O)_n-CH_2-CH_2-$

certain species so as to allow coupling of the chemical reactions taking place on either side of the barrier. The permselective barrier may operate in any of several well-known modes, including a diffusive transport or solution diffusion mode, a pervaporation mode, a carrier-mediated mode or a coupled transport mode. The form of the permselective membrane barrier may be in a flat sheet, a tube, a hollow fiber or spiral-wound.

The configuration of apparatus useful in conducting the method of the present invention must generally comprise means for containing the first side and second side reactants combined with means for securing the permselective barrier in a reactor-bifurcating position and, of course, the permselective barrier itself. To this basic structure may be added suitable access means for adding and retrieving reactants, for stirring the reactants, pressurizing the reactor, etc. One particular embodiment of suitable apparatus is shown in FIG. 6.

There, a reactor 10 is shown supported on a frame 12 and bifurcated into two compartments 14 and 16 by a flat sheet membrane 18 pressed between two collars 20 and 22. Access ports 24, 26, 28 and 30 are provided for adding, retrieving, sampling and stirring the reactants in the two compartments. Stirring motors 32 are connected to stirring paddles 34, the motors being supported upon support 36.

Turning now to more specific embodiments of the present invention, the schematic representations of particular embodiments of the reagent use and regeneration schemes depicted in FIGS. 7-26 will be discussed.

FIGS. 7-12 depict particular exemplary embodiments of the membrane-coupled group transfer reaction use and regeneration scheme shown in FIG. 1.

In FIG. 7, there are shown coupled group transfer reactions that produce the products aldonic acid (first product) and sodium chloride (second product), wherein the group transferred is an electron. On the first side of a permselective barrier comprising a supported-gas membrane that is selectively permeable to iodine (the active form of a reagent) and to hydrogen iodide (the inactive form of the same reagent), D-glucose is reacted with iodine to form aldonic acid and hydrogen iodide, thereby raising the chemical potential of hydrogen iodide and lowering the chemical potential of iodine on the first side. The permselective barrier may be any of the membrane types A, B, D, E, H or I. At the same time on the second side, hydrogen iodide is reacted with sodium hypochlorite to form iodine and sodium chloride, thereby raising the chemical potential of iodine on the second side and lowering the chemical potential of hydrogen iodide on that same side. The relatively higher chemical potential of hydrogen iodide on the first side relative to the second side and relatively higher chemical potential of iodine on the second side relative to the first side provide an effective driving force for the species hydrogen iodide and iodine to permeate the permselective barrier in the directions shown, thereby coupling the reactions on the first and second sides and providing a process for the use and regeneration of iodine.

In FIG. 8, there are shown coupled group transfer reactions that produce the products 2,3-dihydroxy succinic acid (first product) and sodium chlorite (second product), wherein the group transferred is an oxonium ion. On the first side of a permselective barrier comprising a supported-gas membrane that is selectively permeable to both osmium tetroxide (the active form of the osmium oxide reagent) and to osmium dioxide (the inactive form), maleic acid is reacted with osmium tetroxide in the presence of sodium sulfide to form the dihydroxy succinic acid and osmium dioxide, thereby raising the chemical potential of osmium dioxide and lowering the chemical potential of osmium tetroxide on the first side. The permselective barrier may be any of the supported-liquid membrane types noted in Table I as suitable for the coupled reactions shown in FIG. 8. At the same time on the second side of the barrier, osmium dioxide is reacted with sodium chlorate to form osmium tetroxide and sodium hypochlorite, thereby raising the chemical potential of osmium tetroxide and lowering the chemical potential of oxmium dioxide on the second side. The relatively higher chemical potential of osmium dioxide on the first side relative to the second side and the relatively higher chemical potential of osmium dioxide on the second side relative to the first side provide an effective driving force for the two osmium oxide species noted to permeate the permselective barrier in the directions shown in FIG. 8, thereby coupling the reactions on the first and second sides and providing a process for the use and regeneration of osmium tetroxide.

In FIG. 9, there are shown coupled group transfer reactions that produce the products Structure VIII (first product) and Structure XI (second product) wherein the group transferred is an acyl group. On the first side of a permselective barrier comprising a supported-liquid membrane that is selectively permeable to acyl imidazole (the active form of the imidazole reagent) and to imidazole (the inactive form), Structure VII is reacted with acyl imidazole to form Structure VIII and imidazole, thereby raising the chemical potential of imidazole and lowering the chemical potential of acyl imidazole on the first side of the barrier. The permselective barrier may be any of the supported-liquid membrane types in Table I as being suitable for the coupled reactions noted in FIG. 9. At the same time on the second side, imidazole is reacted with Structure X to form acyl imidazole and Structure XI, thereby raising the chemical potential of acyl imidazole and lowering the chemical potential of im!dazole on the second side. As previously noted, the relative differentials in chemical potentials of acyl imidazole and imidazole on the two sides of the barrier provide an effective driving force for those two species to permeate the permselective barrier in the directions shown in FIG. 9, thereby coupling the reactions on the two sides of the barrier and providing a process for the use and regeneration of acyl imidazole.

In FIG. 10, there are shown coupled group transfer reactions that produce the products adenosine triphosphate (ATP--first product) and polyphosphate (second product) wherein the group transferred is a phosphoryl group. On the first side of a permselective barrier comprising an immobilized-liquid membrane that is selectively permeable to acetyl phosphate (the active form of the reagent) and to acetate anion (the inactive form of the same reagent), adenosine diphosphate (ADP) is reacted with acetyl phosphate in the

presence of acetyl kinase to form ATP and the acetate anion, thereby raising the chemical potential of acetate anion and lowering the chemical potential of acetyl phosphate on the first side of the barrier. The permselective barrier may be any of the membrane types noted in Table I as being suitable for the coupled reactions depicted in FIG. 10. At the same time on the second side, acetate anion is reacted with polyphosphate having n phosphoryl units to form acetyl phosphate and polyphosphate having n-1 phosphoryl units, thereby raising the chemical potential of acetyl phosphate and lowering the chemical potential of acetate anion on the second side of the barrier. As previously noted, the relative differentials in chemical potentials of acetate anion and acetyl phosphate on the two sides of the permselective barrier provide an effective driving force for those two species to permeate the permselective barrier in the directions shown in FIG. 10, thereby coupling the reactions on the first and second sides and providing a process for the use and regeneration of acetyl phosphate.

In FIG. 11, there is shown the use of the present invention to carry out group transfer reactions involving reactants that are highly reactive to each other in homogeneous solution and involving multiple redox cycles, explained in greater detail in Example 5 below.

FIG. 12 illustrates a FIG. 1-type reaction scheme using more than one permselective barrier to couple additional reactions.

Other exemplary embodiments of membrane-coupled group transfer reactions suitable for the type of use and regeneration scheme shown in FIG. 1 are noted in Table I.

FIGS. 13-17 depict particular exemplary embodiments of the type of membrane-coupled NAD(P)H use and regeneration scheme shown in FIG. 2.

In FIG. 13, there are shown coupled reactions that use and regenerate the coenzyme NADH to produce the product D-lactate. There, a permselective barrier is shown bifurcating a reactor into a first side and a second side, the barrier being selectively permeable to acetaldehyde (oxidized form of chemical) and to ethanol (reduced form) and comprising any of the membrane types set forth in Table II, first entry. A series of coupled reactions are shown on the first side of the barrier, with a single reaction shown on the second side. On the first side, NADH is oxidized by pyruvate (the oxidized form) in the presence of hydrogen ion such as is supplied by an acid, the reaction being catalyzed by D-lactate dehydrogenase, to form, $NAD^+$ and the desired product D-lactate (the reduced form).

At the same time on the second side of the barrier shown in FIG. 13, acetaldehyde is reduced in the presence of sodium hydroxide by the sacrificial reducing agent borohydride which is a stronger reducing

Table I

| | First Side | | | | Second Side | | |
|---|---|---|---|---|---|---|---|
| Group Transfer | First Reactant | First Product | Inactive Reagent | Barrier | Active Reagent | Second Reactant | Second Product |
| electron | I | II | III | B,D,I, | IV | nitric acid | nitrous acid |
| electron | oxidized lignin | reduced lignin | oxidized anthra-quinone | B,D,I | reduced anthra-quinone | sodium borohydride | sodium borate |
| electron | glucose | aldonic acid | hydrogen iodide | A,B,C,D,E,H,I | iodine | sodium hypochlorite | sodium chlorid |
| electron | aldonic acid | 2-ketoaldonic acid | ferrous ion | C,D,F,G,K,L | ferric ion | chlorine | chloride ion |
| electron | oxidized glutathione | reduced glutathione | bis-methyl thiol | A,B,D,H,I,L | methane thiol | sodium cyano borohydride | sodium borate |
| electron | dibutyl acetylene | 5,6-diketo-decane | ruthenium dioxide | C,D,E,F,G,I,K | ruthenium tetroxide | sodium periodate | sodium iodate |
| electron | p-methoxy-toluene | p-methoxy-benzaldehyde | cerous ion | C,D,F,G,K | ceric ion | hydrogen peroxide | water |
| electron | 1,2-diamino-cyclohexane | adiponitrile | hydrogen bromide | A,B,C,D,E,H,I | bromine | potassium permanganate | manganese dixoide |
| hydride | V | VI | ruthenium dioxide | C,D,E,F,G,I,K,L | ruthenium tetroxide | sodium iodate | sodium iodite |
| hydride | acrolein | acrylic acid | selenium oxide | C,D,E,F,G,I,K | selenium dioxide | hydrogen peroxide | water |
| hydride | 3-chloro butane-2-ol | 3-chloro-butane-2-one | ruthenium doixide | C,D,E,F,G,I,K | ruthenium tetroxide | sodium periodate | sodium iodate |
| hydride | acetaldehyde | acetic acid | iodide ion | A,B,C,D,E,H,I | iodine | hydrogen peroxide | water |

Table I (Cont.)

| | First Side | | | | Second Side | | |
|---|---|---|---|---|---|---|---|
| Group Transfer | First Reactant | First Product | Inactive Reagent | Barrier | Active Reagent | Second Reactant | Second Product |
| hydride | benzyl alcohol | benzaldehyde | ruthenium dioxide | C,D,E,F,G,I,K | ruthenium tetroxide | chlorine gas | chloride ion |
| bromonium ion | tyrosine | 3,5-dibromo-phenyl tyrosine | hydrogen bromide | A,B,C,D,E,H,I | bromine | sodium bromate | sodium bromite |
| chloronium ion | cinnamal-dehyde | 2,3-dichloro cinnamal-dehyde | hydrogen chloride | A,B,C,D,E,H,I | chlorine | sodium chlorate | sodium chlorite |
| oxonium ion | maleic acid | 2,3-dihydroxy succinic acid | osmium dioxide | A,B,C,D,I | osmium tetroxide | sodium chlorate | sodium hypochlorite |
| methyl | sodium thio-phenolate | methyl thio-phenolate | 4-(dial-kylamino) pyridine | B,D,I | N-methyl-4-dialkyl amino pyridinium cation | methyl chloride | hydrogen chloride |
| acetyl | coenzyme A | acetyl coenzyme A | imidazole | B,C,D,H,I,L | N-acetyl imidazole | acetic anhydride | acetic acid |
| acetyl | linalool | O-acetyl linalool | 4-(dial-kylamino) pyridine | B,D,I | N-acetyl-4-(dialkyl-amino) pyridinium cation | acetic anhydride | acetic acid |
| benzoyl | benzyl amine | N-(benzoyl-benzyl amine | imidazole | B,C,D,H,I,L | N-benzoyl imidazole | benzoyl chloride | hydrogen chloride |
| acyl | VII | VIII | imidazole | B,C,D,H,I,L | acyl imidazole (IX) | X | XI |

Table I (Cont.)

| First Side | | | | | Second Side | | |
|---|---|---|---|---|---|---|---|
| Group abvnsfer | First Reactant | First Product | Inactive Reagent | Barrier | Active Reagent | Second Reactant | Second Product |
| acyl | XII | XIII | PEG-imid-azole | C | PEG-acyli-midazole | XV | XVI |
| oxycar-bonyl | aniline | N-(isobutyl)-formanilide | 4-(dial-kylamino) pyridine | B,D,I | N-(isobu-tylformyl)-4-dialkyl-amino pyridinium cation | isobutyl chloroformate | hydrogen chloride |
| alkyl-sufonyl | aniline | N-(p-toluene-sulfonyl)-aniline | 4-(dial-kylamino) pyridine | B,D,I | N-(p-tol-uenesul-fonyl)-4-dialkyl-amino pyridinium cation | p-toluenesul-fonyl chloride | hydrogen chloride |
| phosphoryl | adenosine diphosphate | andenosine triphosphate | acetate | E,J,L | acetyl phosphate | polyphosphate (n units long) | polyphosphate (n-1 units long) |
| dialkyl phosporyl | benzyl amine | N-dialkyl-phosphoryl)-benzyl amine | 4-(dial-kylamino) pyridine | B,D,I | N-dialkyl-phosphor)-yl)-4-dial-kylamino pyridinium cation | dialkyl phosphoro-chloridate | hydrogen chloride |

Table II

| Chemical A Ox/Red | Chemical B Ox/Red | Catalyst 1* | Catalyst 2* | Catalyst 3 | Sacrificial Agent Red/Ox | Barrier |
|---|---|---|---|---|---|---|
| acetaldehyde/ethanol | pyruvate/D-lactate | D-lactate deh. | yeast alcohol deh. | NaOH | borohydride/borate | A,B,D,H,I |
| do | 2-ketoglutarate/L-glutamate | glutamate deh. | do | $PtO_2$ | hydrogen/none | do |
| acetone/isopropanol | xylose/xylitol | xylose red. | glycerol deh. | Pt with Fe or Mo | do | do |
| cyclohexanone/cyclohexanol | pyruvate/L-alanine | L-alanine deh. | horse liver alcohol deh. | NaOH | Ni-Al alloy/$Al(OH)_3$ | do |
| hexafluoroacetone/hexafluoro-2-propanol | L-7,8-dihydrobiopterin/6(R)-L-erythro-5,6, 7,8-tetrahydrobiopterin | dihydrofolate red. | yeast alcohol deh. | none | cyanoborohydride/cyanoborate | do |
| heptafluoro-n-butyraldehyde/heptafluoro-1-butanol | 2-keto isocaproate/L-leucine | L-leucine deh. | horse liver alcohol deh. | Pd with Fe or Mo | hydrogen/none | do |
| acetone/2-propanol | 2-acetyl furan/(S)-2-furanyl methyl carbinol | yeast alcohol deh. | yeast alcohol deh. | NaOH | borohydride/borate | do |
| hexafluoroacetone/hexafluoro-2-propanol | phenylpyruvic acid/L-phenylalanine | L-phenylalanine deh. | glycerol deh. | acetic acid | $Fe/Fe_2O_3$ | do |

Table II (Cont.)

| Chemical A Ox/Red | Chemical B Ox/Red | Catalyst 1* | Catalyst 2* | Catalyst 3 | Sacrificial Agent Red/Ox | Barrier |
|---|---|---|---|---|---|---|
| oxaloacetate/ D,L-malate | 3-dehydro-carnitine/ L-carnitine | carnitine deh. | D,L-malate deh. | Pt with Fe or Mb | hydrogen/none | E,J |
| dihydroxy-acetone/glycerol | do | do | glycerol deh. | NaOH | borohydride/ borate | B,D,H,I |
| decafluoro benzophenone/ decafluoro benzhydrol | beta-chloro-pyruvic acid/ D-chlorolactic acid | lactate deh. | horse liver alcohol deh. | $CH_3COOH$ | $Fe/Fe_2O_3$ | do |
| pentafluoro benzaldehyde/ pentafluoro benzyl alcohol | 3-chloro-2-oxo-butanoic acid/3-chloro-2-hydroxy-butanoic acid | do | do | $Na_2CO_3$ | dithionite/ bisulfite | do |
| hexafluoro-acetone/ hexafluoro-2-propanol | dehydrocholic acid/cholic acid | hydroxy-steroid deh. | yeast alcohol deh. | none | cyanoboro-hydride/ cyanoborate | A,B,D, H,I |
| oxaloacetate/ D,L-malate | ketopanto-lactone/(3R)-dihydro-4,4-diethyl-3-hydroxy-2-furanone | ketopanto-lactone red. | D,L-malate deh. | $PtO_2$ | hydrogen/none | E,J |
| do | cinnamalde-hyde/cinnamyl alcohol | yeast alcohol deh. | do | $PtO_2$ | do | E,J |

*NOTE: The abbreviations "deh." and "red." stand for dehyrogenase and reductase, respectively.

agent than ethanol, thereby forming ethanol and raising its chemical potential on the second side, while simultaneously lowering the chemical potential of acetaldehyde on the same side. Simultaneously with the two aforementioned reactions, $NAD^+$ is reduced on the first side of the barrier with ethanol, the reaction being catalyzed by alcohol dehydrogenase, to form acetaldehyde and regenerate NADH, thereby raising the

chemical potential of acetaldehyde and ethanol on the two sides of the barrier provide an effective driving force for those two species to permeate the permselective barrier in the directions noted in FIG. 13, thereby coupling the first side reactions with the second side reacction and providing a process for the use and regeneration of NADH.

In FIG. 14, there are shown coupled reactions that use and regenerate the coenzyme NADH to produce the product L-glutamate by the stereospecific reductive amination of 2-ketoglutarate. There, a permselective barrier is again shown bifurcating a reactor into a first side and a second side, the barrier being selectively permeable to acetaldehyde (oxidized form of chemical) and to ethanol (reduced form) and comprising any of the membrane types set forth in Table II, second entry. A series of coupled reactions are shown on the first side of the barrier; with a single reaction shown on the second side. On the first side, NADH is oxidized by 2-ketoglutarate and catalyzed by glutamate dehydrogenase in the presence of hydrogen ion, to form $NAD^+$ and the desired product L-glutamate. At the same time on the second side of the barrier, acetaldehyde is reduced with a sacrificial reducing agent such as hydrogen, catalyzed by a platinum oxide catalyst, which is a stronger reducing agent than ethanol, thereby forming ethanol and raising its chemical potential on the second side, while simultaneously lowering the chemical potential of acetaldehyde on the same side.

Simultaneously with the two aforementioned reactions of FIG. 14, $NAD^+$ is reduced on the first side of the barrier with ethanol, the reaction being catalyzed by alcohol dehydrogenase, to form acetaldehyde and regenerate NADH, thereby raising the chemical potential of acetaldehyde on the first side of the barrier and lowering the chemical potential of ethanol on that same side. The differences in chemical potentials of acetaldehyde and ethanol on the two sides of the barrier provide an effective driving force for those two species to permeate the permselective barrier in the directions noted in FIG. 14, thereby coupling the first side reactions, with the second side reaction, and providing a process for the use and regeneration of NADH.

In FIG. 15, there are shown coupled reactions that use and regenerate the coenzyme NADH to produce the product L-alpha-amino acid by the alpha-amino acid transaminase-catalyzed reaction of the corresponding alpha-keto acid and L-glutamate, with L-glutamate regeneration. There is again shown a permselective barrier bifurcating a reactor into a first side and a second side, the barrier being selectively permeable to acetaldehyde (oxidized form of chemical) and to ethanol (reduced form) and comprising any of the membrane types A,B,D,H and I. A series of three coupled reactions are shown on the first side of the barrier, with a single reaction shown on the second side. On the first side, NADH is oxidized by 2-ketoglutarate in the presence of ammonia and hydrogen ion, the reaction being catalyzed by glutamate dehydrogenase, to form $NAD^+$ and L-glutamic acid. The latter in turn is reacted with alpha-keto acid catalyzed by the alpha-amino acid transaminase to form the corresponding L-alpha-amino acid as a desired end product. At the same time on the second side of the barrier, acetaldehyde is reduced with the sacrificial reducing agent hydrogen in the presence of a platinum oxide catalyst to form ethanol, thereby raising its chemical potential on the second side, while simultaneously lowering the chemical potential of acetaldehyde on the same side.

Simultaneously with the two aforementioned reactions shown in FIG. 15, $NAD^+$ is reduced on the first side of the barrier with ethanol, the reaction again being catalyzed by alcohol dehydrogenase, to form acetaldehyde and regenerate NADH, thereby raising the chemical potential of acetaldehyde on the first side of the barrier and lowering the chemical potential of ethanol on that same side. The differences in chemical potentials of acetaldehyde and ethanol on the two sides of the barrier provide an effective driving force for those two species to permeate the permselective barrier in the directions noted in FIG. 13, thereby coupling the first side reactions with the second side reaction and providing a process for the use and regeneration of NADH.

In FIG. 16, there are shown coupled reactions that use and regenerate the coenzyme NADH to produce the product L-alanine. Again, a permselective barrier is shown bifurcating a reactor into a first side and a second side, the barrier being selectively permeable to cyclohexanone (oxidized form of chemical) and to cyclohexanol (reduced form) and comprising any of the membrane types set forth in Table II, fourth entry. Two coupled reactions are shown on the first side of the barrier, with a single reaction shown on the second side. On the first side, NADH is oxidized by pyruvate (the oxidized form) in the presence of hydrogen and ammonium ions, the reaction being catalyzed by L-alanine dehydrogenase, to form $NAD^+$ and the desired product L-alanine (the reduced form). At the same time on the second side of the barrier, cyclohexanone is reduced with the sacrificial reducing agent aluminum in the presence of NaOH and a nickel catalyst, comprising a stronger reducing agent than cyclohexanol, thereby forming cyclohexanol and raising its chemical potential on the second side, while simultaneously lowering the chemical potential of cyclohexanone on the same side.

Simultaneously with the two aforementioned reactions of FIG. 16, $NAD^+$ is reduced on the first side of the barrier with cyclohexanol, the reaction being catalyzed by horse liver alcohol dehydrogenase, to form cyclohexanone and regenerate NADH, thereby raising the chemical potential of cyclohexanone on the first side of the barrier and lowering the chemical potential of cyclohexanol on that same side. The differences in chemical potentials of cyclohexanone and cyclohexanol on the two sides of the barrier provide an effective driving force for those two chemicals to permeate the permselective barrier in the directions noted in FIG. 16, thereby coupling the first side reactions with the second side reaction and providing a process for the use and regeneration of NADH.

In FIG. 17, there are shown coupled reactions that use and regenerate the coenzyme NADPH to produce the product (S)-2-furanyl methylcarbinol. A permselective barrier is again shown bifurcating a reactor into a first side and a second side, the barrier being selectively permeable to acetone (oxidized form) and to 2-propanol (reduced form) and comprising any of the membrane types A,B,D,H and I. Two coupled reactions are shown on the first side of the barrier, with a single reaction shown on the second side. On the first side, NADPH is oxidized by 2-acetyl furan (oxidized form) in the presence of hydrogen ion, the reaction being catalyzed by alcohol dehydrogenase, to form $NADP^+$ and the desired carbinol product (the reduced form). At the same time on the second side of the barrier acetone is reduced with the sacrificial reducing agent borohydride in the presence of NaOH as a catalyst, which comprises a stronger reducing agent than 2-propanol, thereby forming 2-propanol and raising its chemical potential on the second side, while simultaneously lowering the chemical potential of acetone on the same side.

Simultaneously with the two aforementioned reactions of FIG. 17, $NADP^+$ is reduced on the first side of the barrier with 2-propanol in the presence of the catalyst alcohol dehydrogenase, to form acetone and regenerate NADPH, thereby raising the chemical potential of acetone on the first side of the barrier and lowering the chemical potential of 2-propanol on that same side. The differences in chemical potentials of acetone and 2-propanol on the two sides of the barrier provide an effective driving force for those two chemicals to permeate the permselective barrier in the directions noted in FIG. 17, thereby coupling the first side reactions with the second side reaction and providing a process for the use and regeneration of NADPH.

Other exemplary embodiments of membrane-coupled group transfer reactions suitable for the type of use and regeneration scheme shown in FIG. 2 are noted in Table II.

FIGS. 18-20 depict particular exemplary embodiments of the membrane-coupled reaction types shown in FIG. 3 for the use and regeneration of the coenzymes $NAD(P)^+$.

In FIG. 18, there are shown coupled reactions that use and regenerate the coenzyme $NAD^+$ to produce the product cis-3-oxabicyclo[4.3.0]nonan-2-one. There, a permselective barrier is again shown bifurcating a reactor into a first side and a second side, the barrier being selectively permeable to acetone (oxidized form of chemical) and to 2-propanol (reduced form) and comprising any of the membrane types set forth in Table III, first entry. Two coupled reactions are shown on the first side of the barrier, with a single reaction shown on the second side. On the

Table III

| Chemical A Red/Ox | Chemical B Red/Ox | Catalyst 1 | Catalyst 2 | Catalyst 3 | Sacrificial Agent Ox/Red | Barrier |
|---|---|---|---|---|---|---|
| 2-propanol/acetone | cis-1,2-bis (hydroxymethyl) cyclohexane/ cis-3-oxabicyclo [4.3.0] nonan-2-one | horse liver alcohol deh. | horse liver alcohol deh. | sulfuric acid | dichromate/ Cr(III) | A |
| decafluoro benzhydrol/ decafluoro benzophenone | D-sorbitol/ L-sorbose | D-sorbitol deh. | do | ferric chloride | $H_2O_2/H_2O$ | B,D,H,I |
| cyclopentanol/ cyclopentanone | D-mannitol/ D-fructose | D-mannitol deh. | do | Pt | $O_2/H_2O$ | do |
| 1,1,1,3,3,3-hexafluoro-2-propanol/hexa-fluoro acetone | 1,2-dehydro-3-ketosteroids/ 3-ketosteroids | steroid-1-deh. | glycerol deh. | pyridine | Pb tetra-acetate/$PbO_2$ | A |
| phenyltri-fluoromethyl carbinol/ trifluoro acetophenone | D-2-hydroxy-4-methylpentan-oate/2-oxo-4-methylpentan-oate | D-2-hydroxy-4-methyl pentan-oate deh. | horse liver alcohol deh. | ruthenium tetroxide | periodate/ iodate | A,B,D, H,I |
| 1,1,1,3,3,3-hexafluoro-2-propanol/hexa-fluoro acetone | glycerol/ dihydroxy-acetone | glycerol deh. | glycerol deh. | none | nitric acid/ nitrous acid | A |
| 1,1,1-trifluoro 2-propanol/1,1, 1-trifluoro acetone | dehydro-cholic acid/ cholic acid | hydroxy-steroid deh. | yeast alcohol deh. | sulfuric acid | permanganate/ $MnO_2$ | A |

Table III (Cont.)

| Chemical A Red/Ox | Chemical B Red/Ox | Catalyst 1 | Catalyst 2 | Catalyst 3 | Sacrificial Agent Ox/Red | Barrier |
|---|---|---|---|---|---|---|
| 2-propanol/acetone | bridged-bicyclomeso-diols/bridged-bicyclo-lactones | horse liver alcohol deh. | horse liver alcohol deh. | palladium dichloride cupric nitrate | $O_2/H_2O$ | A |
| 1,1,1,3,3,3-hexafluoro-2-propanol/hexa-fluoro acetone | meso-cis-1,2-bis(hydroxy-methyl)cyclo-butane/cis-3-oxabicyclo[3.2.0]heptan-2-one | do | do | none | NaOCl/NaCl | do |

first side, $NAD^+$ is reduced by cis-1,2-bis-(hydroxymethyl) cyclohexane (the reduced form), in the presence of the catalyst horse liver alcohol dehydrogenase to produce NADH and the product cis-3-oxabicyclo[4.3.0]-nonan-2-one. At the same time on the second side of the barrier, 2-propanol is oxized with the sacrificial oxidizing agent dichromate ion in the presence of sulfuric acid to form acetone, the dichromate ion being a

stronger oxidizing agent than acetone, thereby raising the chemical potential of acetone on the second side of the barrier and lowering the chemical potential of 2-propanol.

Simultaneously with the two aforementioned reactions of FIG. 18, NADH is oxidized with acetone in the presence of hydrogen ion on the first side of the barrier, the reaction being catalyzed by horse liver alcohol dehydrogenase to regenerate $NAD^+$ and form 2-propanol, thereby raising the chemical potential of 2-propanol on the first side and lowering the chemical potential of acetone on that same side. As in previous examples, the differences in chemical potentials of acetone and 2-propanol on the two sides of the barrier provide an effective driving force for those two species to permeate the barrier, thereby coupling the first side reactions with the second side reaction. In connection with FIG. 18, it should be noted that the thermodynamics of the coupled reactions depicted therein strongly favor the formation of cis-3-oxabicyclo-[4.3.0]nonan-2-one as well as the trivalent chromium ion, over the diol counterpart and dichromate, respectively.

In FIG. 19, there are shown coupled reactions that use and regenerate the coenzyme $NAD^+$ to produce the product L-sorbose. There, a permselective barrier is again shown bifurcating a reactor into a first side and a second side, the barrier being selectively permeable to decafluoro benzophenone (oxidized form) and to decafluro benzhydrol and comprising any of the membrane types set forth in Table III, second entry. Two coupled reactions are shown on the first side of the barrier, with a single reaction shown on the second side. On the first side, $NAD^+$ is reduced by D-sorbitol (the reduced form) in the presence of the catalyst D-sorbitol dehydrogenase to produce NADH and the product L-sorbose. At the same time on the second side of the barrier, decafluoro benzhydrol is oxidized with the sacrificial oxidizing agent hydrogen peroxide in the presence of ferric ion to form decafluoro benzophenone, the hydrogen peroxide being a stronger oxidizing agent than decafluoro benzophenone, thereby raising the chemical potential of the benzophenone on the second side of the barrier and lowering the chemical potential of the benzhydrol.

Simultaneously with the two aforementioned reactions of FIG. 19, NADH is oxidized with the benzophenone in the presence of hydrogen ion on the first side of the barrier, the reaction being catalyzed by horse liver alcohol dehydrogenase to regenerate $NAD^+$ and form the benzhydrol, thereby raising the chemical potential of that species on the first side and lowering the chemical potential of the benzophenone on the same side. As in previous examples, the differences in chemical potentials of benzophenone and benzhydrol on the two sides of the barrier provide an effective driving force for those two species to permeate the barrier, thereby coupling the first side reactions with the second side reaction. In connection with FIG. 19, it should be noted that the thermodynamics of the coupled reactions depicted therein strongly favor the formation of the benzophenone and water over the benzhydrol and hydrogen peroxide, respectively.

In FIG. 20, there are shown coupled reactions that use and regenerate the coenzyme $NAD^+$ to produce the product dihydroxyacetone. There, a permselective barrier is again shown bifurcating a reactor into a first side and a second side, the barrier being selectively permeable to hexafluoro acetone (oxidized form) and to 1,1,1,3,3,3-hexafluoro-2-propanol (reduced form) and comprising any of the membrane types set forth in Table III, sixth entry. Two coupled reactions are shown on the first side of the barrier, and one reaction on the second side. On the first side, $NAD^+$ is reduced by glycerol (the reduced form) in the presence of the catalyst glycerol dehydrogenase to produce NADH and the product dihydroxyacetone. At the same time on the second side of the barrier, the hexafluoro propanol is oxidized with the sacrificial oxidizing agent nitric acid to form hexafluoro acetone, nitric acid being a stronger oxidizing agent than the hexafluoro acetone, thereby raising the chemical potential of hexafluoro acetone on the second side of the barrier and lowering the chemical potential of the corresponding propanol on the same side.

Simultaneously with the two aforementioned reactions of FIG. 20, NADH is oxidized with hexafluoro acetone in the presence of hydrogen ion on the first side of the barrier, the reaction being catalyzed by glycerol dehydrogenase to regenerate $NAD^+$ and form the corresponding propanol, thereby raising the chemical potential of the propanol on the first side and lowering the chemical potential of the acetone on that same side. As in previous examples, the differences in chemical potentials of the acetone and propanol on the two sides of the barrier provide an effective driving force for those two species to permeate the barrier, thereby coupling the first side reactions with the second side reaction. In connection with FIG. 20, it should be noted that the thermodynamics of the coupled reactions depicted therein strongly favor the formation of the acetone and nitrous acid over the corresponding propanol and nitric acid, respectively.

Other exemplary embodiments of membrane-coupled group transfer reactions suitable for the type of use and regeneration scheme shown in FIG. 3 are noted in Table III.

FIGS. 21-24 depict particular exemplary embodiments of the membrane-coupled reaction types shown in FIG. 4 for the use and regeneration of the coenzymes NAD(P)H.

In FIG. 21, there are shown coupled reactions that use and regenerate the coenzyme NADH to produce

the product L-carnitine. As in previous exemplary embodiments, a permselective barrier is shown bifurcating a reactor into a first side and a second side, the barrier in this case being selectively permeable to a single species, here, phenyl pyruvate (the oxidized form of a chemical), the membrane comprising any of the membrane types set forth in Table IV, ninth entry. In this particular embodiment, the membrane comprises a microporous polymer impregnated to saturation with a mixture comprising 67 wt% dodecane and 33 wt% "Primene" (a mixture of primary alkyl amines containing 12 to 16 carbons made by Rohm and Haas). The membrane exhibits a 100:1 disparity in permeability to phenyl pyruvate anion over L-phenyl lactate anion by virtue of the formation of a Schiff base complex between the alkyl amines and the phenyl pyruvate. The transport of phenyl pyruvate across the permselective barrier is therefore a form of carrier-mediated transport, as opposed to being based upon mere diffusion, requiring Schiff base formation with the carbonyl as well as protonation of the alkyl amines for the transport mechanism.

Two coupled reactions are shown on the first side of the barrier of FIG. 21, with a single trapping reaction shown on the second side. On the first side, NADH is oxidized by 3-dehydrocarnitine (the oxidized form) in the presence of hydrogen ion and catalyzed by

Table IV

| Chemical A Ox/Red | Chemical B Ox/Red | Catalyst 1 | Catalyst 2 | Catalyst 3 | Trapping | Trapped Ox. Chem. A | Barrier |
|---|---|---|---|---|---|---|---|
| acetaldehyde/ ethanol | pyruvate/ D-lactate | D-lactate deh. | yeast alcohol deh. | none | vacuum/cold surface | condensed acetaldehyde | B,D,E, H,I |
| do | 2-ketoglut-arate/ L-glutamate | glutamate deh. | do | sodium bicarbonate | hydroxyl amine | acetaldehyde oxime | A,B,D, E,H,I |
| acetone/ isopropanol | xylose/ xylitol | xylose red. | do | pyridine HCl | semicarba-zide hydro-chloride | acetone semi-carbazone | do |
| cyclohexanone/ cyclohexanol | pyruvate/ L-alanine | L-alanine deh. | horse liver alcohol deh. | none | activated charcoal | adsorbed cyclohexanone | B,D,H,I |
| hexafluoro acetone/ 1,1,1,3,3,3,-hexafluoro-2-propanol | L-7,8-dihydro-biopterin/ 6(R)-L-erythro-5,6,7,8-tetra-hydrobiopterin | dihydrofolate red. | yeast alcohol deh. | sodium bicarbonate | polyethylene imine | polymer-bound Schiff base complex | A,B,D, E,H,I |
| 2,2,3,3,4,4,4-heptafluoro-1-butenal/ 2,2,3,3,4,4,4,-heptafluoro-1-butanol | alpha-keto isocaproate/ L-leucine | L-leucine deh. | horse liver alcohol deh. | pyridine hydro-chloride | ethanolamine | Schiff base complex | do |
| cyclopentanone/ cyclopentanol | dihydrofolic acid/ tetrahydro-folic acid | dihydrofolate red. | do | do | ethylene diamine/ sodium cyanoboro-hydride | N-cyclo-pentyl-diamino-propane | B,D,H,I |

Table IV (Cont.)

| Chemical A Ox/Red | Chemical B Ox/Red | Catalyst 1 | Catalyst 2 | Catalyst 3 | Trapping | Trapped Ox. Chem. A | Barrier |
|---|---|---|---|---|---|---|---|
| trifluoro acetone/ 1,1,1-tri-fluoro-2-propanol | phenylpyru-vate/L-phenyl-alanine | L-phenyl-anine deh. | glycerol deh. | none | sodium bisulfite | hexafluoro-acetone bisulfite adduct | A,B,D, E,H,I |
| phenylpyruvate/ L-phenyllactate | 3-dehydro-carnitine/ L-carnitine | carnitine deh. | L-phenyl-lactate deh. | none | sodium hydroxide | phenyl-pyruvate anion | B,D,E, I |
| dihydroxy acetone/ glycerol | do | do | glycerol deh. | sodium bicarbonate | diethylene triamine | Schiff base complex | B,D,E, H,I |
| decafluoro benzophenone/ decafluoro benzhydrol | beta-chloro-pyruvic acid/ D-chlorolactic acid | lactate deh. | horse liver alcohol deh. | do | N-alkyl amine impregnated clay | bound Schiff base complex | do |
| pentafluoro benzaldehyde/ pentafluoro benzyl alcohol | 3-chloro-2-oxobutanoic acid/ 3-chloro-2-hydroxy-butanoic acid | do | do | do | D-alkylhy-droxylamine-impreganted clay | bound oxime | do |
| hexafluoro acetone/ 1,1,1,3,3,3-hexafluoro-2-propanol | dehydrocholic acid/cholic acid | hydroxy-steroid deh. | yeast alcohol deh. | none | vacuum/ charcoal bed | adsorbed hexafluoro-acetone | do |
| oxaloacetate/ D,L-malate | cinnamalde-hyde/cinnamyl alcohol | yeast alcohol deh. | D,L-malate deh. | sodium bicarbonate | N-alkylamine impregnated charcoal | bound Schiff base complex | E,J |

carnitine dehydrogenase at a pH of about 7 to produce $NAD^+$ and the product L-carnitine. At the same time on the second side of the barrier, the chemical potential of phenyl pyruvate is dramatically lowered thereby chemically trapping it by reaction with sodium hydroxide at a pH of about 13. It is well known that the chemical potential of the conjugate base in an acid-base reaction is governed by the pH of the solution.

Simultaneously with the two aforementioned reactions, NAD+ is reduced with L-phenyl lactate on the first side of the barrier, the reaction being catalyzed by the dehydrogenase shown, to regenerate NADH and form phenyl pyruvate, thereby raising the chemical potential of the pyruvate form on the first side and lowering the chemical potential of the lactate form on the same side of the barrier. Because of the difference in pH between the first and second sides, pyruvate is transported rapidly from the first side to the second side, where the alkyl amines that cross the membrane can be protonated, but is not transported back from the second side to the first side because of the high alkalinity on the second side prevents alkyl amine from becoming protonated. This creates a disparity in the transport rates of phenyl pyruvate from the first and from the second sides of the barrier so as to result in net permeation of the barrier in the direction noted in FIG. 21, thereby coupling the first side reactions with the second side trapping reaction.

In FIG. 22 there are shown coupled reactions that use and regenerate the coenzyme NADH to produce the product D-lactate. A permselective barrier is shown bifurcating a reactor into two sides, the barrier in this case being selectively permeable to a single species, namely, acetaldehyde, the membrane comprising any of the membrane types in Table IV, first entry. In this particular embodiment, the membrane comprises a dense polymer, supported-liquid or solvent-swollen dense polymer in which acetaldehyde exhibits high solubility. Acetaldehyde dissolves in the membrane phase and permeates through the membrane by diffusion. At the second side, acetaldehyde undergoes evaporation as a result of the vacuum at the second side. Two coupled reactions are shown on the first side with a single trapping reaction on the second side. On the first side, NADH is oxidized by pyruvate (the oxidized form) in the presence of hydrogen ion and catalyzed by D-lactate dehydrogenase to produce $NAD^+$ and the product D-lactate.

At the same time on the second side of the barrier, acetaldehyde in gaseous form is trapped by condensing it to a liquid by contacting it with a cold surface, thereby lowering the chemical potential of acetaldehyde in gaseous form on the second side of the barrier. Simultaneously with the two aforementioned reactions, NAD+ is reduced with ethanol on the first side of the barrier, the reaction being catalyzed by alcohol dehydrogenase to regenerate NADH and form acetaldehyde dissolved in water, thereby raising the chemical potential of acetaldehyde on the first side and lowering the chemical potential of ethanol on the same side of the barrier. Thus, acetaldehyde permeates the barrier in the direction noted in FIG. 22, thereby coupling the first side reactions with the second side trapping reaction.

In FIG. 23, there are shown another set of coupled reactions that use and regenerate the coenzyme NAD H to produce the product D-xylitol. Again, a permselective barrier bifurcates a reactor into two sides, the barrier comprising any of the membrane types set forth in Table IV, third entry, and being selectively permeable to acetone. Two coupled reactions are shown on the first side of the barrier, with a single trapping reaction shown on the second side. On the first side, NADPH is oxidized by D-xylose (the oxidized form) in the presence of hydrogen ion and catalyzed by xylose reductase to produce $NADP^+$ and the product D-xylitol. At the same time on the second side of the barrier, acetone is chemically trapped as acetone semicarbazone by reaction with semicarbazide catalyzed by pyridine hydrochloride, thereby lowering the chemical potential of acetone on the second side of the barrier.

Simultaneously with the two aforementioned reactions of FIG. 23, $NADP^+$ is reduced with isopropanol upon the first side of the barrier, the reaction being catalyzed by alcohol dehydrogenase to regenerate NADPH and form acetone, thereby raising the chemical potential of acetone on the first side of the barrier. The difference in chemical potentials of acetone on the two sides of the barrier provide an effective driving force for acetone to permeate the barrier in the direction noted in FIG. 23, thereby coupling the first side reactions with the second side trapping reaction.

In FIG. 24, there are shown coupled reactions that use and regenerate the coenzyme NADH to produce the product L-phenylalanine in an analogous fashion to that described above for FIG. 23. The permselective barrier for the coupled reactions of FIG. 22 may be any of the types described in Table IV, eighth entry.

Other exemplary embodiments of membrane-coupled group transfer reactions suitable for the type of use and regeneration scheme shown in FIG. 4 are noted in Table IV.

FIGS. 25-27 depict particular exemplary embodiments of the membrane-coupled reaction types shown in FIG. 5 for the use and regeneration of the coenzymes $NAD(P)^+$.

In FIG. 25, there are shown coupled reactions that use and regenerate the coenzyme NAD+ to produce the product cis-3-oxabicyclo[4.3.0]nonan-2-one. A permselective barrier is again shown bifurcating a reactor into a first and second side, the barrier being selectively permeable to 2-propanol, the membrane comprising the membrane type set forth in Table V, first entry. Two coupled reactions are shown on the first side of the barrier, with a single trapping reaction shown on the second side. On the first side, $NAD^+$ is reduced by the (hydroxymethyl)cyclohexane in the presence of the catalyst noted to form NADH and the corresponding ketone noted in FIG. 23. On the second side, 2-propanol is chemically trapped by reaction with a polymeric phenylisocyanate to form a polymer bound phenyl urea, thereby lowering the chemical

potential of 2-propanol on the second side. Simultaneously with the aforementioned first and second side reactions, NADH is oxidized with acetone in the presence of hydrogen ion and catalyzed by the catalyst noted to regenerate $NAD^+$ and 2-propanol, thereby raising the chemical potential of 2-propanol on the first side of the membrane. The difference in chemical potential of 2-propanol on the two sides of the barrier provides an effective driving force for 2-propanol to diffuse through the permselective barrier in the direction noted in FIG. 25, thereby coupling the reactions on the first side with the reaction on the second side.

In FIG. 26, there are shown coupled reactions that use and regenerate the coenzyme NAD(P)+ to produce the product 11-beta-hydroxycortisol. The coupled reactions and the trapping reaction are as noted in FIG. 26, with hexafluoro-2-propanol permeating the permselective barrier in the direction noted and the permselective barrier being any of the types shown in Table V, third entry.

In FIG. 27, there are shown coupled reactions that use and regenerate the coenzyme $NAD^+$ to produce the product D-fructose in analogous fashion to that discussed in connection with FIGS. 25 and 26, the trapping reaction comprising reacting decafluorobenzhydrol with a polymeric carboxylic acid anhydride

Table V

| Chemical A Red/Ox | Chemical B Red/Ox | Catalyst 1 | Catalyst 2 | Catalyst 3 | Trapping | Trapped Red. Chem. A | Barrier |
|---|---|---|---|---|---|---|---|
| 2-propanol/ acetone | cis-1,2-bis (hydroxy-methyl)cyclo-hexane/cis-3-oxabicyclo [4.3.0] nonan-2-one | horse liver alcohol deh. | horse liver | none | polymeric phenyliso-cyanate | polymer bound phenylurea | A |
| decafluoro benzhydrol/ decafluoro benzophenone | D-mannitol/ D-fructose | D-mannitol deh. | horse liver alcohol deh. | triethyl-amine | polymeric carboxylic acid anhydride | polymer bound half ester | B,D,H,I |
| 1,1,1,3,3,3-hexafluoro-2-propanol/ hexafluoro acetone | 11-deoxy-cortisol/ 11-beta-hydroxy-cortisol | steroid-11-beta-hydroxylase | glycerol deh. | none | sodium hydroxide | 1,1,1,3,3,3-hexafluoro-2-propanoxide anion | A,B,D, H,I |
| phenyltri-fluoromethyl carbinol/ trifluoro acetophenone | D-2-hydroxy-4-methylpentano-ate/2-oxo-4-methylpentano-ate | D-2-hydroxy-4-methyl-pentan-oate deh. | horse liver alcohol deh. | vacuum | cold surface | condensed phenyltri-fluoromethyl carbinol | B,D,H,I |
| 1,1,1,3,3,3-hexafluoro-2-propanol/ hexafluoro acetone | glycerol/ dihydroxy-acetone | glycerol deh. | glycerol deh. | none | sodium hydroxide | 1,1,1,3,3,3-hexafluoro-2-propanoxide anion | A |
| 1,1,1-tri-fluoro 2-propanol/ 1,1,1-tri-fluoro acetone | dehydrocholic acid/cholic acid | hydroxysteroid deh. | yeast alcohol deh. | vacuum | cold surface | condensed 1,1, 1-trifluoro-2-propanol | B,D,H,I |

and catalyzed with triethylamine to form a polymer bound half ester. The permselective barrier may be any of the membrane types shown in Table V, second entry.

Other exemplary embodiments of membrane-coupled group transfer reactions suitable for the type of use and regneration scheme shown in FIG. 5 are noted in Table V.

## EXAMPLES

### Example 1

This example illustrates how the present invention is used to carry out the regiospecific oxidation of D-glucose to aldonic acid shown schematically in FIG. 7 using the strong but inexpensive oxidizing agent, sodium hypochlorite. In a typical procedure, a supported-liquid membrane is prepared by soaking a Celgard 2400 microporous membrane in a mixture of 4-alkyl pyridine (25 wt%) and hexadecane (75 wt%). The supported-liquid membrane is then clamped between the compartments of a bifurcated reactor of the type shown in FIG.6. The first compartment is loaded with 100 ml of an aqueous solution of D-glucose (50 mM) and potassium iodide (10 mM) adjusted to pH 2 by the addition of 1 M sulfuric acid. The second compartment is loaded with 100 ml of an aqueous solution of sodium hypochlorite (100 mM) prepared by passing chlorine gas through dilute sodium hydroxide solution. After the reaction is complete, aldonic acid is recovered as described in 133 J. Biol. Chem. 293 (1940).

### Example 2

This example illustrates how the present invention is used to achieve highly selective oxidations employing catalytic quantities of osmium tetroxide, shown schematically in FIG. 8. Osmium tetroxide is an expensive but highly specific reagent. The scheme also uses stoichiometric quantities of sodium chlorate, an inexpensive but relatively unselective oxidizing agent. A supported-gas membrane consisting of air in the pores of a sheet of Goretex (PTFE, 40 microns thick, 70% porosity, W. L. Gore & Associates), is clamped between the compartments of a bifurcated reactor of the type shown in FIG. 6. The first compartment is loaded with 100 ml of an aqueous solution of maleic acid (200 mM), sodium sulfide (200 mM), and osmium tetroxide (0.50 mM). The second compartment is loaded with 100 ml of an aqueous solution of sodium chlorate (400 mM). When the reaction is complete, the product is isolated from the solution in the first compartment, removed, then filtered to remove elemental sulfur, and the filtrate is then evaporated to yield 2,3-dihydroxy succinic acid.

### Example 3

This example illustrates the use of the present invention for the preparation of peptides as shown schematically in FIG. 9. The membrane pores of a hollow-fiber module (polyvinylidine difluoride fibers in a stainless steel shell with organic-solvent-resistant potting materials made by Bend Research Research, Inc. of Bend, Oregon) are filled with a mixture of a long-chain perfluoroalkane (70 wt%) and a long-chain perfluoroalcohol (30 wt%). A solution containing $AminoAcid_1$-PEG ester (Structure VII in Table B) and imidazole in anhydrous formamide is pumped through the lumens of the hollow fibers of the module. $AminoAcid_1$-PEG ester is prepared by the coupling reaction of benzoyloxycarbonyl-$AminoAcid_1$ acid chloride and PEG followed by the removal of the benzoyloxycarbonyl(BOC) protecting group. A solution containing activated BOC-$AminoAcid_2$-N-(4′-hydroxy-3′-nitro)benzoyl-PEI ester (Structure X in Table B) is pumped through the shell side of the module. BOC-$AminoAcid_2$-N-(4′-hydroxy-3′nitro)benzoyl-PEI ester is prepared in two steps. in the first step, 4′-hydroxy-3′-nitrobenzoic acid is coupled to PEI using dicyclohexylcarbodiimide. And in the second step, BOC-$AminoAcid_2$ in its anhydride form is allowed to react with N-(4′-hydroxy-3′-nitrobenzoyl)-PEI (Structure XI in Table B).

After the dipeptide-forming reaction is complete, the BOC protecting group can be removed from BOC-$AminoAcid_2$-$AminoAcid_1$-PEG ester (Structure VIII in Table B). The dipeptide-PEG ester formed is then used in the next cycle to add another amino acid. When the desired polypeptide sequence has been synthesized, the PEG moiety is removed by the hydrolysis of the ester.

### Example 4

This example illustrates the use of the present invention for the preparation of the expensive coenzyme adenosine triphosphate (ATP) from adenosine diphosphate (ADP) using inexpensive polyphosphoric acid as the phosphoryl donor, as schematically shown in FIG. 10. A supported-liquid membrane is prepared by soaking a Celgard 2500 flat-sheet microporous membrane in a mixture of N-alkyltrimethylammonium dihydrogen phosphate (40 wt%) and hexadecane (60 wt%). This supported-liquid membrane is then clamped between the compartments of a bifurcated reactor of the type shown in FIG. 6. The first compartment is loaded with 100 ml of an aqueous solution of phosphate buffer (50 mM, pH 7.5), ADP (5 mM), magnesium chloride (10 mM), sodium acetate (50 mM), and acetyl phosphate kinase from E. coli (E.C. 2.7.2.1, 50 units). The second compartment is loaded with 100 ml of a mixture consisting of phosphoric acid (50 wt%) and $P_2O_5$ (50 wt%). The ATP-producing capacity of this system can be used to carry out ATP-requiring enzyme-catalyzed transformation, such as suggested by Wong et al in 48 J. Org. Chem. 3199 (1983).

## Example 5

This example illustrates the use of the present invention for carrying out group transfer reactions involving reagents that are normally highly reactive towards each other when used in homogenous solution. The reagents are rendered compatible by isolating them in separate compartments separated by a permselective barrier. The reactants, however, can freely move between the two compartments as a result of the permselectivity of the barrier. The preparation of 1,1,-dideuterio-ethanol serves to illustrate the utility of the invention in this respect.

Current methods for incorporation of deuterium into the C-1 position of primary alcohols involve redox reaction with carboxylic acids as intermediates, as noted below:

$RCH_2OH \rightarrow RCO_2H \rightarrow RCD_2OH$

This approach is satisfactory in many cases; however, it requires the use of strong oxidizing and reducing agents and is not suitable for molecules bearing sensitive functional groups. An alternative approach, partial oxidation to the corresponding aldehyde and reduction back to the alcohol, can be accomplished using milder reagents:

$RCH_2OH \rightarrow RCHO \rightarrow RCHDOH$

However, repeated redox cycles are required to exchange both hydrogens at the C-1 position with deuterium. This is not practical with conventional procedures because significant losses are associated with each redox cycle resulting in unacceptable product yields.

Application of the present invention allows the repeated redox cycles without attendant loss in yield. The process is illustrated in FIG. 11. A sheet of microporous polypropylene (Celgard 2400, Celanese, Charlottsville, North Carolina) is clamped between the two halves of a two-compartment reactor. The first compartment is loaded with 100 ml of a solution containing ethanol (500 mM), sodium dichromate (500 mM), and enough concentrated sulfuric acid to bring the pH down to 1. The second compartment is loaded with 110 ml of a solution containing sodium hydroxide (500 mM, pH 13.5) and sodium borodeuteride (200 mM). The mixtures in each of the two compartments are stirred at room temperature.

Progress of the reaction is monitored by measuring the extent of deuterium incorporation into ethanol. Aliquots are periodically removed from the second compartment and quenched by adjusting the pH to 7 by the addition of sulfuric acid. Short path distillation yields a sample of labeled ethanol. Deuterium content is measured by derivatizing the alcohol by treatment with bis(trimethylsilyl)acetamide in tetrahydrofuran and anlyzing the derivative by gas chromatography-mass spectrometry.

## Example 6

An NADH use and regeneration process of the type illustrated in FIG. 2 is exemplified. A thin-film-composite polymer membrane consisting of a thin dimethyl siloxane film on a non-woven support (manufactured by UOP Fluid Systems Division, San Diego, California) was clamped between the two sections of a reactor of the type shown in FIG. 6. Referring to FIGS. 2 and 13, the following concentrations of reactants in 100 ml of an aqueous solution were added to the first side: 0.2 mM $NAD^+$; 200 mM ethanol

(reduced form of chemical A); 200 mM sodium pyruvate (oxidized form of chemical B); 400 units of D-lactate dehydrogenase (catalyst 1) from Leuconostoc mesenteroides (E.C.1.1.1.28); 200 units of yeast alcohol dehydrogenase (catalyst 2) from yeast (E.C.1.1.1.1, Grade A 7011); 40 mM of a pH 8.3 phosphate buffer; and 1 mM each of EDTA and mercaptoethanol (as an enzyme stabilizer). The following concentrations of reactants in 100 ml of an aqueous solution were added to the second side: 50 mM sodium hydroxide (pH 12); 200 mM ethanol (reduced form of chemical A) and 400 mM of sodium borohydride (sacrificial reducing agent). In such a scheme acetaldehyde corresponds to the oxidized form of chemical A, and D-lactate corresponds to the reduced form of chemical B, while sodium borate corresponds to the oxidized sacrificial reducing agent, and there is no third catalyst. In the membrane-assisted coupled reactions, NADH is continuously used and regenerated to form D-lactate from pyruvate.

The reactions were allowed to proceed for 130 hours and monitored by periodically removing aliquots from the first side and analyzing them for pyruvic acid, ethanol and acetaldehyde. The results of such analysis are shown in FIGS. 29 and 30. The yield of D-lactic acid (D-lactate) from the conversion of pyruvic acid (pyruvate) was greater than 90%, with no significant accummulation of acetaldehyde and the concentrations of NADH and ethanol remaining relatively constant. On the basis of the relative number of moles of NADH present and the number of moles of pyruvate reduced, NADH was regenerated about 1,000 times during the course of the reaction.

To demonstrate the advantages of the membrane-assisted regeneration process and apparatus of the present invention, two control experiments were conducted. In the first one, the same apparatus and reactants were used as noted in the Example above, except that no dehydrogenase enzyme catalysts were included and the process was continued for 190 hours while monitoring the concentration of pyruvic acid only. The result was that the final concentration of pyruvic acid was the same as its initial value. Since it is known that direct contact between sodium borohydride and pyruvate results in immediate and irreversible reduction of pyruvate to D,L-lactic acid, this first control experiment demonstrated that sodium borohydride is not transported across the permselective barrier and that, by inference, the reduction of pyruvate in the Example was not due to leakage of sodium borohydride across the membrane.

In the second control experiment, a mixture of the same reactants placed in the first side of the reactor in the Example were placed in a flask and the concentrations of pyruvic acid, ethanol and acetaldehyde were monitored over a 190 hour period and the results plotted on the same graphs (FIGS. 29 and 30) representing the results of the Example. The results of the second control experiment showed a 15% yield for the pyruvate to lactate conversion, an accumulation of acetaldehyde to a 40 mM concentration, and a 15% consumption of ethanol. Thus, it is apparent that the membrane-assisted regeneration process and apparatus of the present invention increased the yield of lactate from 15% to >90%, prevented the accumulation of acetaldehyde (that results in the irreversible degradation of alcohol dehydrogenase), maintains the concentration of ethanol at a relatively constant value, thereby ensuring maximum efficiency for the catalyst alcohol dehydrogenase, and very substantially increases the rate of conversion of pyruvate to lactate (about a tenfold increase in the first 50 hours).

## Example 7

Another NADH use and regeneration process of the type illustrated in FIG. 2 is exemplified using the same apparatus used in Example 6 except that the membrane used was a Celgard 2400 supported-gas membrane (air being the gas) having the specifications set forth in Table A. In this Example, NADH is continuously used and regenerated to produce L-glutamate from its corresponding alpha-keto acid utilizing the stereospecific reductive amination of 2-ketoglutarate to L-glutamate that is catalyzed by the enzyme glutamate dehydrogenase. Referring to FIGS. 2 and 14, the following concentrations of reactants in 100 ml of an aqueous solution were added to the first side: 0.5 mM NAD+; 150 mM ethanol (reduced form of chemical A); 10 mM 2-ketoglutarate (oxidized form of chemical B); 10 units glutamate dehydrogenase (catalyst 1) from beef liver (E.C.1.4.1.3); 10 units yeast alcohol dehydrogenase (catalyst 2) having the same grade and from the same source as Example 5; 25 mM ammonia; and 100 mM pH 8.0 phosphate buffer. As in Example 6, acetaldehyde corresponds to the oxidized form of chemical A, and L-glutamate to the reduced form of chemical B, while sodium borate corresponds to the oxidized sacrificial reducing agent, and there is no third catalyst. The following concentrations of reactants in 100 ml of an aqueous solution were added to the second side: 150 mM ethanol; 500 mM sodium borohydride (sacrificial reducing agent); and sodium hydroxide to pH 12.5. As in Example 6, aliquots were taken from the first side at regular intervals and analyzed for the disappearance of 2-ketoglutarate. The results are shown as the plot labeled

"Membrane-Assisted" in FIG. 31. Concentration of NADH remained constant.

For comparison, the same amounts and concentrations of the first side reactants were mixed in a flask and incubated for a similar period of time and aliquots taken for analysis of 2-ketoglutarate content. The results are shown also in FIG. 31, by the plot labeled "Without Membrane."

As is apparent from the two plots in FIG. 31, the membrane-assisted process of the present invention results in a steady reduction of 2-ketoglutarate (corresponding to a steady production of L-glutamate), while the non-membrane-assisted reactions result in a progressive loss of L-glutamate productivity due to the inhibition of alcohol dehydrogenase by accumulating acetaldehyde.

## Example 8

The reductive amination of 2-ketoglutarate to form L-glutamate schematically shown in FIG. 14 can also be carried out on a preparative scale employing an inexpensive, NADPH-dependent, high specific activity, glutamate dehydrogenase enzyme obtained from a bacterium of the genus Proteus.

A supported-gas film consisting of the air present in the pores of a microporous polypropylene membrane is used as the permselective barrier, namely, a hollow-fiber module with 1850 $cm^2$ of surface area, manufactured by Hoecht Celanese (Separations Products Division, Charlottsville, North Carolina). One liter of a solution consisting of 2-ketoglutarate (10 mM), ammonium bicarbonate (250 mM, pH 8.3), NADP (0.25 mM), ethanol 150 mM), glutamate dehydrogenase from Proteus (E.C.1.4.1.4, 250 units), and alcohol dehydrogenase from Leuconostoc mesenteroides (E.C.1.1.1.2, 250 units) is recirculated through the lumens of the fibers at a rate of 300 ml/min. One liter of a solution consisting of ethanol (150 mM) and 0.1 wt% platinum oxide-hydrate catalyst made in the manner set forth by Adams et al. in Vol. 1 of Organic Synthesis at page 463 (1941) is circulated at 300 ml/min first through the shell side of the module and then through a reaction chamber through which hydrogen gas is bubbled continuously.

The progress of the reaction is monitored by measuring the disappearance of 2-ketoglutarate. Aliquots are removed at various times, treated with acidic 2,4-dinitrophenyl-hydrazine, and the resulting hydrazone complex separated and quantified by reverse-phase HPLC coupled to UV detection. The progress of the reaction is also monitored by measuring the appearance of L-glutamate. Aliquots are removed at various times, are treated with o-phthalaldehyde and the resulting colored complex is separated and quantified by HPLC coupled to UV detection.

Since 2-ketoglutarate was shown to be an inhibitor of alcohol dehydrogenase ($k_i$ = 13.8 mM), 2-ketoglutarate is fed to the first side of the reactor at a rate commensurate with its consumption. A total of 22 grams (250 mmoles) is added over a two-and-half-day period. After the reaction is complete, the mixture is filtered through a UF membrane (10,000 molecular-weight cutoff) to remove the enzymes. The filtrate is concentrated under vacuum to yield the product, L-glutamate.

## Example 9

Another NADH use and regeneration process of the type illustrated in FIG. 2 is exemplified using the apparatus of Example 6. Here, NADH may be continuously used and regenerated to produce alpha-amino acids from their corresponding alpha-keto acids by reactions catalyzed by alpha-amino acid transaminases. Several recent published patent applications (Rozell, PCT Appl. WO 87/1727; Walter and Sherwin, U.K. Appl 86/GB2161159; and Rozell, EP Appl. 84/1104017) disclose the use of bacterial transaminases to produce alpha-amino acids. However, the production of alpha-amino acids by the disclosed methods involves the stoichiometric consumption of an alpha-amino acid, such as L-glutatmate, and the stoichiometric production of an alpha-keto acid, such as 2-ketoglutarate. The cost of the L-glutamate and the problem of how to dispose of the 2-ketoglutarate are factors that make the disclosed processes uneconomical. This example illustrates how the enzyme glutamate dehydrogenase, readily available from bacterial, yeast, or animal sources, when used by the method of the present invention, offers a straightforward means of recycling the L-glutamate required in the transaminase reaction. The reaction for the overall process is

alpha-keto acid + $H_2$ + $NH_3$ → L-amino acid + $H_2O$.

An example of such a reaction scheme that also uses and regenerates L-glutamate is shown in FIG. 15, which may be used to produce the L-alpha-amino acid L-phenylalanine. To the first side of the apparatus of Example 6 is added 100 ml of an aqueous solution containing the following concentrations of reactants: 0.5

mN NAD+; 150 mM ethanol (reduced form of chemical A); 30 mM phenylpyruvic acid; 10 units of alcohol dehydrogenase (catalyst 2) from yeast (E.C.1.1.1.1, Grade A 7011); 10 units of glutamate dehydrogenase (catalyst 1) from beef liver (E.C.1.4.1.3); 10 mM 2-ketoglutarate; glutamic-phenylpyruvic transaminase comprising 3g of dried cells of Saccharomyces cerevisiae in 100 ml; 25 mM ammonia; and 100 mM pH 8.0 phosphate buffer. The second side of the reactor was loaded with 100 ml of an aqueous solution (pH 7.0) of 100 mM ethanol, 100 mM ferric chloride and 2 wt% platinum oxide, and hydrogen gas was bubbled therethrough by a stainless steel fritted tube. As in previous Examples, aliquots were taken at regular intervals over a 48-hour period and analyzed for the disappearance of phenylpyruvic acid and the appearance of L-phenylalanine, with near quantitative results.

## Example 10

Using the same two-compartment reactor described in Example 6 and a supported-liquid "type D" membrane as the permselective barrier, the reductive amination of pyruvate to the amino acid L-alanine is conducted as shown schematically in FIG. 16. The membrane consisted of a Celgard microporous membrane the pores of which had been filled with a mixture of tri-n-octyl phosphine oxide (33 wt%) and dodecane (67 wt%). The first compartment is loaded with 100 ml of a solution containing ammonium hydroxide (250 mM, pH 10), $NAD^+$ (0.25 mM), cyclohexanol (200 mM), sodium pyruvate (200 mM), 100 units of L-alanine dehydrogenase from Bacillus subtilis (E.C.1.4.1.1), and 100 units of alcohol dehydrogenase from horse liver (E.C.1.1.1.1). The second compartment is loaded with 100 ml of 10 wt% aqueous sodium hydroxide and Raney nickel catalyst (a 50%-sponge nickel-aluminum alloy suplied by Davidson Chemical Division of W. R. Grace). Reaction progress is monitored by measuring the disappearance of pyruvate using an enzyme assay based on lactate dehydrogenase. When the reaction is complete, the product is separated from the enzyme catalysts by means of an ultrafiltration membrane and concentrated under vacuum. The resulting product is recrystallized from ethanol to give pure L-alanine.

## Example 11

Using the same two-compartment reactor and permselective barrier described in Example 6, the regeneration of NADPH as part of a process to produce optically active (S)-2-furyl methyl carbinol is conducted as schematically shown in FIG. 17. (S)-2-furyl methyl carbinol is a useful synthon for the synthesis of alkaloids. The first compartment is loaded with 100 ml of an aqueous solution containing tris hydrochloride buffer (50 mM, pH 7.6), NADP (0.4 mM), 2-propanol (200mM), 2-acetyl furan (200 mM), and alcohol dehydrogenase from Thermoanaerobium brockii (E.C.1.1.1.2, 300 units). The second compartment is loaded with aqueous pH 12 sodium borohydride solution, as in Example 6. Progress of the reaction is monitored by removing aliquots from the first compartment and analyzing them for acetyl furan and (S)-2-furyl methyl carbinol. When the reaction is complete, the product is isolated and characterized as reported by Drueckhammer et al. in 53 J. Org. Chem. 1607 (1988).

## Example 12

An NAD+ use and regeneration process of the type illustrated in FIG. 3 is exemplified. Referring to FIGS. 3 and 18, the membrane-assisted NAD+ regeneration of the present invention is coupled to the horse liver alcohol dehydrogenase-catalyzed oxidation of cis-1, 2-bis(hydroxymethyl)cyclohexane (a diol) to optically active cis-3-oxabicyclo[4.3.0]nonan-2-one (a lactone). Using the apparatus of Example 6, the first side of the reactor was loaded with 100 ml of an aqueous solution containing the following concentrations of components: 2 mM $NAD^+$; 50 mM acetone (oxidized form of chemical A); 10 units alcohol dehydrogenase from horse liver (E.C.1.1.1.1); and 100 mM pH 9 glycine buffer. The second side of the reactor was loaded with 100 ml of an aqueous solution containing 100 mM sodium dichromate (as the sacrificial oxidizing agent) and 50 mM acetone adjusted to pH 1-2 with concentrated sulfuric acid.

The reactions were allowed to proceed over a 340-hour period with aliquots periodically taken from the first side and analyzed for concentrations of acetone, isopropanol, the diol, and the lactone. For comparison,

the same first side components were mixed in a flask and allowed to react for the same period of time and tested for the presence of the same components in the same manner. The results are shown in FIGS. 32-35, comprising plots of the concentrations of the components noted over the course of the reactions. Examination of FIGS. 32-35 show: (1) the concentration of acetone remains relatively constant when the membrane-assisted coupling process and apparatus of the present invention are used but is depleted when they are not used; (2) the concentration of isopropanol remains below that which inhibits the conversion of NADH to NAD+ when the membrane-assisted coupling process and apparatus of the present invention are used but rises to inhibiting concentrations when they are not used; and (3) the diol is quantitatively oxidized to the lactone when the membrane-assisted coupling process and apparatus of the present invention are used but only 60-65% oxidized when they are not.

## Example 13

This example illustrates how the present invention can be used to regenerate $NAD^+$ during the oxidation of D-sorbitol, an inexpensive sugar produced from glucose, into the valuable sweetner, D-fructose, shown schematically in FIG. 19. The permselective barrier comprises a supported-liquid membrane consisting of a mixture of isohexadecanol (15 wt%) and dodecane (85 wt%) in a polypropylene hollow-fiber membrane module with 1 $ft^2$ of area. One liter of a first solution is prepared, comprising glycine buffer (100 mM, pH 8.6), D-sorbitol (100 mM), $NAD^+$ (0.5 mM), decafluorobenzophenone) (10 mM), D-sorbitol dehydrogenase from Candida utilis (E.C.1.1.1.14, 300 units), and alcohol dehydrogenase from horse liver (E.C. 1.1.1.1, 250 units). This solution is pumped through the lumens of the hollow fiber module at a flow rate of 250 ml/min. One liter of a second solution comprising sulfuric acid (0.3 M), hydrogen peroxide (30 wt%), and ferrous sulfate (0.1 M) is prepared and pumped through the shell side of the module at a flow rate of 400 ml/min. The product, D-fructose, can be recovered by removing the enzyme catalysts by ultrafiltration, extracting the filtrate with several portions of hexane to remove residual decafluorobenzophenone, and concentrating the aqueous layer by evaporation under vacuum.

## Example 14

This example illustrates how the present invention can be used to regenerate $NAD^+$ during the oxidation of glycerol, a byproduct of fat hydrolysis, into a value-added product, dihydroxyacetone, shown schematically in FIG. 20. A dense polymer membrane consisting of polyacetylene cast on a Celgard microporous support is clamped between the first and second compartments of a two-compartment reactor. The first compartment is loaded with 100 ml of sodium bicarbonate buffer (100 mM, pH 8), glycerol (50 mM) hexafluoroacetone (1 mM), and 2-mercaptoethanol (0.25 mM). Glycerol dehydrogenase from Enterobacter aerogenes (E.C.1.1.1.6, 65 units) and $NAD^+$ (0.07 mM) are then added. The solution is maintained under argon in a pH range of 7.5 to 8.3. The second compartment is loaded with nitric acid (6 M). The progress of the reaction is monitored by gas chromatography. The product, dihydroxyacetone, is recovered by removing the enzyme catalyst using a 10,000-dalton molecular-weight-cutoff ultrafiltration membrane and concentrating the filtrate under reduced pressure.

## Example 15

This example, shown schematically in FIG. 21, illustrates a method of chemically trapping the product generated during the regeneration of NADH (FIG. 21) for the production of L-carnitine, an expensive substance used clinically in the treatment of lipid storage myopathy. A supported-liquid membrane was prepared by soaking a Celgard 2400 flat-sheet microporous membrane in a mixture comprising 33 wt% Primene (a mixture of $C_{16}$-$C_{22}$ primary amines manufactured by Rohm and Haas of Philadelphia, Pennsylvania) and 67 wt% dodecane. The so-prepared membrane was clamped between the two halves of a bifurcated reactor of the type shown in FIG. 6. Other experiments not discussed herein demonstrated that the permeability of this membrane to phenylpyruvate (first side at pH 7; second side at pH 13) was 2.0 cm/hr; by contrast, the permeability of the membrane to 3-dehydrocarnitine under similar conditions was

less than 0.01 cm/hr. Thus, the membrane exhibits a selectivity greater than 200 for the permeation of phenylpyruvate over 3-dehydrocarnitine. The first side of the reactor is loaded with 100 ml of an aqueous solution containing potassium phosphate buffer (50 mM, pH 7.0), $NAD^+$ (1 mM), dithiothreitol (0.2 mM), carnitine dehydrogenase from Pseudomonas putida (25 units), L-phenyllactate (100 mM), and L-lactate dehydrogenase from rabbit muscle (E.C.1.1.1.28, 75 units). The second compartment is loaded with 100 ml of a 0.1 M solution (pH 13) of potassium hydroxide.

Since 3-dehydrocarnitine is unstable in solution, 3-dehydrocarnitine is added to the first compartment at a rate of 0.5 mmole/hr. After a 90-hour period of addition, L-carnitine is isolatd by passing the solution in the first compartment through an ultrafiltration membrane to remove enzyme catalysts, acidifying the filtrate, and adsorbing the L-carnitine on a column of cation-exchange resin (Bio-Rad AG 50 W-X8, $H^+$ form). The adsorbed L-carnitine is recovered by eluting the column with a solution of 1 M ammonium chloride, concentrating the eluate by evaporation under vacuum, and recrystallizing the product from anhydrous ethanol-acetone.

## Example 16

As noted above, one highly advantageous aspect of the present invention is the transport of oxidized Chemical A produced during the regeneration of NAD(P)H from the first side to the second side thereby removing the inhibition of the catalytic action of catalyst 2. Physically trapping oxidized Chemical A in the second compartment prevents back-transport and comprises another method of enhancing the overall efficiency of the coupled reactions. This example illustrates the use of a mode of transport and trapping referred to as permeation-evaporation or pervaporation, schematically shown in FIG. 22. In a pervaporation mode oxidized Chemical A (here acetaldehyde) diffuses through the permselective barrier and evaporates on the second side. Oxidized Chemical A (acetaldehyde) vapor is trapped on the far side by condensing it on a cold surface with the use of reduced pressure. An exemplary procedure is described below.

A pervaporation test apparatus was set up as shown in FIG. 28 using three permselective barriers. A solution containing the first side components described in Example 6 was added to the feed solution reservoir of the pervaporation test apparatus and recirculated over the surfaces of the permselective barriers at a rate of 100 ml/min. The pressure on the downstream side of each of the membranes was maintained at 0.5 to 2 cmHg using a vacuum pump. Acetaldehyde, ethanol, and water were recovered from permeate vapors by condensation in a liquid-nitrogen cold trap. The progress of the reaction was monitored by measuring the amount of acetaldehyde collected in the cold trap. When the reaction is complete, the product was isolated by using ultrafiltration to remove the catalyst then evaporating the filtrate under reduced pressure.

A wide array of permselective barriers can be used to carry out the process described in this Example. The permeabilities and acetaldehyde-ethanol selectivities of several dense polymer and supported-liquid membranes are summarized in Table C. (Permeability is defined as the rate of solute permeation through 1 $cm^2$ of membrane normalized to the concentration of solute in the feed solution, while selectivity is defined as the ratio of permeabilities.) It is noteworthy that the supported-liquid membranes in Table C exhibited particularly high selectivities.

The removal rate of acetaldehyde, defined as the acetaldehyde flux through the permselective barrier (mass of acetaldehyde transported through 1 $cm^2$ of membrane per minute) depends on the pressure on the second side of the membrane. This phenomenon is illustrated in FIG. 36 (upper graph) for a poly-(dimethyl)siloxane dense polymer membrane. However, it is important to note that the selectivity is relatively insensitive to the pressure on the second side (FIG. 36, lower graph), which means that the second or downstream side is preferentially operated at low pressures.

## Example 17

This example illustrates the use of chemical trapping of the product produced in the regeneration of

Table C

| Membrane Type | Feed-Compartment Conditions | | | Second-Compartment Conditions (cmHg) | Permeability (cm/min) | | Acetaldehyde/Ethanol Selectivity |
|---|---|---|---|---|---|---|---|
| | Acetaldehyde Concentration (mM) | Ethanol Concentration (mM) | Temperature (°C) | | Acetaldehyde | Ethanol | |
| Dense Polymer Membranes | | | | | | | |
| Poly(dimethyl)siloxane | 35 | 60 | 42 | 0.8 | 0.055 | 0.013 | 4.4 |
| Silicone/carbonate copolymer | 35 | 60 | 45 | 0.4 | 0.0050 | 0.0012 | 4.2 |
| Poly(etheramide) block copolymer | 35 | 50 | 42 | 0.4 | 0.0010 | 5.E-04 | 0.52 |
| Cellulose acetate | 28 | 54 | 40 | 0.4 | 1.8E-04 | 3.1E-05 | 5.9 |
| Poly(urethane) | 30 | 60 | 40 | 0.4 | 2.7E-05 | 6.7E-06 | 4.0 |
| Poly(vinylidene chloride) | 30 | 50 | 40 | 0.2 | 4.6E-07 | 6.9E-08 | 6.7 |
| Cellulose triacetate | 24 | 48 | 40 | 0.4 | 2.2E-04 | 3.1E-05 | 7.1 |
| Polyethylene | 24 | 48 | 40 | 1.7 | 1.2E-05 | 1.8E-06 | 6.7 |
| Cellulose acetate butyrate | 30 | 62 | 40 | 2.4 | 4.6E-05 | 2.1E-05 | 2.1 |
| Supported-Liquid Membranes | | | | | | | |
| 1M Primene JM-T* in dodecane | 40 | 200 | 25 | 0.4 | 0.0070 | 2.6E-04 | 25 |
| 1M Adogen 172** in dodecane | 40 | 200 | 25 | 0.4 | 0.0040 | 1.9E-04 | 25 |

*From Rohm and Haas of Philadelphia, PA

**Froom Sherex Chemical Co. of Dublin, OH

NADPH during the reduction of xylose to xylitol, a sweetener used by diabetic patients. The process is schematically illustrated in FIG. 23 and is described below. A dense polymer membrane composed of poly-(dimethyl)siloxane (from UOP Fluid Systems of San Diego, California) is clamped between the two halves of the two-compartment reactor of FIG. 6. The first compartment is loaded with 100 ml of a solution containing glycylglycine buffer (100 mM, pH 7.5), $NADP^{+}$ (3 mM), xylose (50 mM), 2-propanol (200 mM), xylose reductase from Candida pelliculosa (90 units), and alcohol dehydrogenase from Leuconostoc mesenteroides (E.C.1.1.1.2, 200 units). The second compartment is loaded with a solution consisting of pyridine hydrochloride (100 mM) and semicarbazide (saturated). The xylitol is recovered by removing the enzyme catalysts by means of ultrafiltration and concentrating the filtrate by lyophilization.

## Example 18

This example, schematically shown in FIG. 24, illustrates another means of trapping the product resulting from the regeneration of NADH during the reductive amination of phenylpyruvate to form L-phenylalanine, an important amino acid used in the production of the synthetic sweetener Aspartame. The permselective barrier used in this example is the supported-gas membrane in a hollow-fiber configuration described previously in Example 8. A 1-liter solution containing phenylpyruvate (0.5 mM), ammonium bicarbonate (750 mM, pH 9.0), NADH (0.2 mM), 1,1,1-trifluoro-2-propanol (50 mM), L-phenylalanine dehydrogenase from Brevebacterium sp. (250 units), and alcohol dehydrogenase from yeast (E.C.1.1.1.1, 400 units) is circulated through the lumens of the fibers at a rate of 300 ml/min. One liter of a solution consisting of saturated sodium bisulfite in water is circulated at 500 ml/min through the shell side of the module. To minimize the effect of enzyme inhibition due to the presence of excess substrate, phenylpyruvate substrate solution (50 mM) and 1,1,1-trifluoro-2-propanol (50 mM) is slowly added to the first side over the course of the experiment. The progress of the reaction is monitored and the L-phenylalanine isolated as described in Example 8.

## Example 19

This example illustrates the use of chemical trapping of the product generated in the regeneration of $NAD^{+}$ during the oxidation of cis-1,2-bis(hydroxymethyl)-cyclohexane, shown schematically in FIG. 25. A supported-gas membrane of the type described in Example 5 is clamped between the compartments of a two-compartment reactor of the type shown in FIG. 6. The first compartment is loaded with 100 ml of an aqueous solution similar to that described in Example 12. The second compartment is loaded with dry resin particles prepared by the polymerization of 4-vinyl-phenylisocyanate. The product of the oxidation process, cis-3-oxabicyclo[4.3.0]nonan-2-one, is isolated by extracting the aqueous solution in the first compartment with several portions of dichloromethane, drying the organic layer over anhydrous magnesium sulfate, and removing the organic solvent by evaporation.

## Example 20

This example illustrates the use of chemical trapping of the product generated in the regeneration of $NADP^{+}$ during the oxidation of 1-deoxycortisol, shown schematically in FIG. 26. A dense polymer membrane of the type described in Example 6 is clamped between the compartments of a two-compartment FIG. 6-type reactor. The first compartment is loaded with 100 ml of tris buffer (100 mM, pH 7.4), 11-deoxycortisol (20 mM), $NADP^{+}$ (2 mM), hexafluoro-2-propanol (50 mM), steroid 11-beta-hydroxylase from bovine adrenal cortex (E.C.1.14.1.6, 50 units), and glycerol dehydrogenase from Aspergillus niger (E.C.1.1.1.6, 80 units). The contents of the first compartment are stirred to introduce atmospheric oxygen. The second compartment is loaded with 100 ml of an aqueous solution of 0.1 M sodium hydroxide at pH 13. After the reaction is complete, the product (11-beta-hydroxycortisol) is recovered by extracting the contents of the first side with several portions of diethyl ether, drying the organic phase over anhydrous magnesium sulfate, and evaporating the ether under reduced pressure.

Example 21

This example, shown schematically in FIG. 27, illustrates the use of chemical trapping of the product generated in the regeneration of $NAD^+$ during the oxidation of D-mannitol to produce D-fructose, a valuable sweetening agent. In a typical procedure, a supported-liquid membrane prepared by filling the pores of a Celgard 2500 microporous membrane with a mixture of tri-n-octyl-phosphine oxide (25 wt%) and dodecane (75 wt%) is clamped between the compartments of a two-compartment, FIG. 6-type reactor. The first compartment is loaded with 100 ml of an aqueous solution tris buffer (100 mM, pH 5.3), D-mannitol (100 mM), $NAD^+$ (2 mM), decafluorobenzophenone (20 mM), D-mannitol dehydrogenase from Lactobacillus brevis (E.C.1.1.1.67, 50 units), and alcohol dehydrogenase from horse liver (E.C.1.1.1.1, 60 units). The second compartment is loaded with particles of resin, prepared by the polymerization of maleic anhydride, suspended in a mixture of tri-n-octyl phosphine oxide and n-hexane (1:3, wt:wt) and triethylamine (2 wt%). During the course of the reactions, decafluorobenzophenone is added to the first compartment so as to maintain its steady-state concentration at 20 mM. When the reaction is complete, the product D-fructose is isolated by first extracting the contents of the first compartment with several portions of dichloromethane to remove residual decafluorobenzophenone; the aqueous layer is collected and lyophilized to yield D-fructose.

## Claims

1. A process for the use and regeneration of the chemically active form of a reagent characterized in that said use and regeneration takes place in a reactor divided into at least two regions by at least one permselective barrier that is selectively permeable to both the chemically active and the chemically inactive forms of said reagent, said at least two regions comprising a first side and a second side, said process comprising the simultaneous steps of:
(a) allowing said chemically active form of said reagent to react with a first reactant on the first side of said permselective barrier in a group transfer reaction whereby a chemically active group is transferred from said chemically active form of said reagent to said first reactant so as to obtain a first product and the chemically inactive form of said reagent, thereby raising the chemical potential of the inactive form of said reagent on said first side and lowering the chemical potential of the active form of said reagent on said first side; and
(b) allowing said chemically inactive form of said reagent to react with a second reactant on the second side of said permselective barrier in a group transfer reaction whereby said chemically active group is transferred from said second reactant to said chemically inactive form of said reagent so as to obtain said chemically active form of said reagent and a second product, thereby raising the chemical potential of the chemically active form of said reagent on said second side and lowering the chemical potential of the chemically inactive form of said reagent on said second side;
the difference in the chemical potentials of the active and inactive forms of said reagent between, said first side and said second side causing said chemically inactive form of said reagent to permeate said permselective barrier from the first side thereof to the second side thereof, and further causing said chemically active form of said reagent to permeate said permselective barrier from the second side thereof to the first side thereof.

2. The process of claim 1 wherein the reactions in steps (a) and (b) are catalyzed by a first catalyst and a second catalyst, respectively.

3. The process of claim 1 wherein said chemically active group transferred is selected from an electron, a hydride group, a bromonium ion, a chloronium ion, an oxononium ion, a methyl group, an alkyl group, an acyl group, an acetyl group, an alkylsulfonyl group, an arylsulfonyl group, a benzoyl group, an alkylphosphoryl group, a dialkyl phosphoryl group, an oxycarbonyl group, and a phosphoryl group.

4. The process of claim 1 wherein the respective chemically active/chemically inactive forms of said reagent are selected from iodine/iodide ion, ferric ion/ferrous ion, methane thiol/bis-methane thiol, ruthenium tetroxide/ruthenium dioxide, ceric ion/cerous ion, bromine/bromide ion, selenium dioxide/selenium oxide, chlorine/chloride ion, osmium tetroxide/osmium dioxide, acetyl phosphate/acetate anion, N-acetyl imidazole/imidazole, N-benzoyl imidazole/imidazole, acyl imidazole/imidazole, polyethyleneglycol-acylimidazole/polyethyleneglycol-imidazole, N-methyl-4-dialkylamino pyridinium cation/4-(dialkylamino) pyridine,N-acetyl-4-(dialkylamino)pyridinium cation/4-(dialkylamino)pyridine, N-isobutoxycarbonyl-4-dialkylaminopyridinium cation/4-(dialkylamino) pyridine, N-(P-toluenesulfonyl)-4-dialkylaminopyridinium cation/4-(dialkylamino)pyridine, N-(dialkylphosphoryl)-4-dialkylaminopyridinium cation/4-(dialkylamino)pyridine, and 2,3-dicyano-5,6-dichloroquinone/2,3-dicayno-5,6-dichloro-hydroquinone.

5. The process of claim 1 wherein said first product is selected from reduced lignin, aldonic acid, 2-ketoaldonic acid, reduced glutathione, 5,6-diketo decane, p-methoxy-benzaldehyde, adiponitrile, acrylic acid, 3-chloro-butan-2-one, acetic acid, benzaldehyde, 3,5-dibromophenyl tyrosine, 2,3-dichlorocinnamaldehyde, cyclohexane-1,2-diol, 2,3-dihydroxy succinic acid, methyl thiophenolate, acetyl coenzyme A, O-acetyl linalool, N-(benzoyl)benzylamine, N-(isobutyl)formanilide, N-(p-toluenesulfonyl)aniline, adenosine triphosphate, N-(dialkylphosphoryl)benzyl amine, 3-(3′,4′-dimethoxyphenyl)-3-keto-1-propan-1-ol, 3-keto-di-O-isopropylidine-D-glucofuranose, benzoyloxycarbonyl-AminoAcid$_2$-AminoAcid$_1$-polyethyleneglycol ester, and benzoyloxycarbonyl-AminoAcid$_1$-AminoAcid$_2$-phenylalkyl ester.

6. The process of claim 1 wherein said second product is selected from nitrous acid, sodium borate, sodium chloride, chloride ion, sodium iodate, manganese dioxide, sodium iodite, sodium bromite, sodium chlorite, hydrogen chloride, acetic acid, N-(4′-hydroxy-3′-nitrobenzoyl)-polythyleneimine, and 4-(N-polyethyleneglycol-N-methyl)aminopyridine.

7. The process of claim 1 wherein in the chemically active reagent comprises the coenzymes NAD(P)H and said use and regeneration takes place in a reactor divided into at least two regions by at least one permselective barrier that is selectively permeable to both the oxidized and reduced forms of chemical A, said at least two regions comprising a first side and a second side, said process comprising the simultaneous steps of:

(a) oxidizing NAD(P)H on the first side of said permselective barrier by the oxidized form of chemical B in the presence of hydrogen ions and a first catalyst so as to obtain NAD(P)+ and a desired product comprising the reduced form of chemical B;

(b) reducing the oxidized form of chemical A on the second side of said permselective barrier with a sacrificial reducing agent that is a stronger reducing agent than the reduced form of chemical A, so as to obtain the reduced form of chemical A, thereby raising the chemical potential of the reduced form of chemical A on said second side and lowering the chemical potential of the oxidized form of chemical A on said second side; and

(c) reducing $NAD(P)^+$ on the first side of said permselective barrier with the reduced form of chemical A in the presence of a second catalyst so as to obtain NAD(P)H and the oxidized form of chemical A, thereby raising the chemical potential of the oxidized form of chemical A on said first side and lowering the chemical potential of the reduced form of chemical A on said first side;

the difference in the chemical potentials of the oxidized and reduced forms of chemical A between said first side and said second side causing the oxidized form of chemical A to permeate said permselective barrier from the first side thereof to the second side thereof and the reduced form of chemical A to permeate said permselective barrier from the second side thereof to the first side thereof, wherein chemical A comprises a redox couple and is a substrate for said second catalyst, and the thermodynamics of said process strongly favor the formation of the reduced form of chemical B and an oxidized form of said sacrificial reducing agent over the oxidized form of chemical B and the reduced form of said sacrificial reducing agent.

8. The process of claim 1 wherein the chemically reactive reagent comprises the coenzymes $NAD(P)^+$ and said use and regeneration takes place in a reactor divided into at least two regions by at least one permselective barrier that is selectively permeable to both the oxidized and reduced forms of chemical A, said at least two regions comprising a first side and a second side, said process comprising the simultaneous steps of:

(a) reducing $NAD(P)^+$ on the first side of said permselective barrier by the reduced form of chemical B in the presence of a first catalyst so as to obtain NAD(P)H and a desired product comprising the oxidized form of chemical B;

(b) oxidizing the reduced form of chemical A on the second side of said permselective barrier with a sacrificial oxidizing agent that is a stronger oxidizing agent than the oxidized form of chemical A so as to obtain the oxidized form of chemical A, thereby raising the chemical potential of the oxidized form of chemical A on said second side and lowering the chemical potential of the reduced form of chemical A on said, second side; and

(c) oxidizing NAD(P)H on said first side of said permselective barrier with the oxidized form of chemical A in the presence of a second catalyst so as to obtain $NAD(P)^+$ and the reduced form of chemical A, thereby raising the chemical potential of the reduced form of chemical A on said first side and lowering the chemical potential of the oxidized form of chemical A on said first side;

the difference in the chemical potentials of the oxidized and reduced forms of chemical A between said first side and said second side causing the reduced form of chemical A to permeate said permselective barrier from the first side thereof to the second side thereof and causing the oxidized form of chemical A to

permeate said permselective barrier from the second side thereof to the first side thereof, wherein chemical A comprises a redox couple and is a substrate for said second catalyst, and the thermodynamics of said process strongly favor the formation of the oxidized form of chemical B and the reduced form of said sacrificial oxidizing agent over the reduced form of chemical B and the oxidized form of said sacrificial oxidizing agent.

9. The process of claim 7 or 8 wherein said first and second catalyst are NAD(P) coenzyme-linked oxidoreductase enzymes.

10. The process of claim 7 or 8 wherein step (b) is conducted in the presence of a third catalyst.

11. The process of claim 7 or 8 wherein chemical A and chemical B are members of a redox pair.

12. The process of claim 7 wherein, the respective oxidized/reduced forms of chemical A are selected from acetaldehyde/ethanol, acetone/isopropanol, cyclohexanone/cyclohexanol, hexafluoroacetone/1,1,1,3,3, 3-hexafluoro-2-propanol, heptafluoro-n-butyraldehyde/2,2, 3,3,4,4,4-heptafluoro-1-butanol, cyclopentanone/cyclopentanol, oxaloacetate/D,L-malate, dihydroxyacetone/glycerol, decafluorobenzophenone/decafluorobenzhydrol, and pentafluorobenzaldehyde/pentafluorobenzyl alcohol.

13. The process of claim 7 wherein the respective oxidized/reduced forms of chemical B are selected from pyruvate/D-lactate, 2-ketoglutarate/L-glutamate, xylose/xylitol, pyruvate/L-alanine, L-7,8-dihydrobiopterin/6(R)-L-erythro-5,6,7,8-tetrahydrobiopterin, alpha-keto isocaproate/L-leucine, dihydrofolic acid/tetrahydrofolic acid, phenylpyruvic acid/L-phenylalanine, 3-dehydrocarnitine/L-carnitine, beta-chloropyruvic acid/D-beta-chlorolactic acid, 3-chloro2-oxo-butanoic acid/3-chloro-2-hydroxy-butanoic acid, dehydrocholic acid/cholic acid, ketopanto lactone/(3R)-dihydro-4,4-diethyl-3-hydroxy-2-furanone, cinnamaldehyde/cinnamyl alcohol, and 2-acetyl furan/(S)-2-furanyl methyl carbinol.

14. The process of claim 7 wherein said sacrificial reducing agent of step (b) is selected from borohydride, cyanoborohydride, dithionite, hydrogen, iron and nickel-aluminum alloy.

15. The process of claim 8 wherein the respective reduced/oxidized forms of chemical A are selected from 2-propanol/acetone, decafluorobenzhydrol/decafluorobenzophenone, cyclopentanol/cyclopentanone, 1,1,1,3,3,3-hexafluoro-2-propanol/hexafluoroacetone, phenyl-trifluoromethyl carbinol/trifluoroacetophenone, 1,1,1,3,3,3-hexafluoro-2-propanol/hexafluoroacetone, 1,1,1-trifluoro-2-propanol/1,1,1-trifluoroacetone, and 1,1,1,3,3,3-hexafluoro-2propanol/hexafluoroacetone.

16. The process of claim 8 wherein the respective reduced/oxidized forms of chemical B are selected from cis-1,2-bis(hydroxymethyl)cyclohexane/cis-3-oxabicyclo[4.3.0] nonan-2-one, D-sorbitol/L-sorbose, D-mannitol/D-fructose, 1,2-dehydro-3-ketosteroids/3-ketosteroids, D-2-hydroxy-4-methylpentanoate/2-oxo-4-methylpentanoate, glycerol/dihydroxy-acetone, dehydrocholic acid/cholic acid, bridged-bicylo-meso-diols/bridged-bicyclolactones, and meso-cis-1,2-bis(hydroxymethyl)-cyclobutane/cis-3-oxabicyclo[3.2.0]-heptan-2-one.

17. The process of claim 8 wherein said sacrificial oxidizing agent of step (b) is selected from dichromate, hydrogen peroxide, oxygen, periodate, nitric acid, permanganate, lead tetra acetate and hypochlorite.

18. The process of claim 7 characterized in that the following step is substituted for step (b):
chemically or physically trapping on the second side of said permselective barrier the oxidized form of chemical A so as to lower the chemical potential of the oxidized form of chemical A on the second side of said permselective barrier;
and further characterized in that the difference in the chemical potentials of chemical A between the first side and the second side of said permselective barrier causing the oxidized form of chemical A to permeate said permselective barrier from the first side thereof to the second side thereof.

19. The process of claim 18 wherein the reduced form of chemical A is replenished.

20. The process of claim 18 wherein step (b) comprises allowing said oxidized form of chemical A to react irreversibly with an agent selected from a complexing agent and a polymeric agent.

21. The process of claim 18 wherein step (b) comprises condensation, absorption or adsorption of said oxidized form of chemical A.

22. The process of claim 18 wherein chemical A and chemical B are members of a redox pair.

23. The process of claim 18 wherein the respective oxidized/reduced forms of chemical A are selected from acetaldehyde/ethanol, acetone/isopropanol, cyclopentanone/cyclopentanol, cyclohexanone/cyclohexanol, hexafluoroacetone/1,1,1,3,3,3-hexafluoro-2-propanol, 2,2,3,3,4,4,4-heptafluoro-1-butenal/2,2,3,3,4,4,4-heptafluoro-1-butanol, trifluoroacetone/1,1,1-trifluoro-2-propanol, phenylpyruvate/L-phenyllactate, dihydroxy acetone/glycerol, decafluorobenzophenone/decafluorobenzhydrol, pentafluorobenzaldehyde/pentafluorobenzyl alcohol, and oxaloacetate/D,L-malate.

24. The process of claim 18 wherein the respective oxidized/reduced forms of chemical B are selected from pyruvate/D-lactate, 2-ketoglutarate/L-glutamate, xylose/xylitol, pyruvate/L-alanine, L-7,8-dihydrobiopterin/6(R)-L-erythro-5,6,7,8-tetrahydrobiopterin, alpha-keto-isocaproate/L-leucine, dihydrofolic acid/tetrahydrofolic acid, phenylpyruvic acid/L-phenylalanine, 3-dehydrocarnitine/L-carnitine, beta-chloropyruvic acid/D-beta-chlorolactic acid, 3-chloro-2oxobutanoic acid/3-chloro-2-hydroxybutanoic acid, dehydrocholic acid/cholic acid, and, cinnamaldehyde/cinnamyl alcohol.

25. The process of claim 18 wherein said first and second catalysts are NAD(P) coenzyme-linked oxidoreductase enzymes.

26. The process of claim 8 characterized in that the following step is substituted for step (b):
chemically or physically trapping on the second side of said permselective barrier said reduced form of chemical A, thereby lowering the chemical potential of the reduced form of chemical A on the second side of said permselective barrier;
and further characterized in that the difference in the chemical potentials of chemical A between the first side and the second side of said permselective barrier causing the reduced form of chemical A to permeate said permselective barrier from the first side thereof to the second side thereof.

27. The process of claim 26 wherein the oxidized form of chemical A is replenished.

28. The process of claim 26 wherein step (b) comprises allowing said reduced form of chemical A to react irreversibly with an agent selected from a complexing agent and a polymeric agent.

29. The process of claim 26 wherein step (b) comprises condensation, absorption or adsorption of said reduced form of chemical A.

30. The process of claim 26 wherein chemical A and chemical B are members of a redox pair.

31. The process of claim 26 wherein the respective reduced/oxidized forms of chemical A are selected from 2-propanol/acetone, decafluorobenzhydrol/decafluorobenzophenone, 1,1,1,3,3,3-hexafluoro-2-propanol/hexafluoroacetone, phenyltrifluoromethyl carbinol/trifluoroacetophenone, and 1,1,1-trifluoro-2-propanol/1,1,1-trifluoroacetone.

32. The process of claim 26 wherein the respective reduced/oxidized forms of chemical B are selected from cis-1,2-bis(hydroxymethyl)cyclohexane/cis-3-oxabicyclo[4.3.0]nonan-2-one, D-mannitol/D-fructose, 1,2-dehydro-3-ketosteroids/3-ketosteroids, D-2-hydroxy-4-methylpentanoate/2-oxo-4-methylpentanoate, glycerol/dihydroxyacetone, and dehydrocholic acid/cholic acid.

33. The process of claim 26 wherein said first and second catalysts are NAD(P) coenzyme-linked oxidoreductase enzymes.

34. The process of claims 1, 7, 8, 18 or 26 wherein said permselective barrier is selected from a supported-gas membrane, a supported-liquid membrane, a cast polymer membrane, a thin-film composite membrane, a liquid-swollen polymer membrane, and a series of membrane contactors.

35. The process of claims 1, 7, 8, 18 or 26 wherein said permselective barrier itself contains no reactive components other than those to which it is permeable.

36. The process of claims 1, 7, 8, 18 or 26 wherein said process is continuous.

37. The process of claims 1, 7, 8, 18 or 26 wherein said permeation of said permselective barrier is by a mechanism selected from diffusive transport, carrier-mediated transport, coupled transport, and pervaporation.

FIG.1.

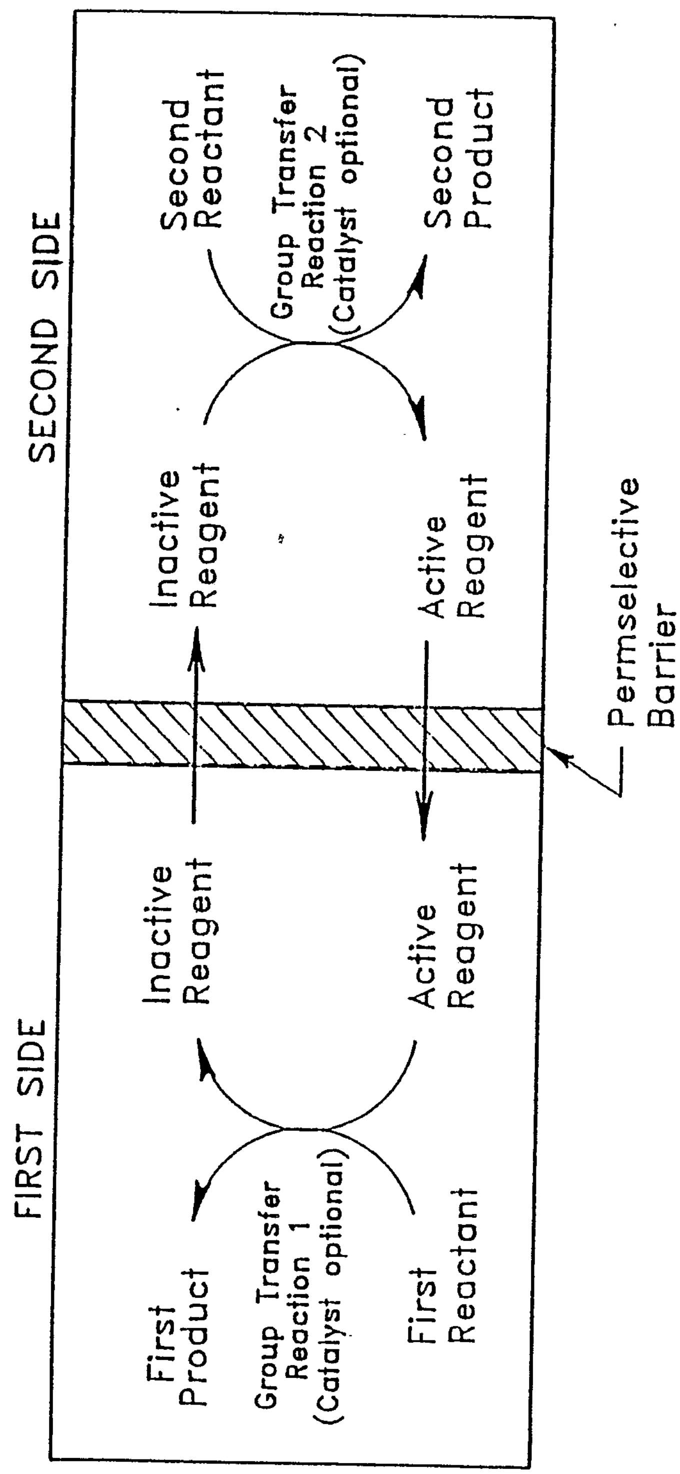
FIRST SIDE
SECOND SIDE
First Product
Group Transfer Reaction 1 (Catalyst optional)
First Reactant
Inactive Reagent
Active Reagent
Inactive Reagent
Active Reagent
Second Reactant
Group Transfer Reaction 2 (Catalyst optional)
Second Product
Permselective Barrier

FIG. 2.

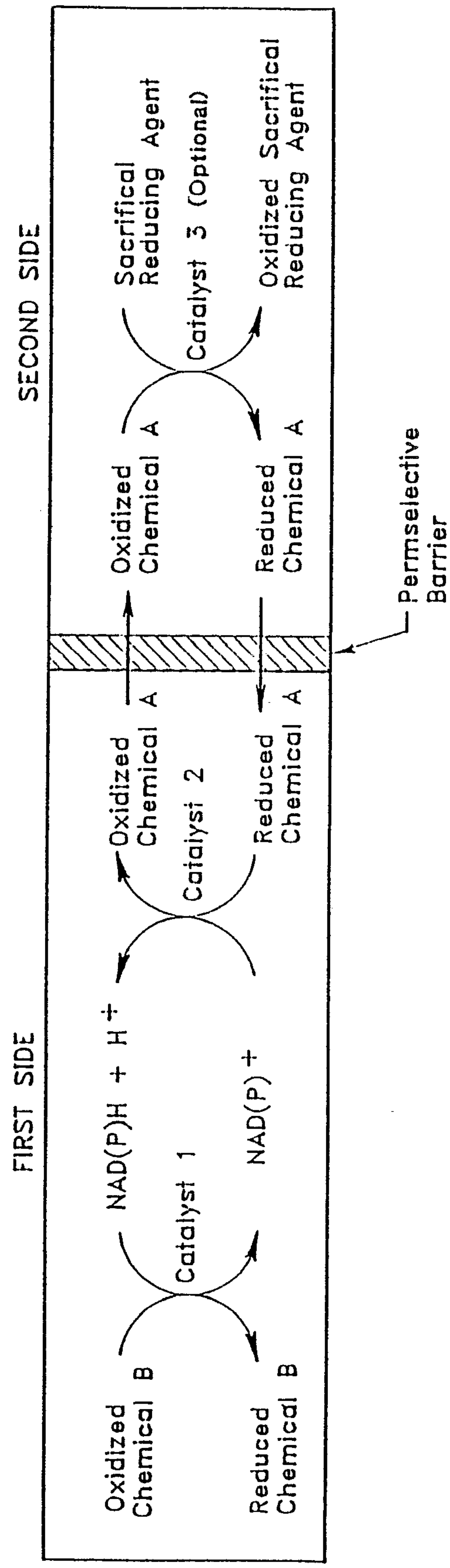
FIRST SIDE
SECOND SIDE
Oxidized Chemical B
Reduced Chemical B
Catalyst 1
NAD(P)H + H+
NAD(P)+
Catalyst 2
Oxidized Chemical A
Reduced Chemical A
Oxidized Chemical A
Reduced Chemical A
Catalyst 3 (Optional)
Sacrifical Reducing Agent
Oxidized Sacrifical Reducing Agent
Permselective Barrier

FIG. 3.

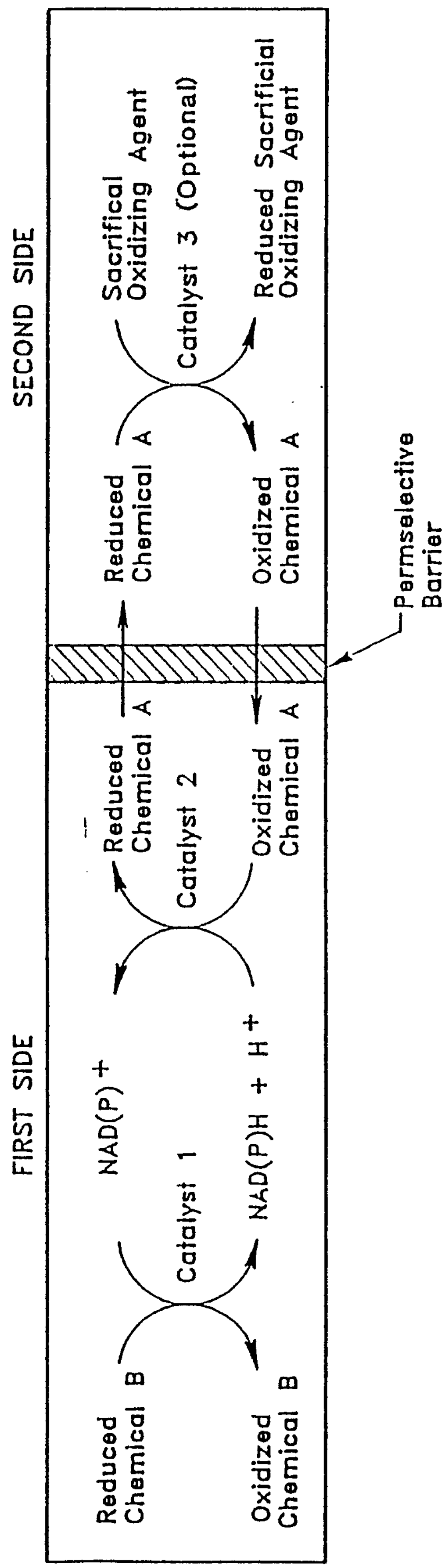
FIRST SIDE
SECOND SIDE
Reduced Chemical B
Oxidized Chemical B
Catalyst 1
NAD(P)+
NAD(P)H + H+
Catalyst 2
Reduced Chemical A
Oxidized Chemical A
Permselective Barrier
Reduced Chemical A
Oxidized Chemical A
Catalyst 3 (Optional)
Sacrifical Oxidizing Agent
Reduced Sacrificial Oxidizing Agent

FIG. 4

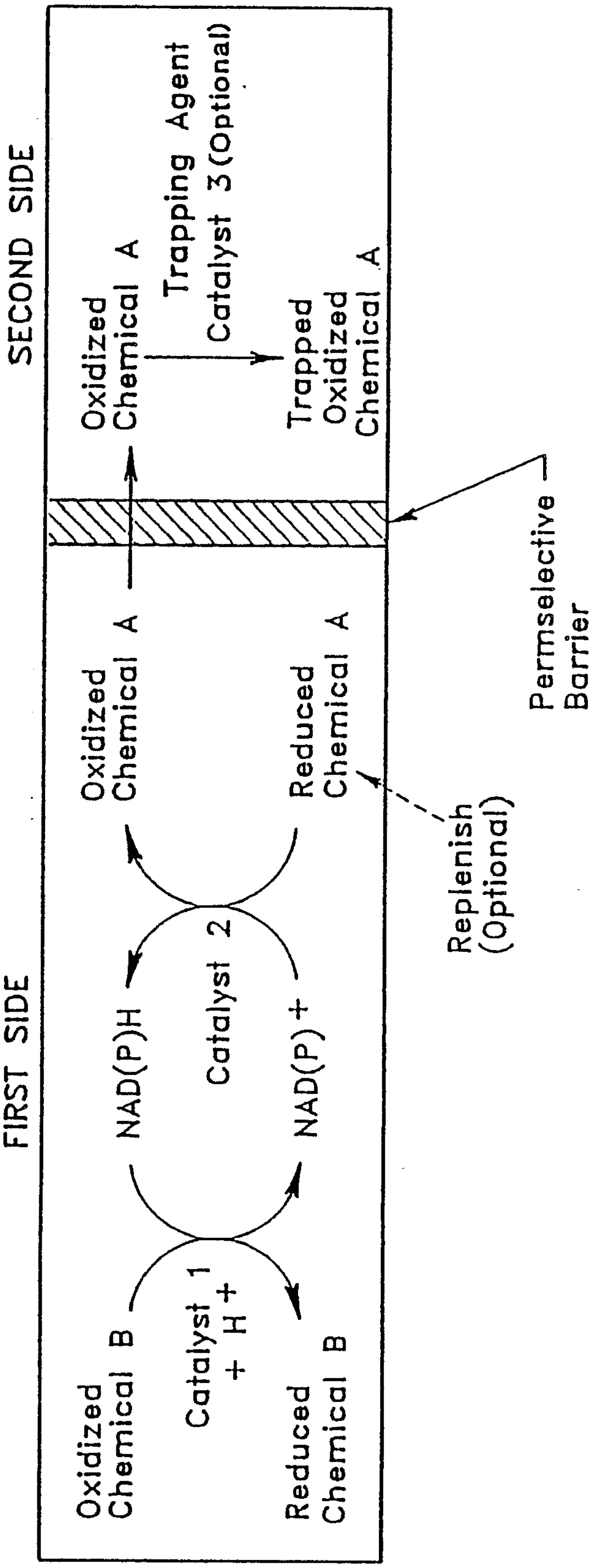
FIRST SIDE
SECOND SIDE
Oxidized Chemical B
Catalyst 1 + H +
Reduced Chemical B
NAD(P)H
Catalyst 2
NAD(P) +
Oxidized Chemical A
Reduced Chemical A
Oxidized Chemical A
Trapping Agent
Catalyst 3 (Optional)
Trapped Oxidized Chemical A
Permselective Barrier
Replenish (Optional)

FIG.5.

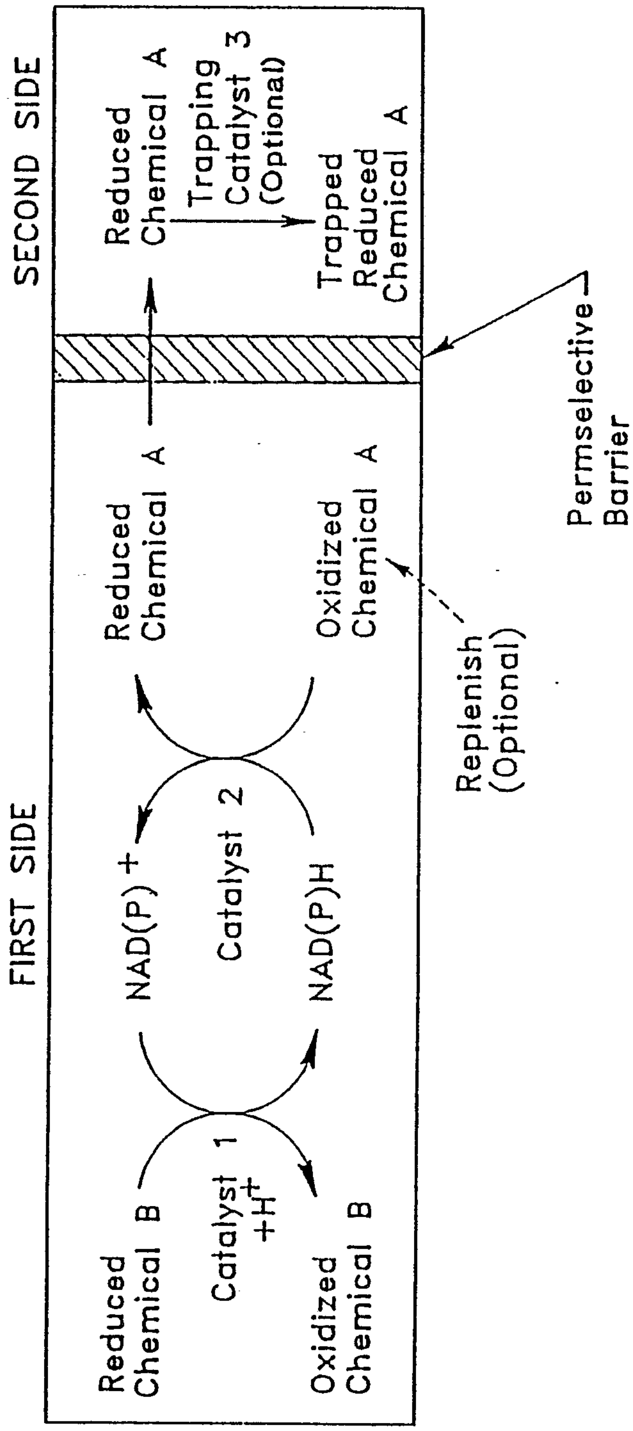
FIRST SIDE
SECOND SIDE
Reduced Chemical B
Catalyst 1 +H+
Oxidized Chemical B
NAD(P)+
Catalyst 2
NAD(P)H
Reduced Chemical A
Oxidized Chemical A
Replenish (Optional)
Reduced Chemical A
Trapping Catalyst 3 (Optional)
Trapped Reduced Chemical A
Permselective Barrier

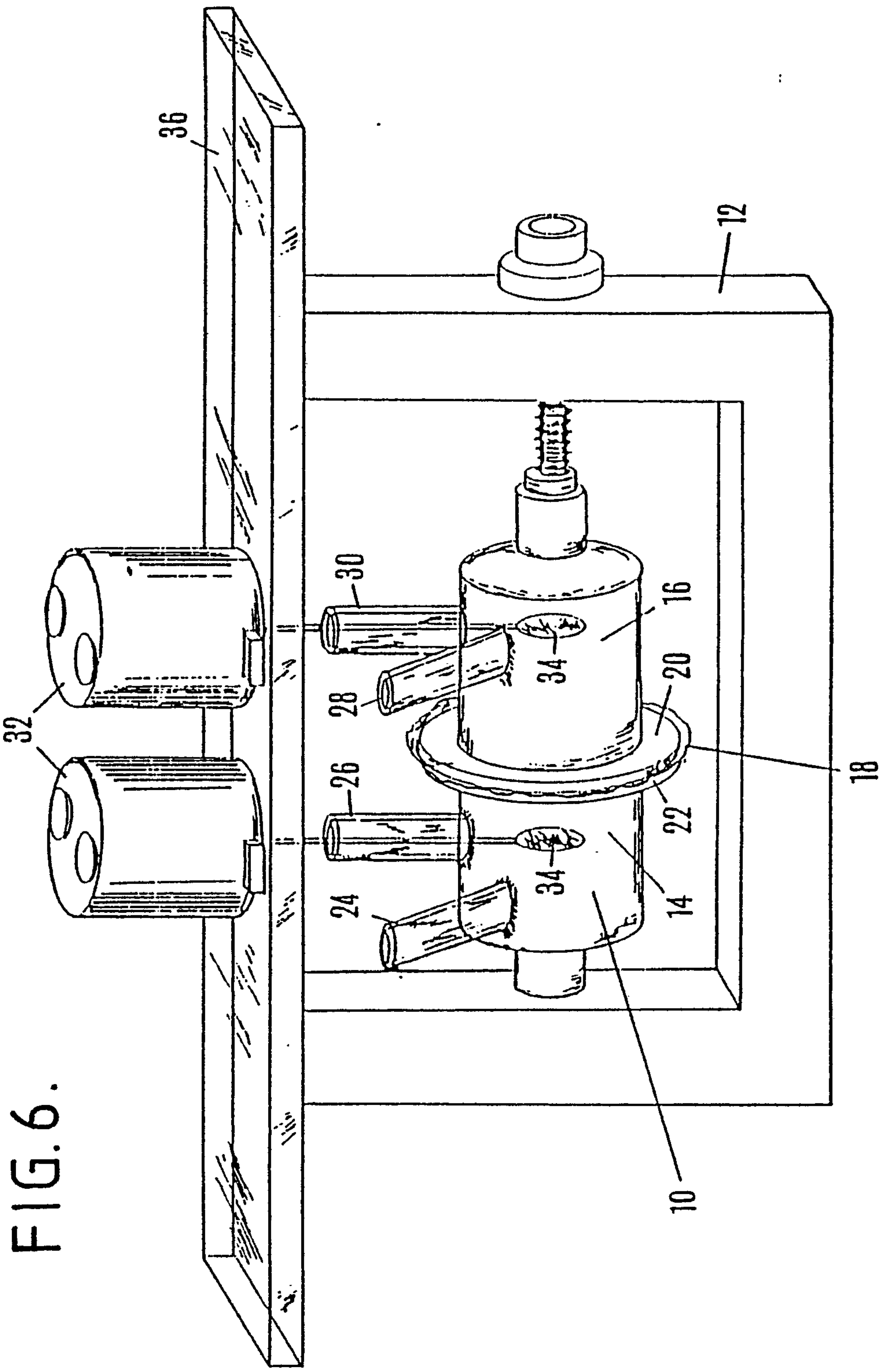


FIG.6.

FIG.7.

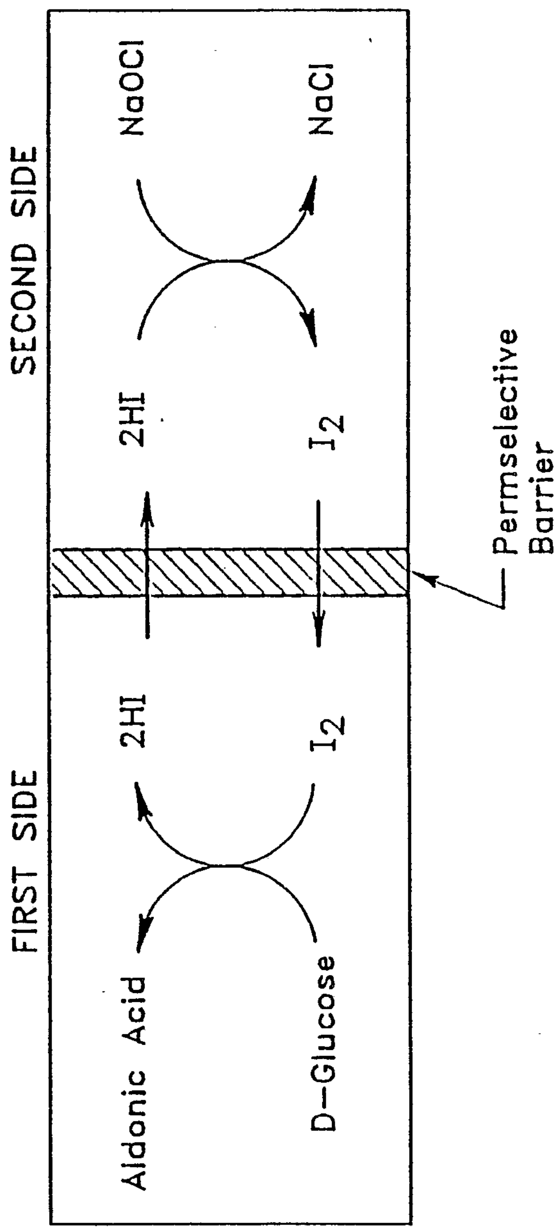
FIRST SIDE
SECOND SIDE
Aldonic Acid
D-Glucose
2HI
$I_2$
2HI
$I_2$
NaOCl
NaCl
Permselective Barrier

FIG.8.

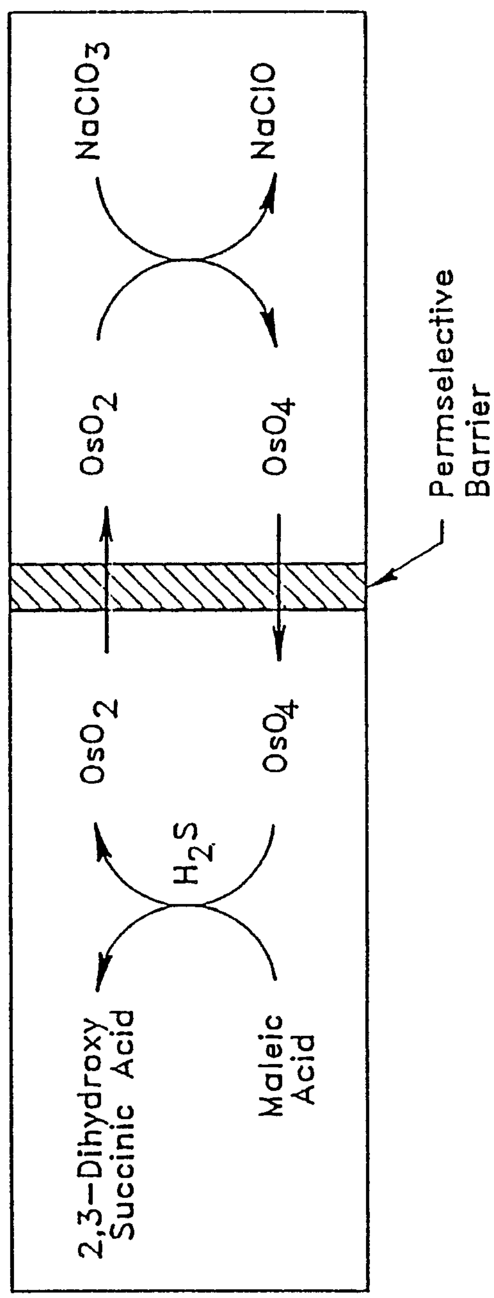
FIRST SIDE
SECOND SIDE
2,3-Dihydroxy Succinic Acid
Maleic Acid
H2S
OsO2
OsO4
OsO2
OsO4
NaClO3
NaClO
Permselective Barrier

FIG.9.

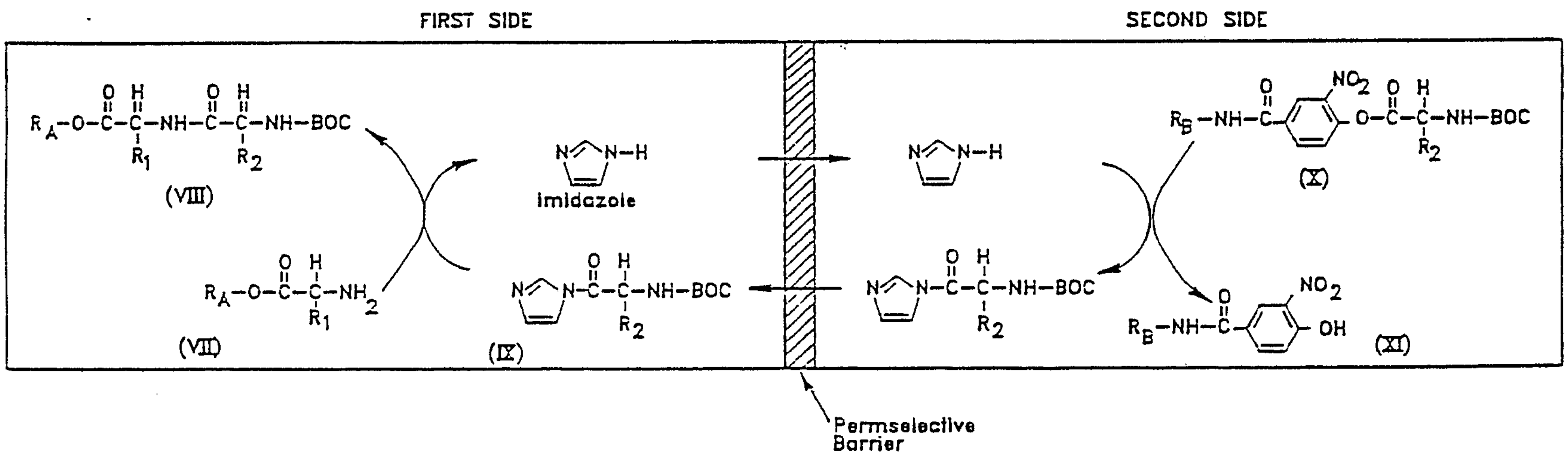
FIRST SIDE
SECOND SIDE
Imidazole
Permselective Barrier
(VII)
(VIII)
(IX)
(X)
(XI)

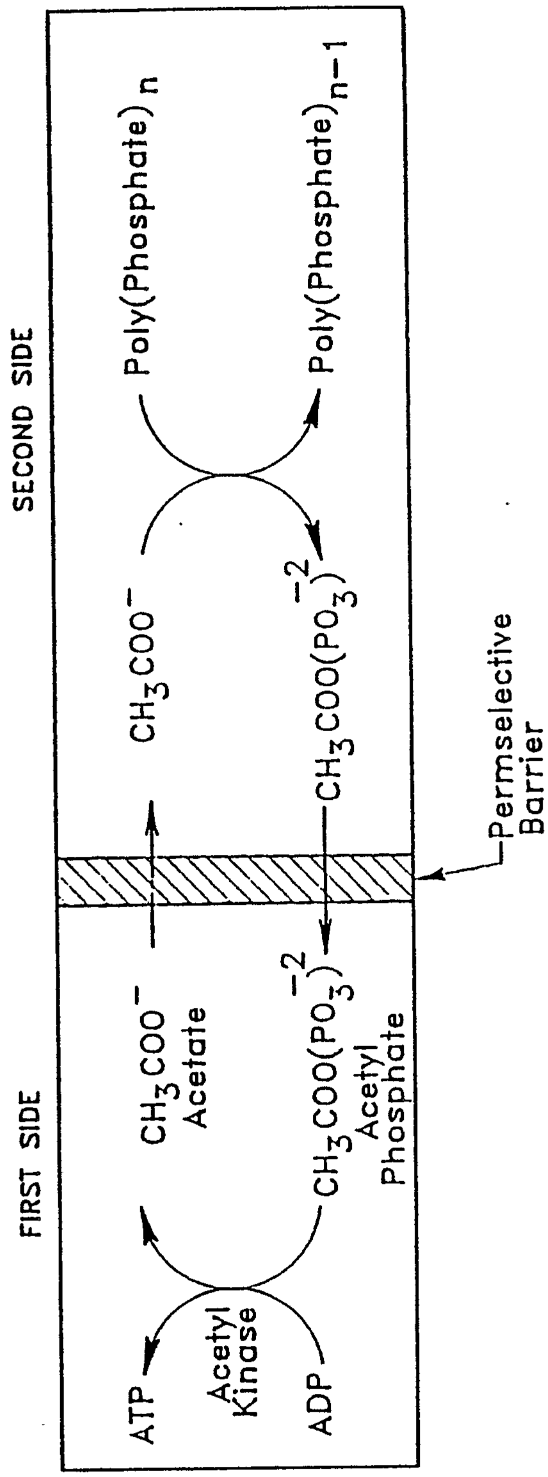
FIRST SIDE
SECOND SIDE
ATP
Acetyl Kinase
ADP
$CH_3COO^-$
Acetate
$CH_3COO(PO_3)^{-2}$
Acetyl Phosphate
$CH_3COO^-$
$CH_3COO(PO_3)^{-2}$
Poly(Phosphate)$_n$
Poly(Phosphate)$_{n-1}$
Permselective Barrier

FIG.10.

FIG. 11.

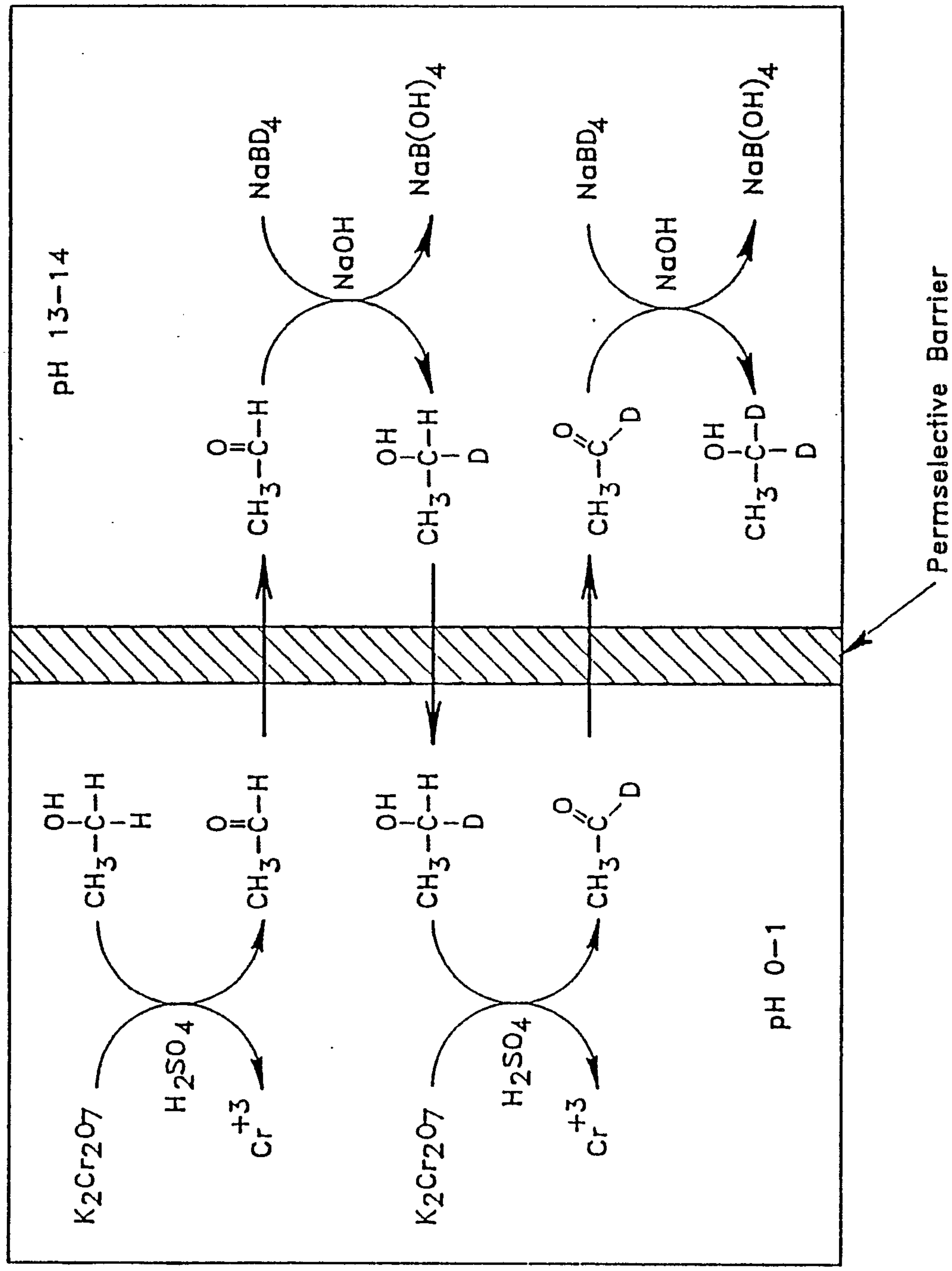
pH 0–1
pH 13–14
$K_2Cr_2O_7$
$H_2SO_4$
$Cr^{+3}$
$NaBD_4$
NaOH
$NaB(OH)_4$
Permselective Barrier

FIG.12.

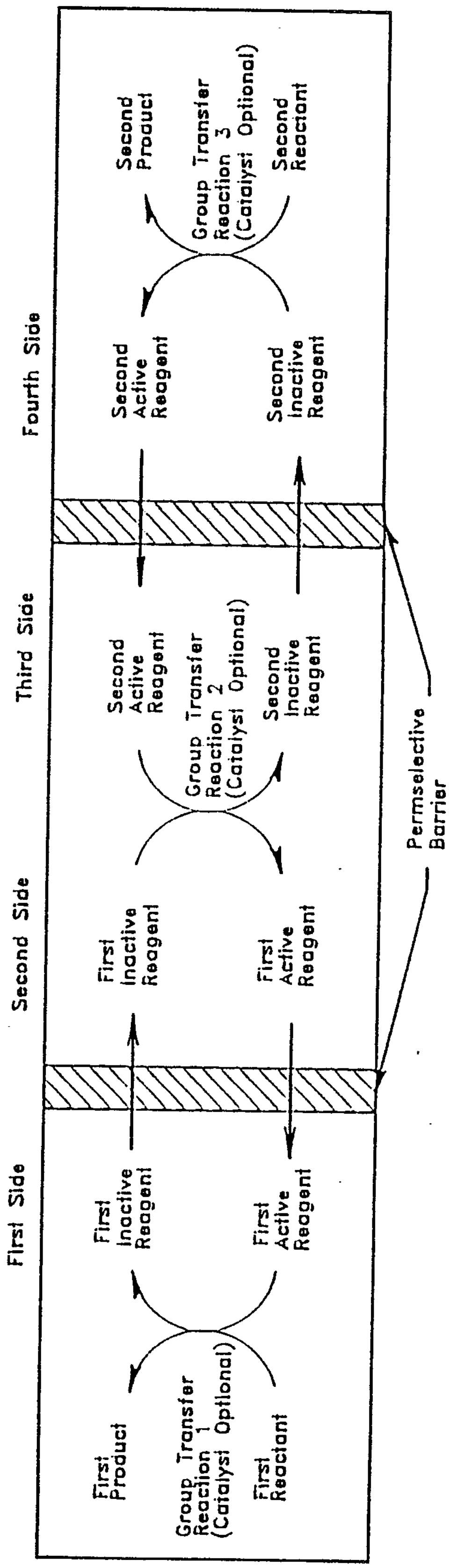
First Side
Second Side
Third Side
Fourth Side
First Product
Group Transfer Reaction 1 (Catalysi Optional)
First Reactant
First Inactive Reagent
First Active Reagent
First Inactive Reagent
First Active Reagent
Group Transfer Reaction 2 (Catalysi Optional)
Second Active Reagent
Second Inactive Reagent
Second Active Reagent
Second Inactive Reagent
Group Transfer Reaction 3 (Catalysi Optional)
Second Product
Second Reactant
Permselective Barrier

FIG. 13.

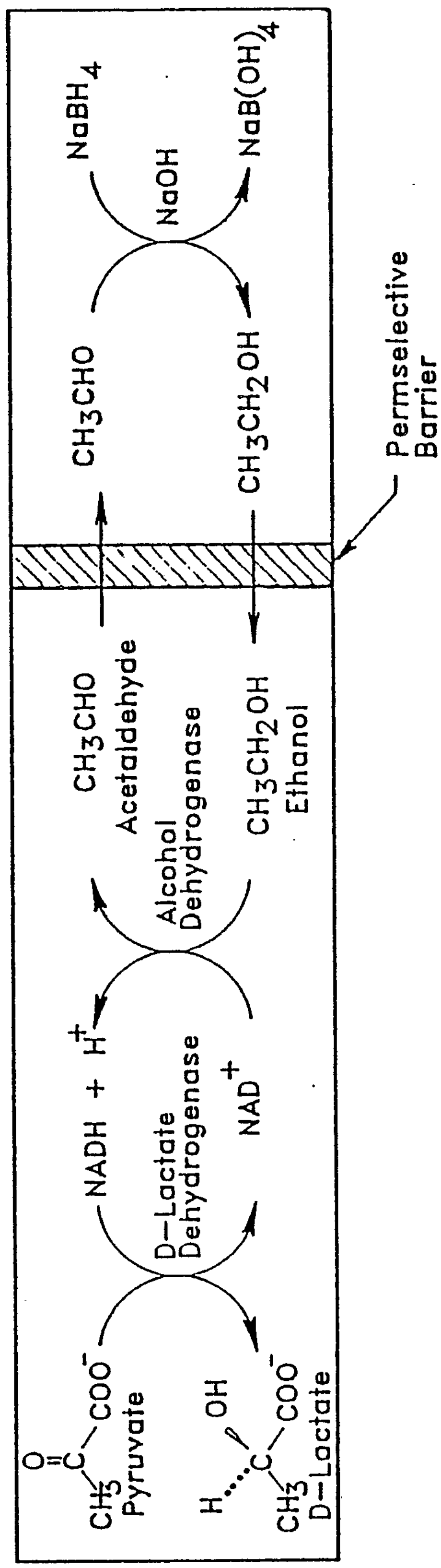
FIRST SIDE
SECOND SIDE
Pyruvate
D-Lactate
D-Lactate Dehydrogenase
NADH + $H^+$
$NAD^+$
Alcohol Dehydrogenase
Acetaldehyde
Ethanol
$NaBH_4$
NaOH
$NaB(OH)_4$
Permselective Barrier

FIG.14.

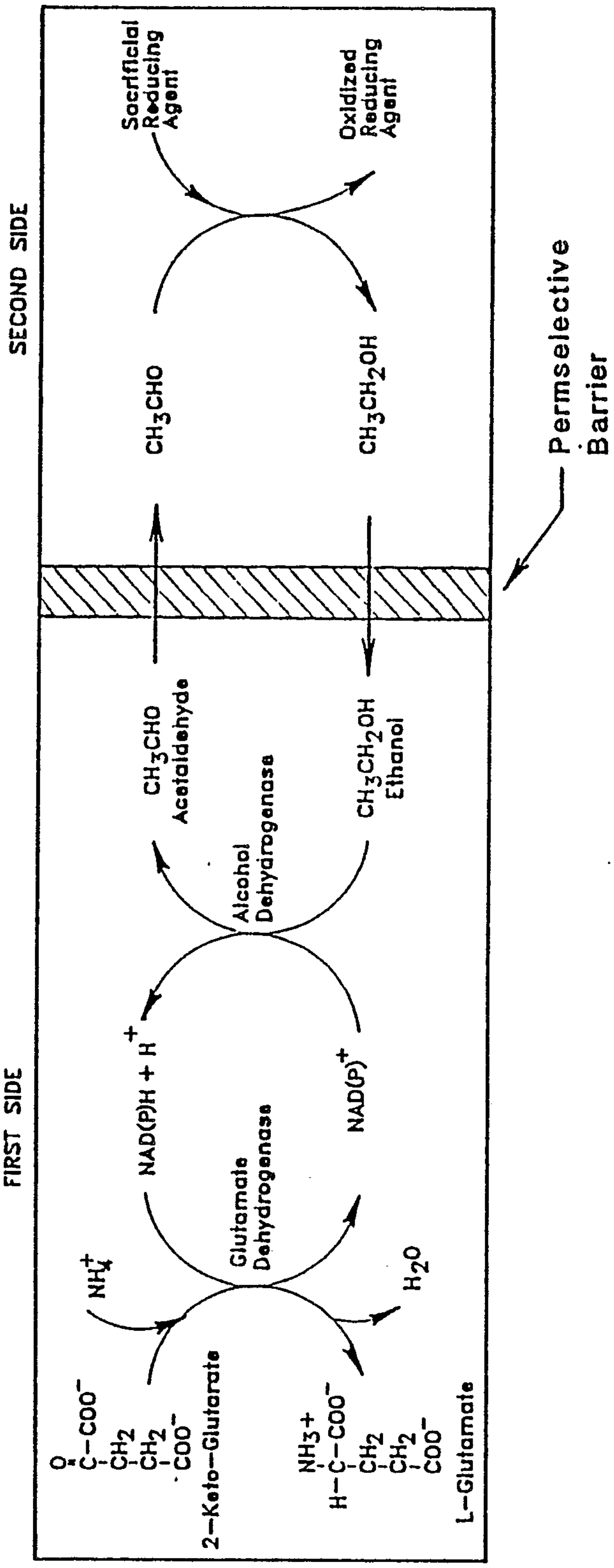
SECOND SIDE
FIRST SIDE
Sacrificial Reducing Agent
Oxidized Reducing Agent
$CH_3CHO$
$CH_3CH_2OH$
Permselective Barrier
$CH_3CHO$ Acetaldehyde
$CH_3CH_2OH$ Ethanol
Alcohol Dehydrogenase
NAD(P)H + $H^+$
$NAD(P)^+$
Glutamate Dehydrogenase
$NH_4^+$
$H_2O$
2-Keto-Glutarate
L-Glutamate

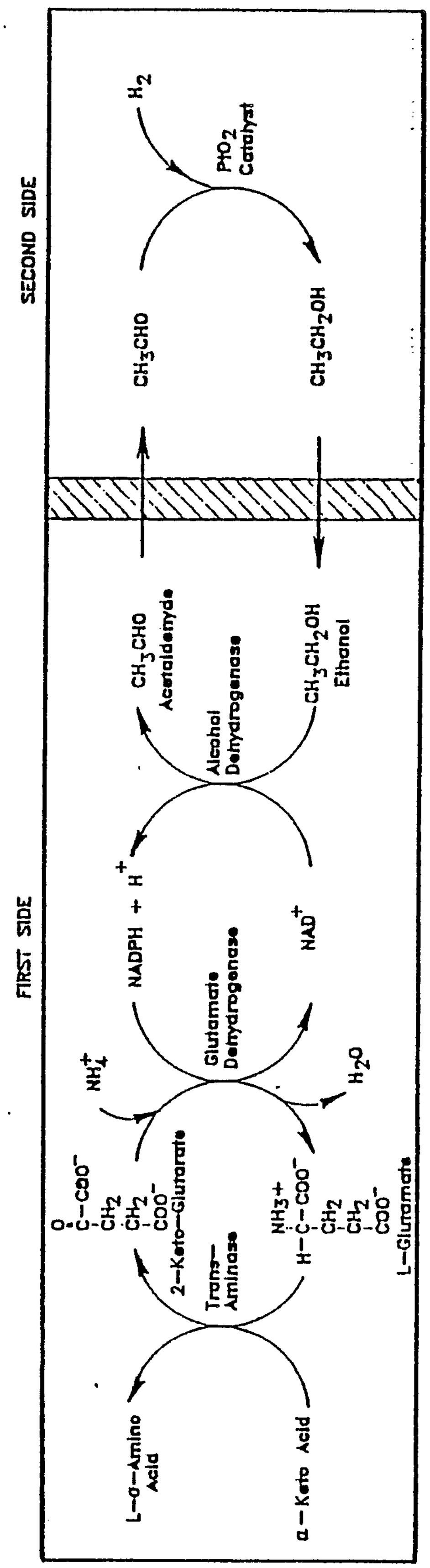
FIRST SIDE
SECOND SIDE
L-α-Amino Acid
α-Keto Acid
Trans-Aminase
2-Keto-Glutarate
L-Glutamate
$NH_4^+$
$H_2O$
Glutamate Dehydrogenase
NADPH + $H^+$
$NAD^+$
Alcohol Dehydrogenase
$CH_3CHO$ Acetaldehyde
$CH_3CH_2OH$ Ethanol
$CH_3CHO$
$CH_3CH_2OH$
$H_2$
$PtO_2$ Catalyst

FIG.15.

FIG.16.

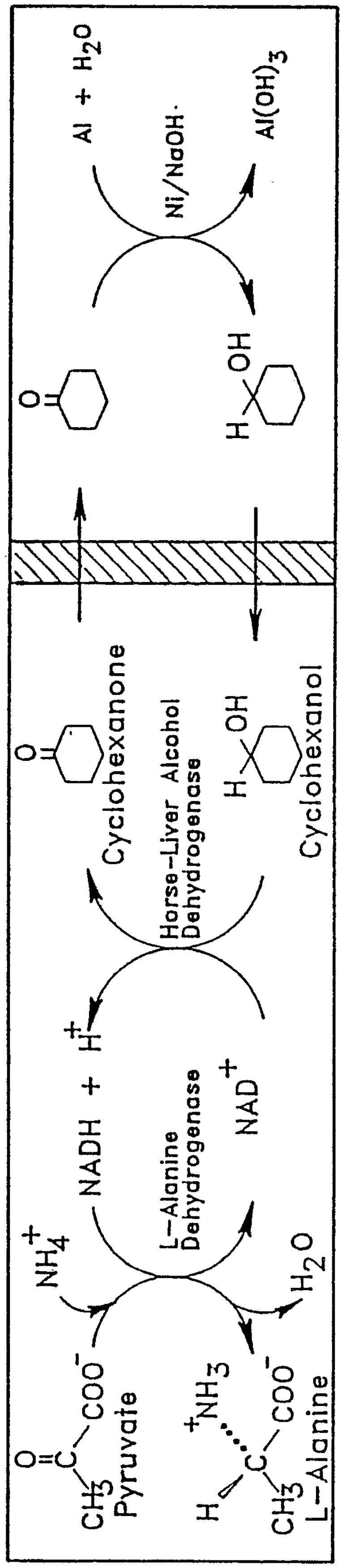
FIRST SIDE
SECOND SIDE
Pyruvate
L-Alanine
$NH_4^+$
$H_2O$
L-Alanine Dehydrogenase
NADH + $H^+$
$NAD^+$
Horse-Liver Alcohol Dehydrogenase
Cyclohexanone
Cyclohexanol
Al + $H_2O$
Ni/NaOH
$Al(OH)_3$

FIG. 17.

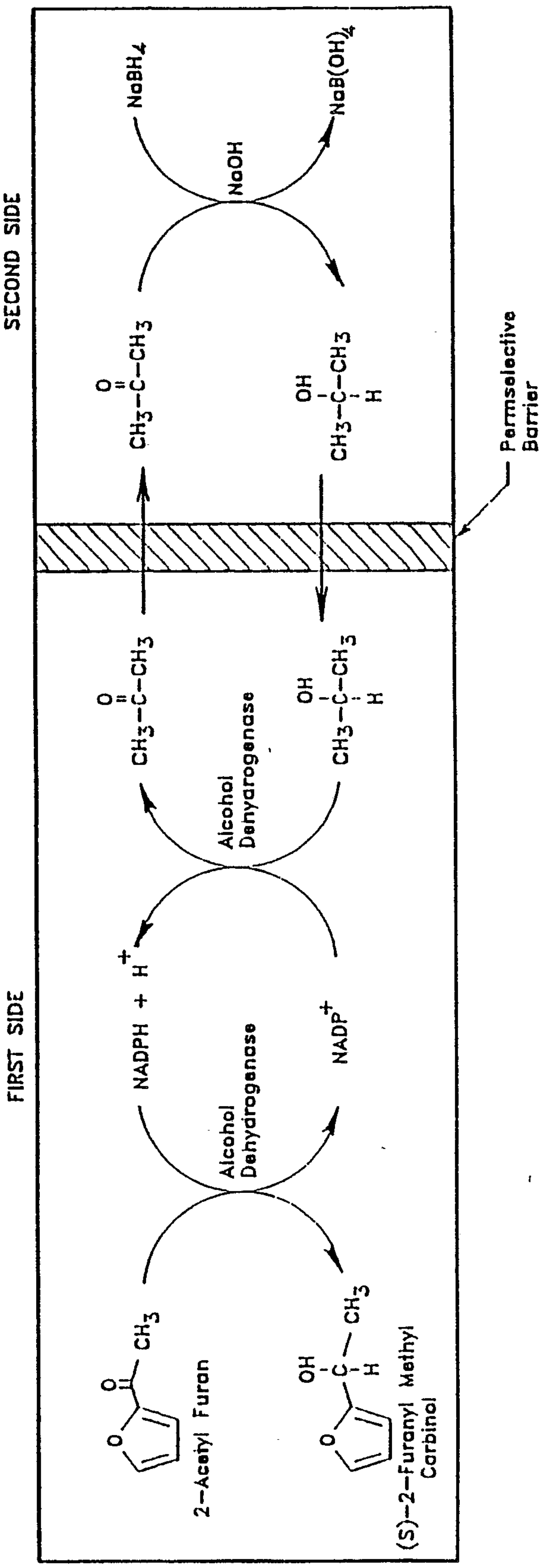


2-Acetyl Furan

(S)-2-Furanyl Methyl Carbinol

# FIG. 18.

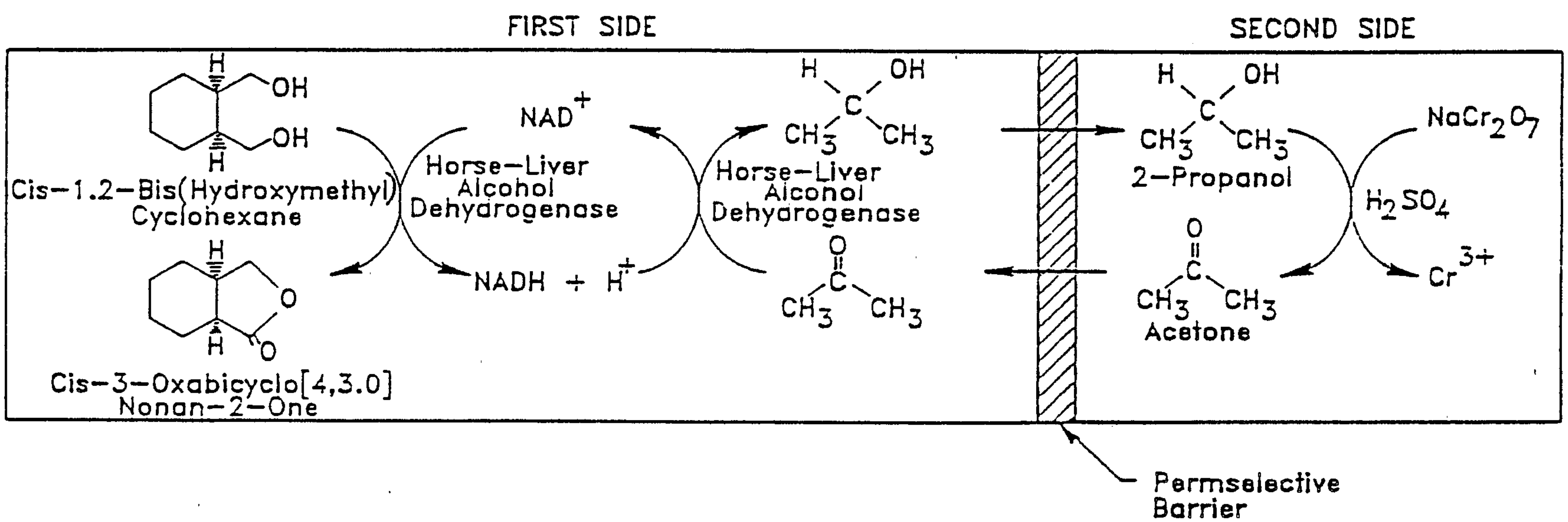
FIRST SIDE
SECOND SIDE
Cis-1.2-Bis(Hydroxymethyl) Cyclohexane
Cis-3-Oxabicyclo[4,3.0] Nonan-2-One
Horse-Liver Alcohol Dehydrogenase
$NAD^+$
NADH + $H^+$
Horse-Liver Alcohol Dehydrogenase
2-Propanol
Acetone
$NaCr_2O_7$
$H_2SO_4$
$Cr^{3+}$
Permselective Barrier

FIG. 19.

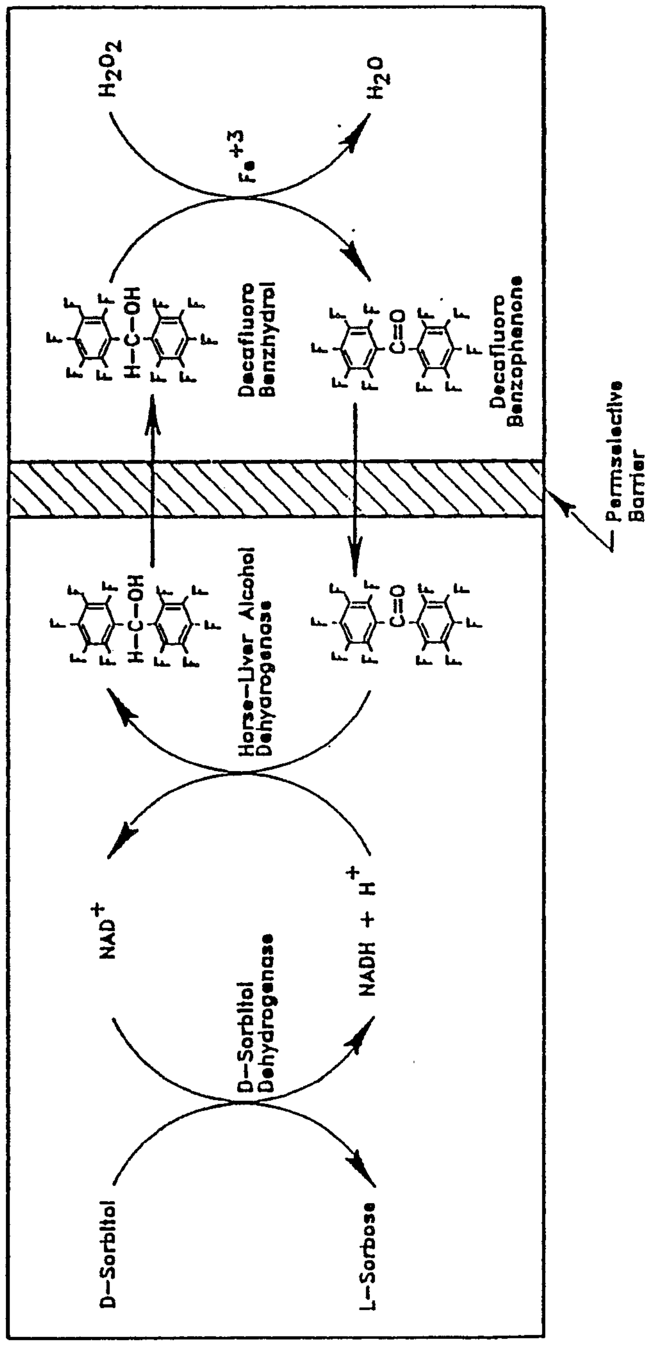
D-Sorbitol
L-Sorbose
D-Sorbitol Dehydrogenase
NAD+
NADH + H+
Horse-Liver Alcohol Dehydrogenase
Decafluoro Benzhydrol
Decafluoro Benzophenone
H2O2
H2O
Fe+3
Permselective Barrier

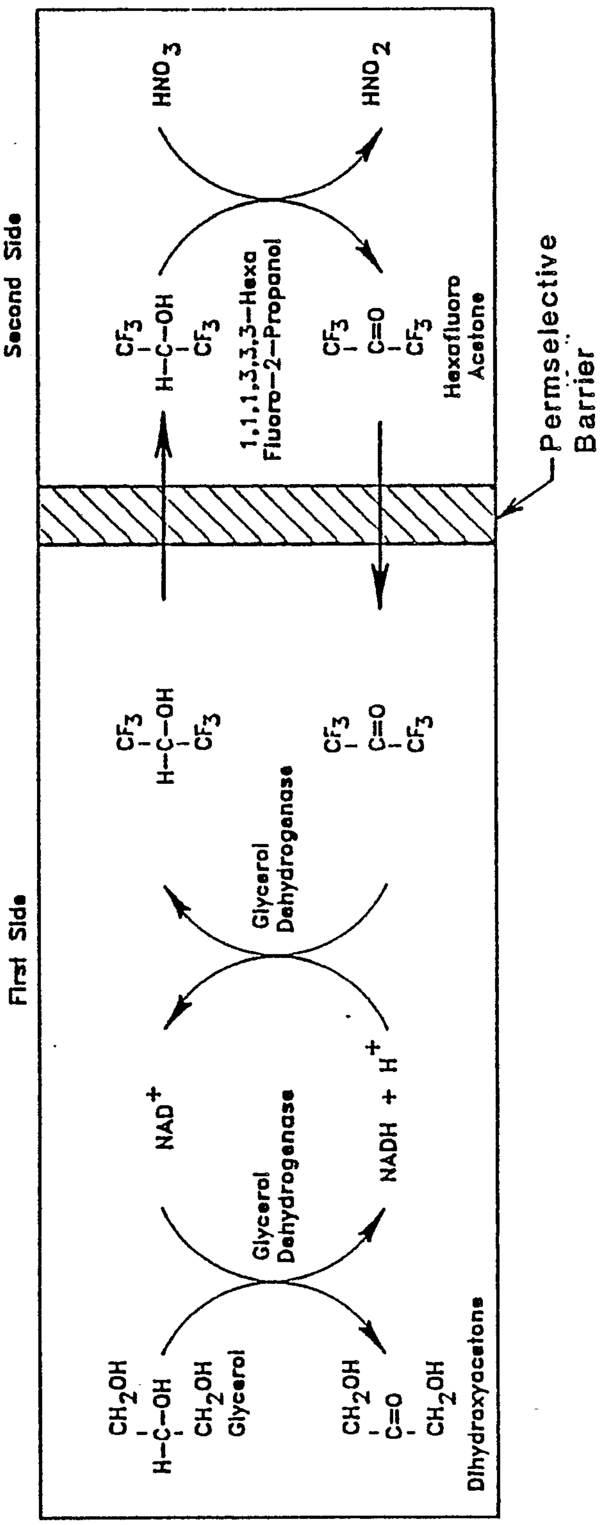
First Side
Second Side
Glycerol
Dihydroxyacetone
Glycerol Dehydrogenase
NAD+
NADH + H+
Glycerol Dehydrogenase
1,1,1,3,3,3-Hexa Fluoro-2-Propanol
Hexafluoro Acetone
HNO3
HNO2
Permselective Barrier

FIG. 20.

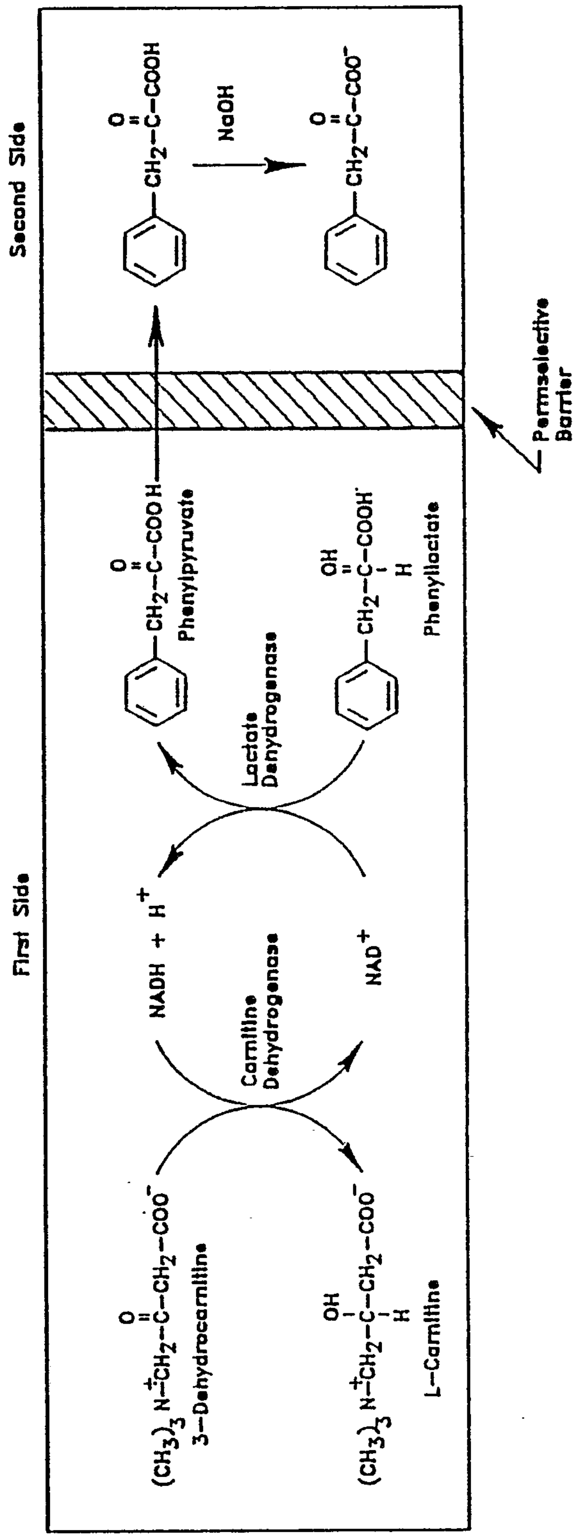
Second Side
First Side
NaOH
Phenylpyruvate
Phenyllactate
Lactate Dehydrogenase
NADH + H+
NAD+
Carnitine Dehydrogenase
3-Dehydrocarnitine
L-Carnitine
Permselective Barrier

FIG. 21.

FIG. 22.

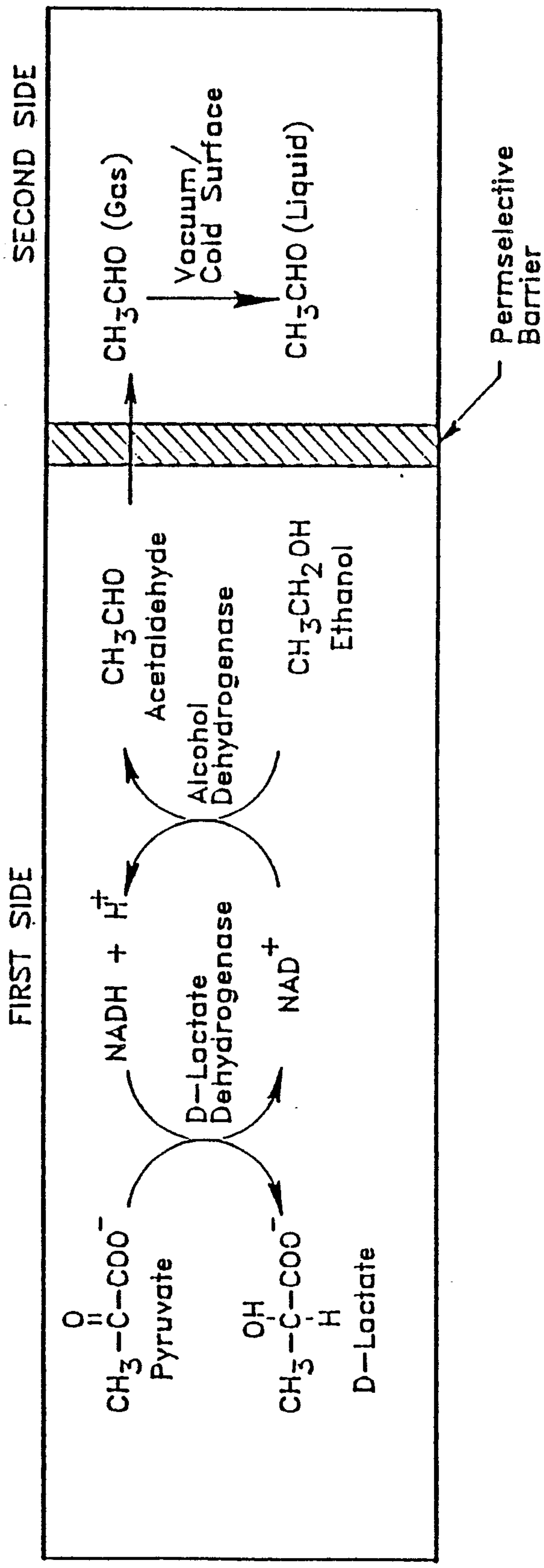
FIRST SIDE
SECOND SIDE
Pyruvate
D-Lactate
D-Lactate Dehydrogenase
NADH + H+
NAD+
Alcohol Dehydrogenase
Acetaldehyde
Ethanol
CH3CHO (Gas)
Vacuum/ Cold Surface
CH3CHO (Liquid)
Permselective Barrier

FIG. 23.

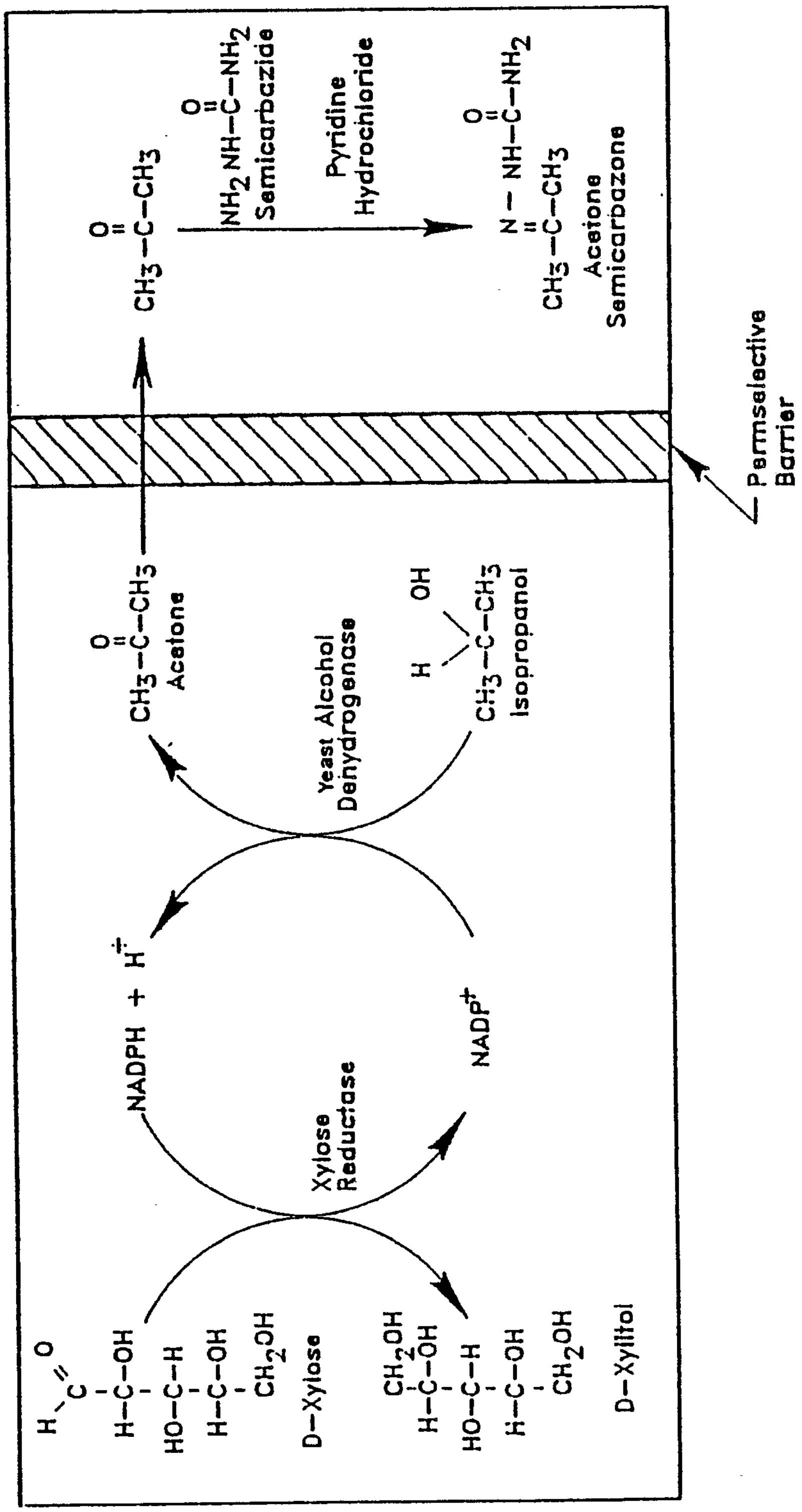

D-Xylose
D-Xylitol
Xylose Reductase
NADPH + H+
NADP+
Yeast Alcohol Dehydrogenase
Acetone
Isopropanol
Semicarbazide
Pyridine Hydrochloride
Acetone Semicarbazone
Permselective Barrier

FIG.24.

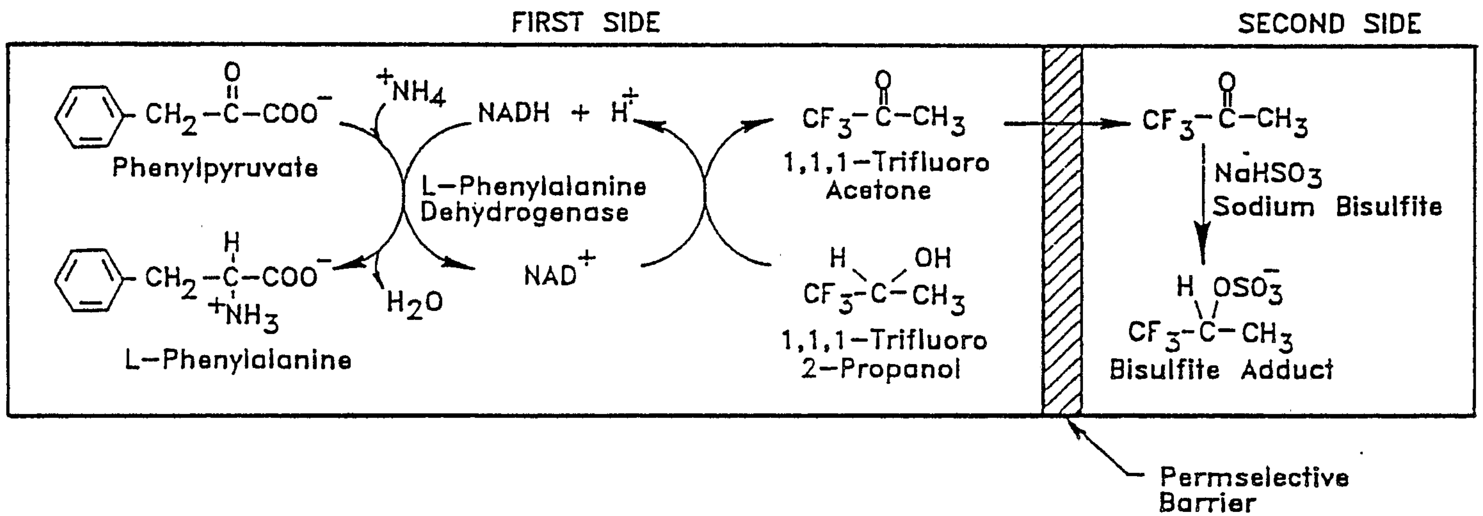
FIRST SIDE
SECOND SIDE
Phenylpyruvate
L-Phenylalanine
L-Phenylalanine
Dehydrogenase
1,1,1-Trifluoro
Acetone
1,1,1-Trifluoro
2-Propanol
Sodium Bisulfite
Bisulfite Adduct
Permselective
Barrier

FIG. 25.

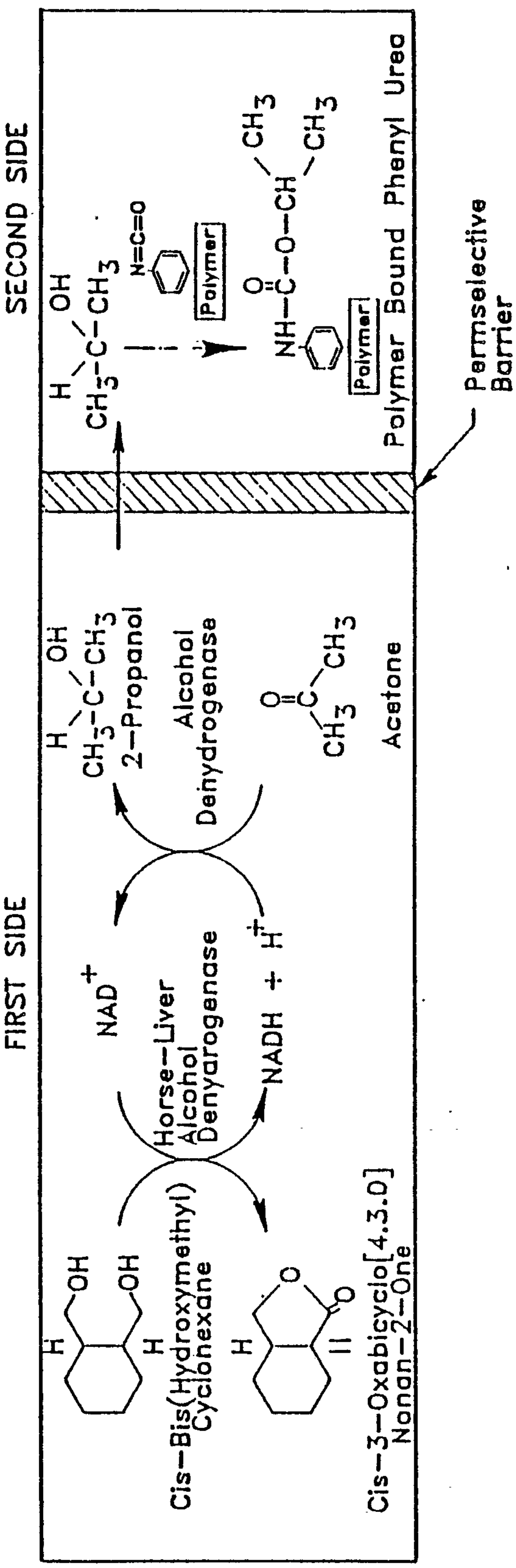
FIRST SIDE
SECOND SIDE
Cis-Bis(Hydroxymethyl) Cyclonexane
Horse-Liver Alcohol Denyarogenase
NAD+
NADH + H+
Alcohol Denydrogenase
2-Propanol
Acetone
Cis-3-Oxabicyclo[4.3.0] Nonan-2-One
Polymer Bound Phenyl Urea
Permselective Barrier

FIG. 26.

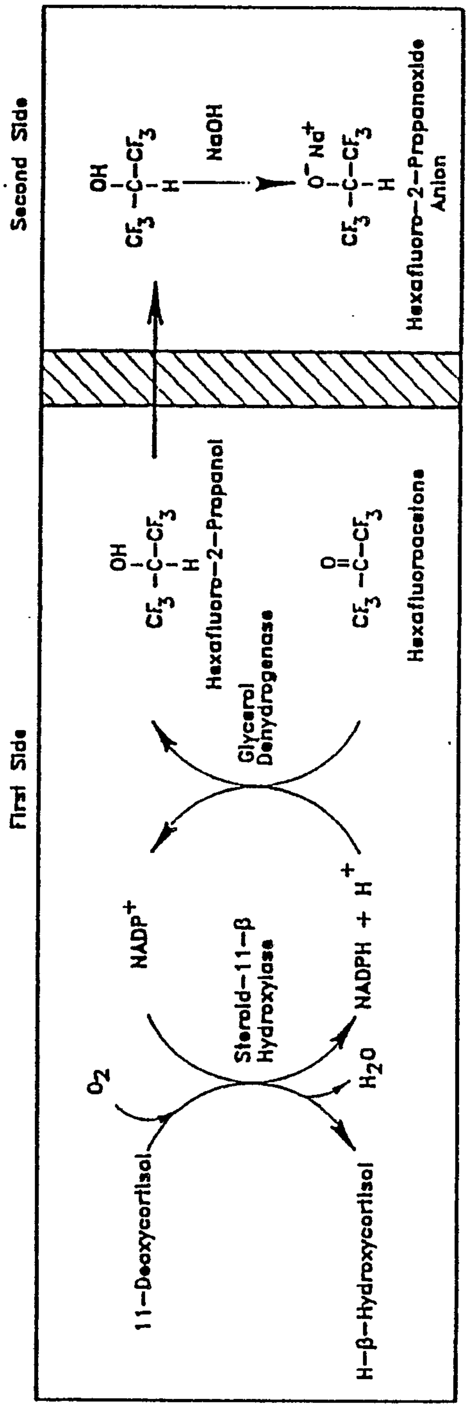
First Side
Second Side
11-Deoxycortisol
H-β-Hydroxycortisol
Steroid-11-β Hydroxylase
NADP+
NADPH + H+
Glycerol Dehydrogenase
Hexafluoro-2-Propanol
Hexafluoroacetone
NaOH
Hexafluoro-2-Propanoxide Anion

FIG. 27.

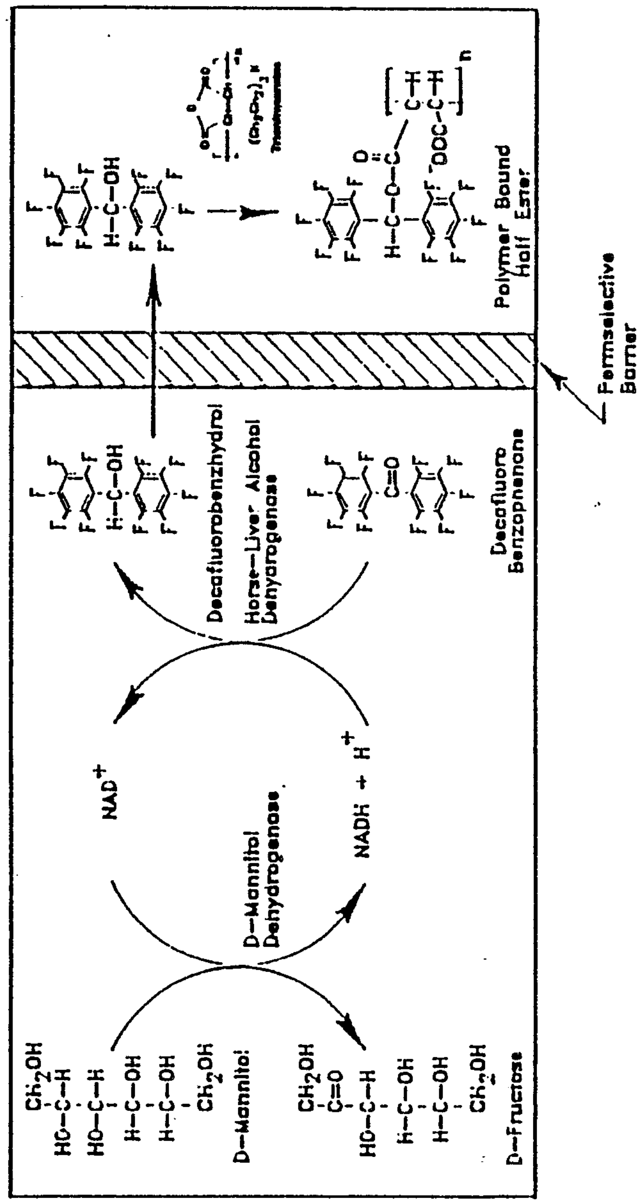
D-Mannitol
D-Fructose
D-Mannitol Dehydrogenase
NAD$^+$
NADH + H$^+$
Decafluorobenzhydrol
Horse-Liver Alcohol Dehydrogenase
Decafluoro Benzophenone
Polymer Bound Half Ester
Permselective Barrier

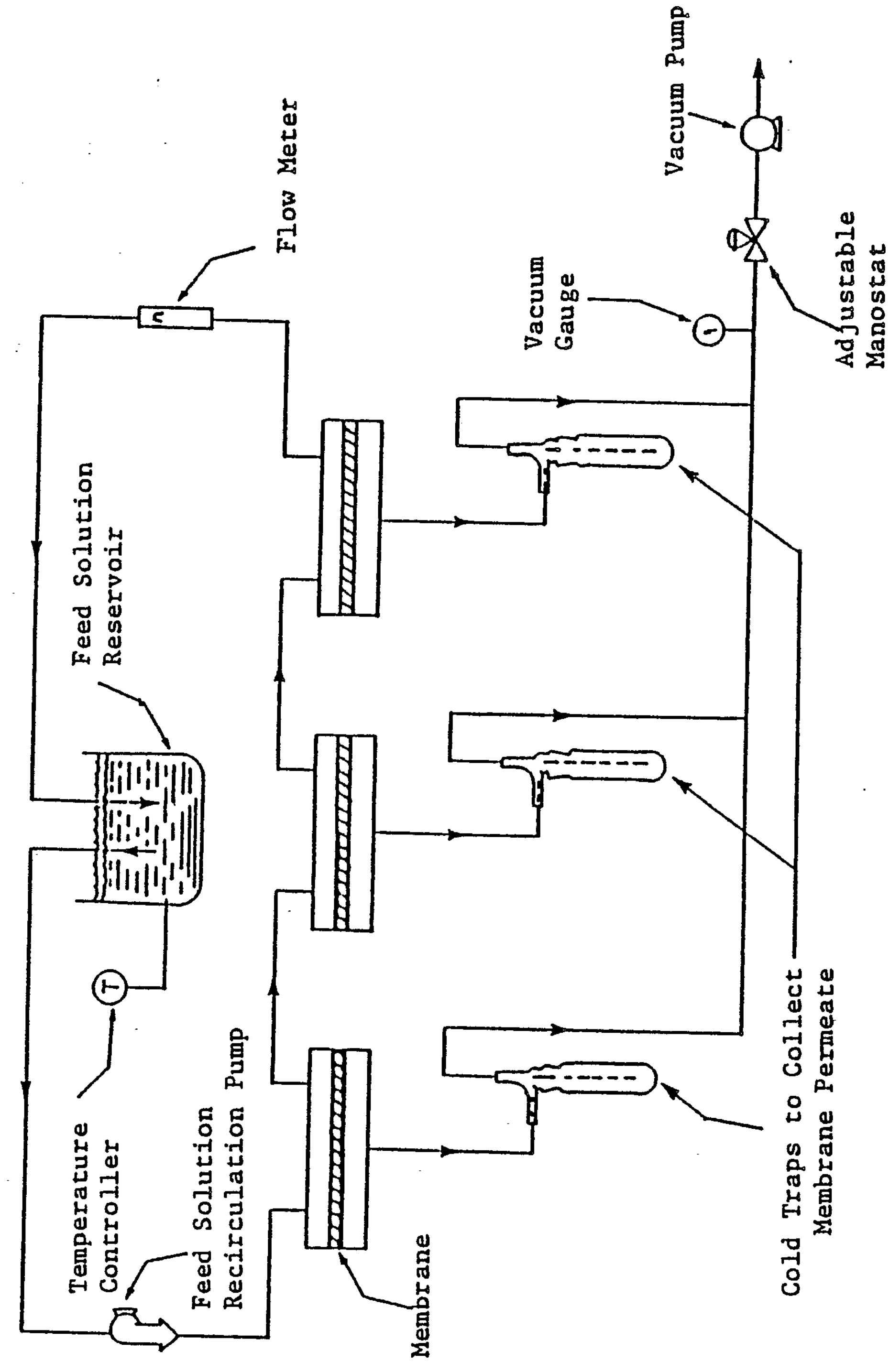

Flow Meter
Feed Solution Reservoir
Temperature Controller
Feed Solution Recirculation Pump
Membrane
Vacuum Gauge
Adjustable Manostat
Vacuum Pump
Cold Traps to Collect Membrane Permeate


FIG.28.

FIG. 29.

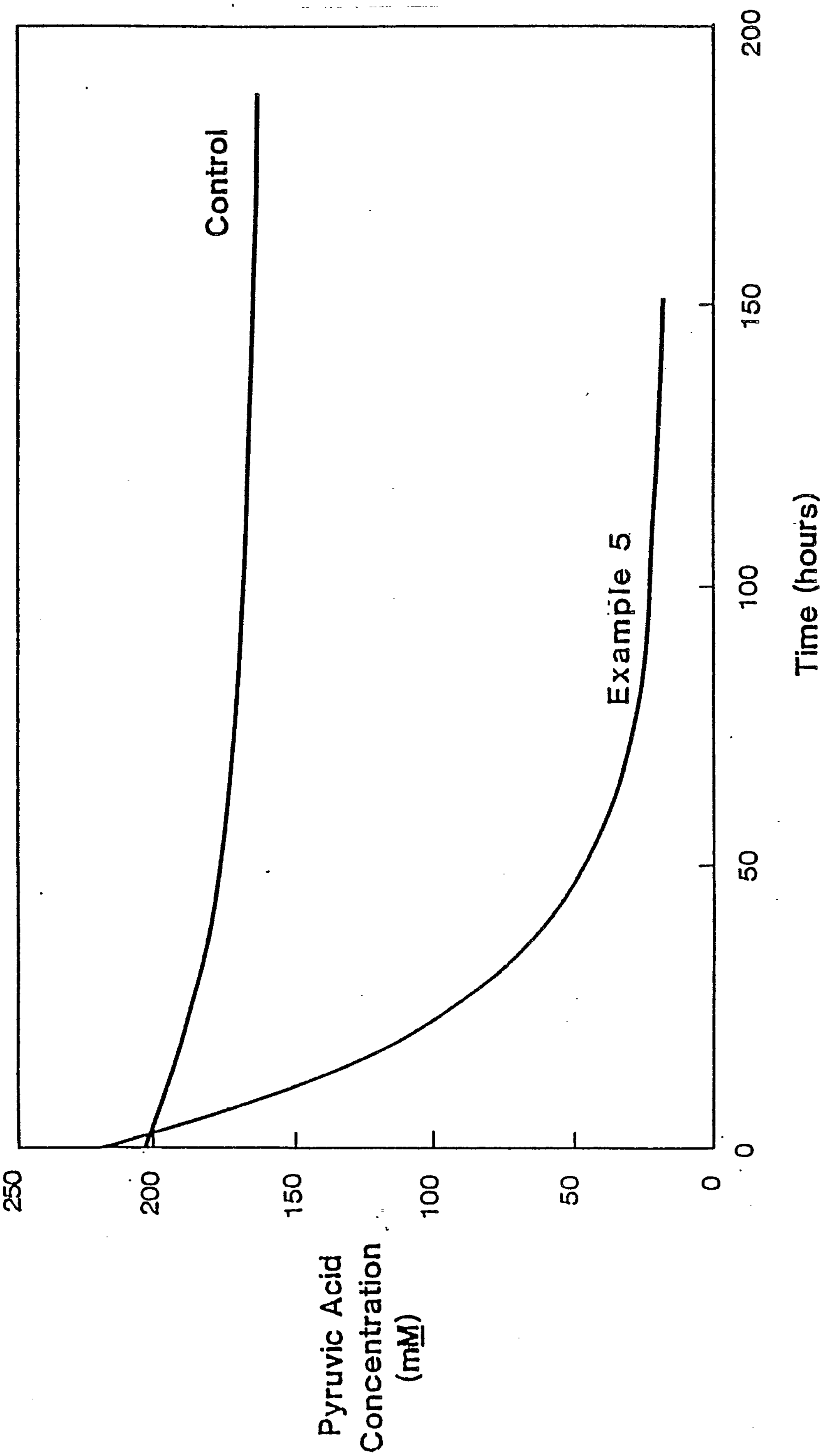
Control
Example 5
Pyruvic Acid Concentration (mM)
250
200
150
100
50
0
Time (hours)
0
50
100
150
200

FIG. 30.

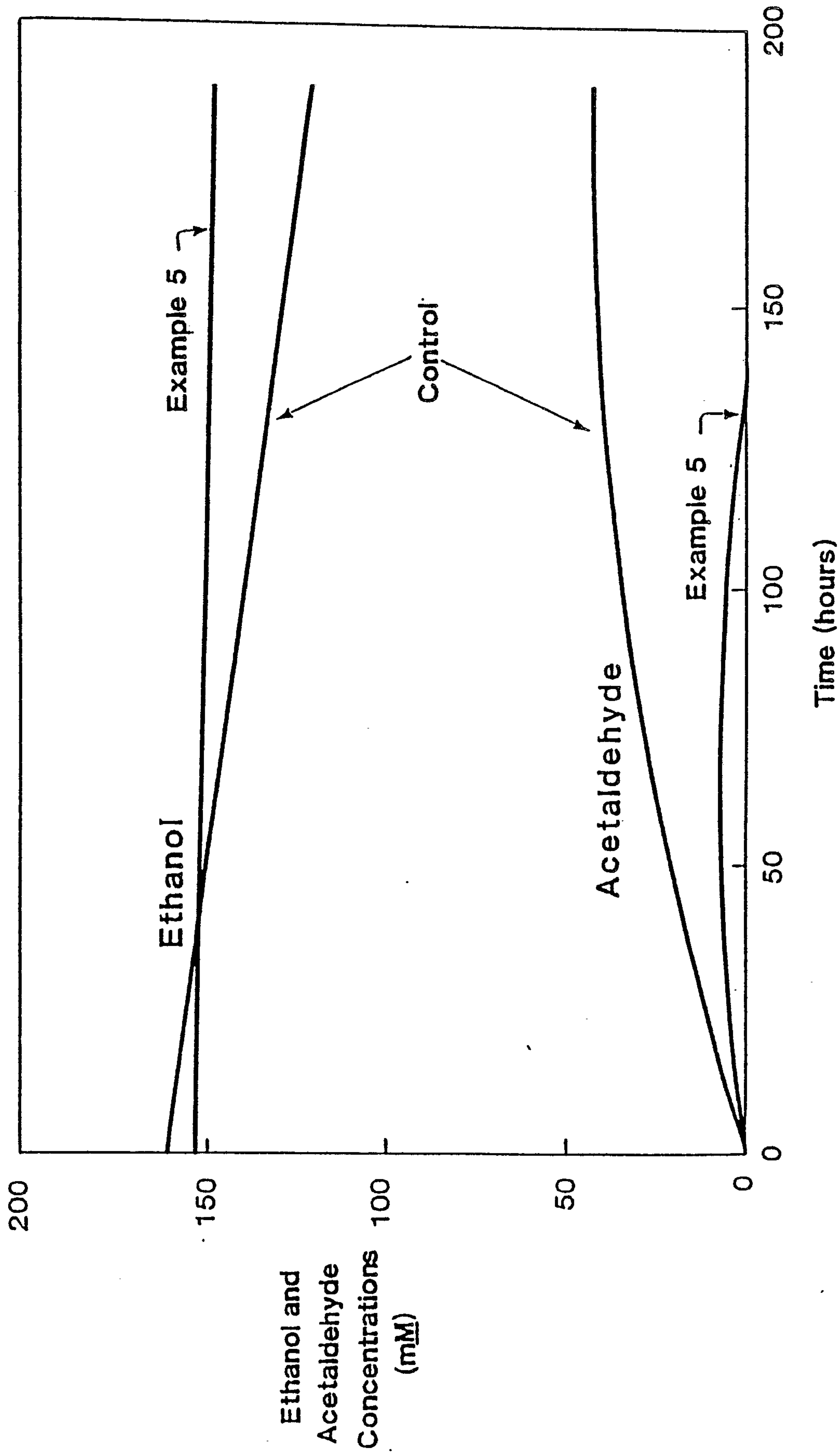
Ethanol
Acetaldehyde
Example 5
Control
Example 5
0
50
100
150
200
Time (hours)
0
50
100
150
200
Ethanol and Acetaldehyde Concentrations (mM)

FIG. 31.

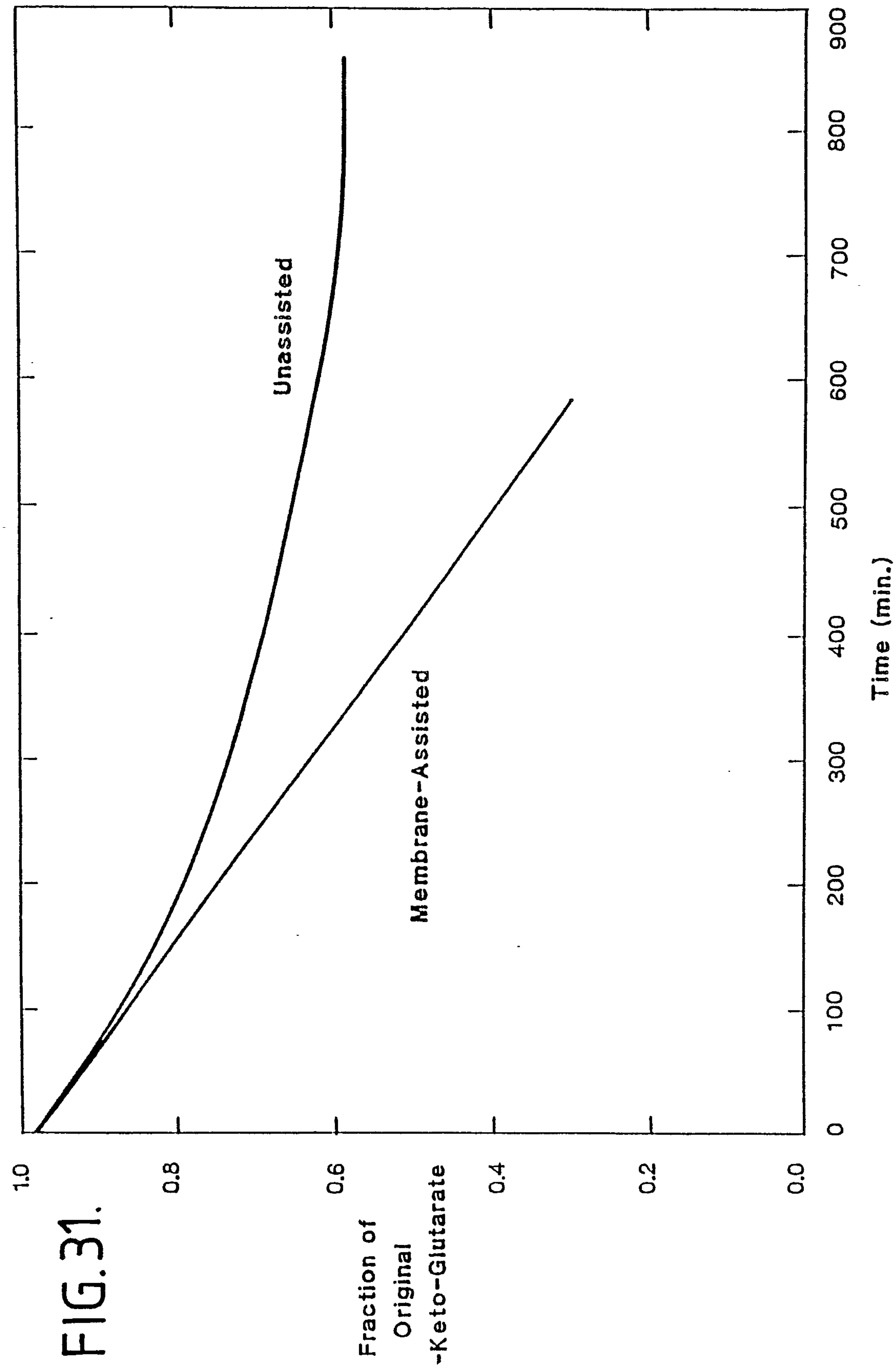
Fraction of
Original
-Keto-Glutarate
1.0
0.8
0.6
0.4
0.2
0.0
0
100
200
300
400
500
600
700
800
900
Time (min.)
Unassisted
Membrane-Assisted

FIG. 32.

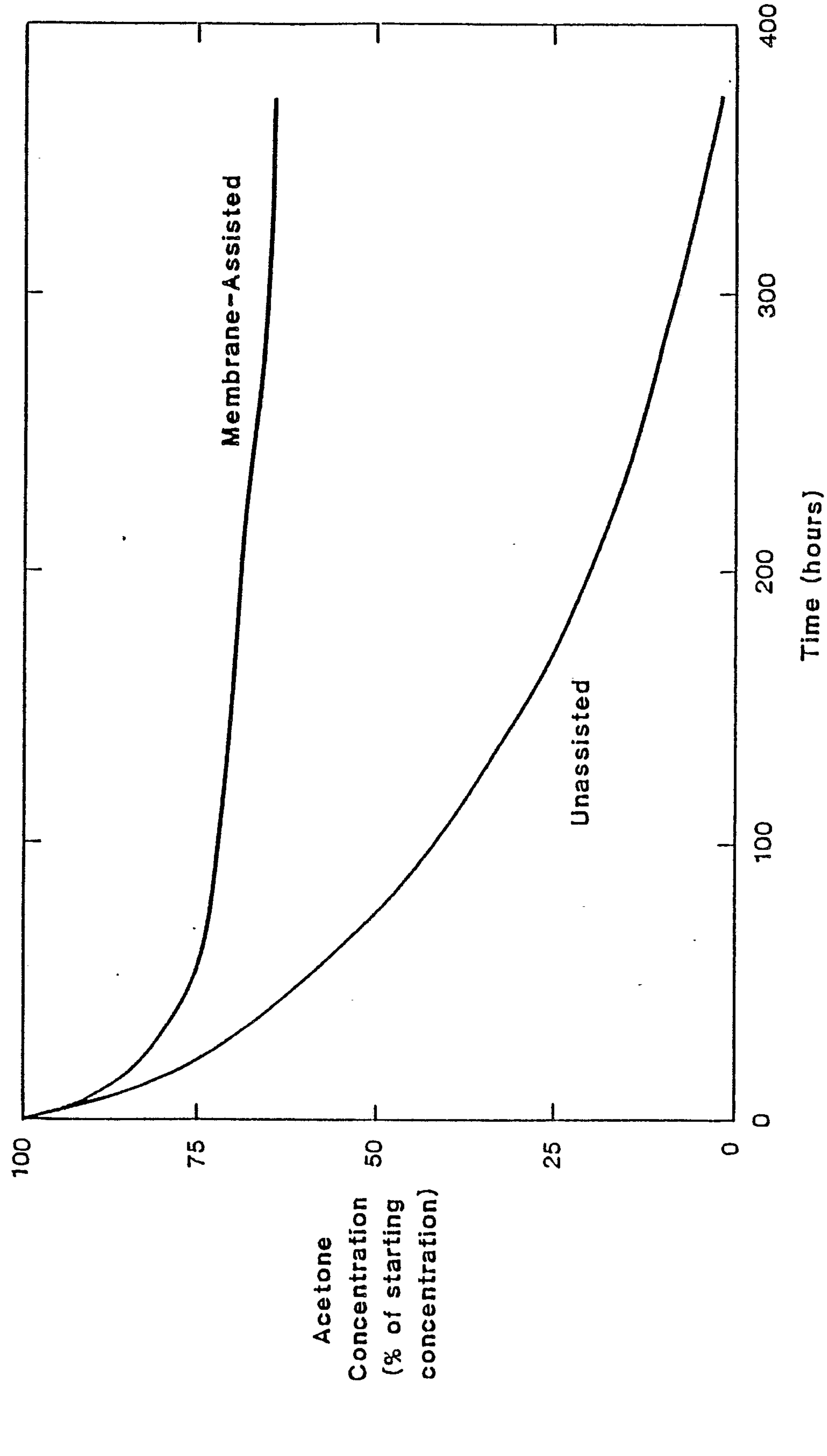
Acetone
Concentration
(% of starting
concentration)
100
75
50
25
0
Membrane-Assisted
Unassisted
0
100
200
300
400
Time (hours)

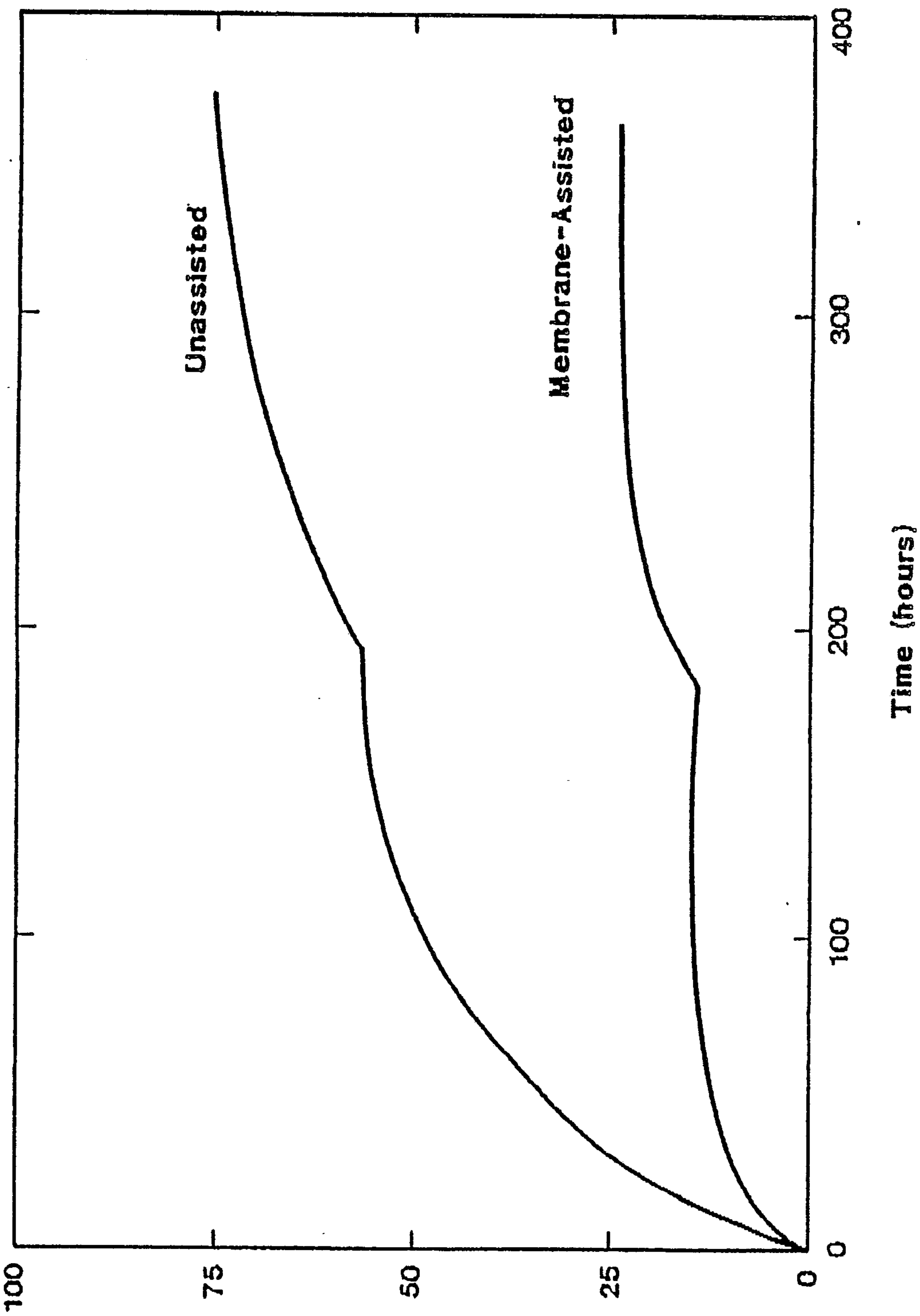
Unassisted
Membrane-Assisted
100
75
50
25
0
0
100
200
300
400
Time (hours)
Isopropanol Conversion (%)

FIG.33.

FIG. 34.

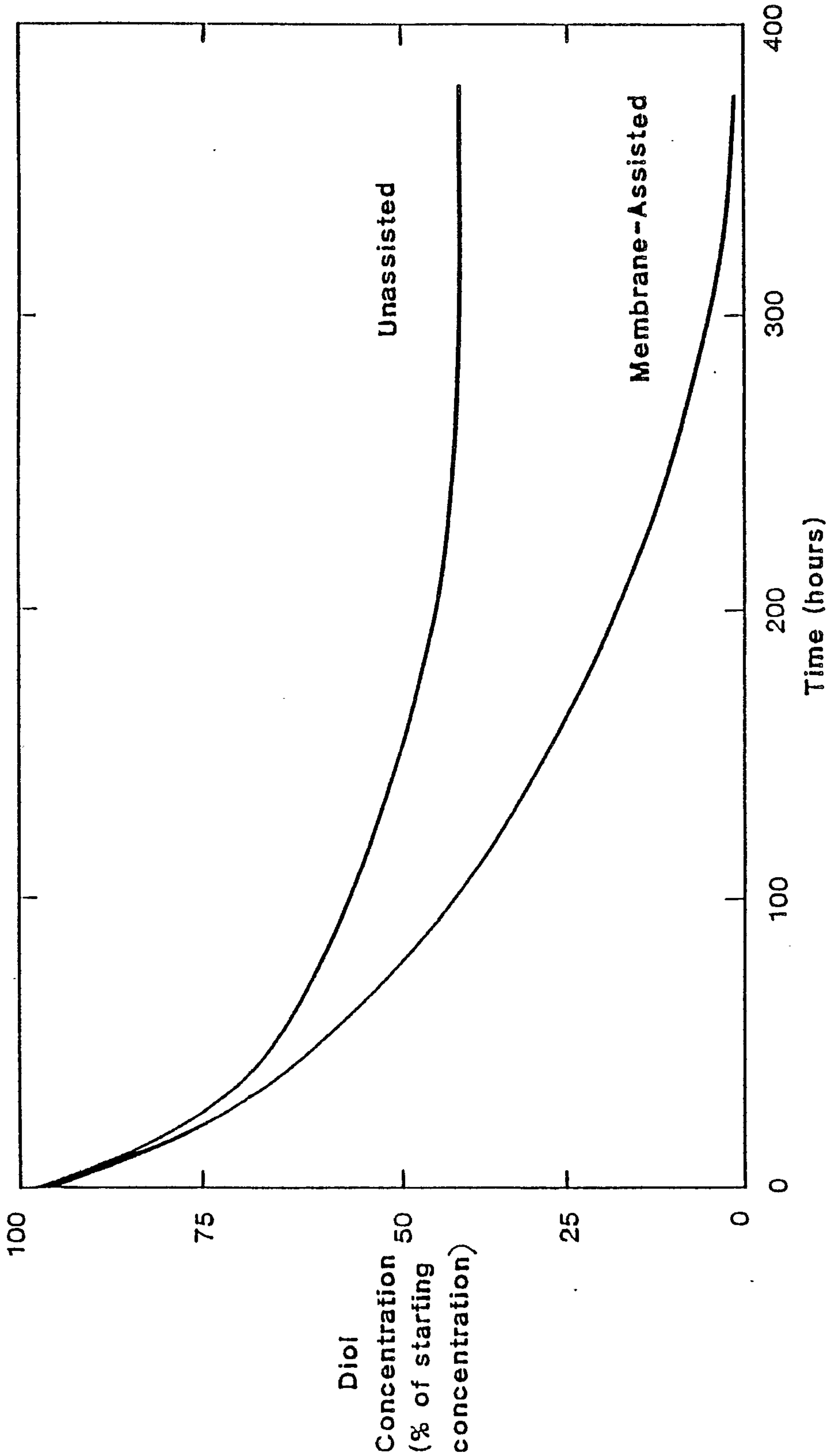
Diol Concentration (% of starting concentration)
100
75
50
25
0
Time (hours)
0
100
200
300
400
Unassisted
Membrane-Assisted

FIG.35.

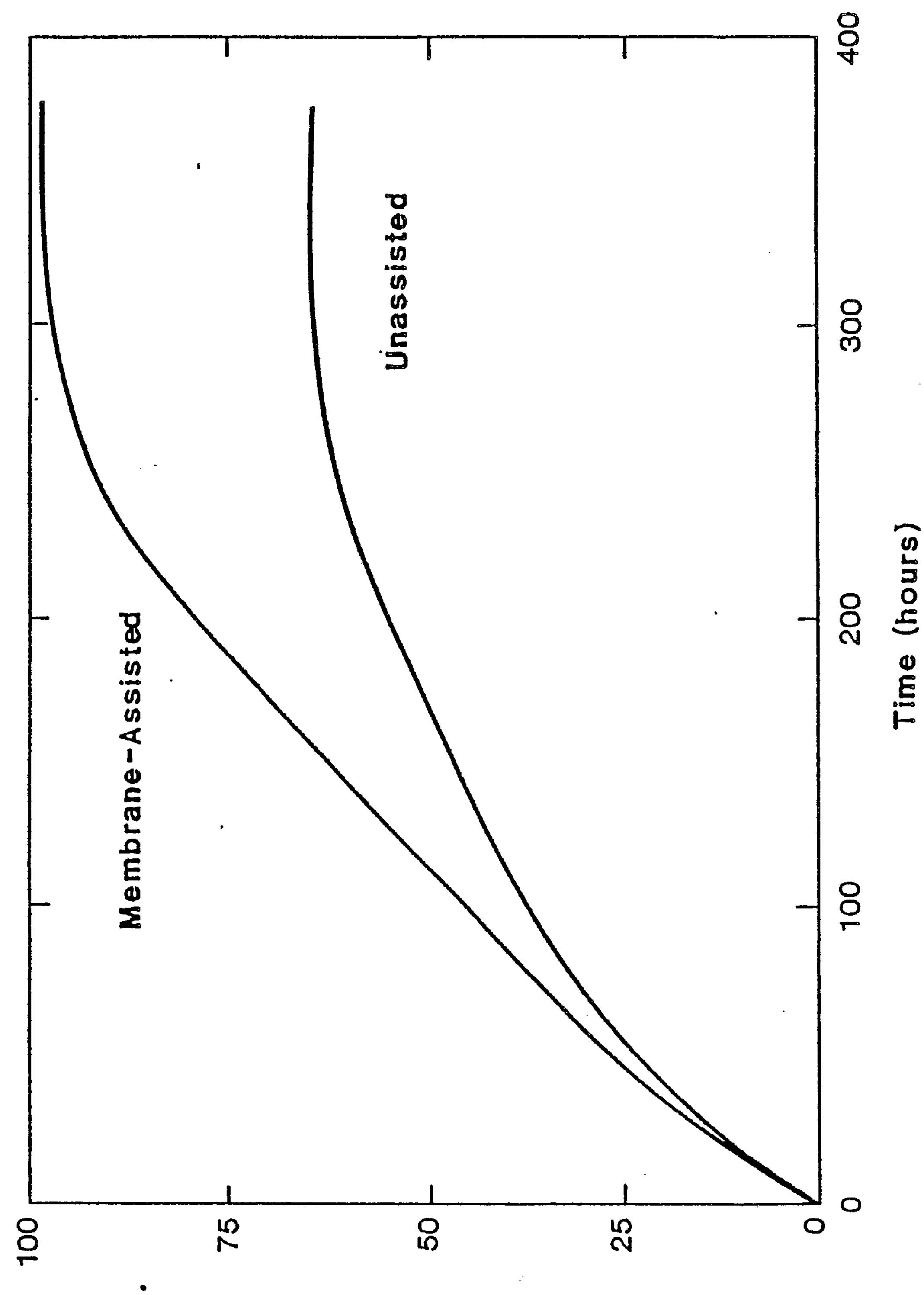
Lactone Conversion (%)
100
75
50
25
0
0
100
200
300
400
Time (hours)
Membrane-Assisted
Unassisted

FIG. 36.

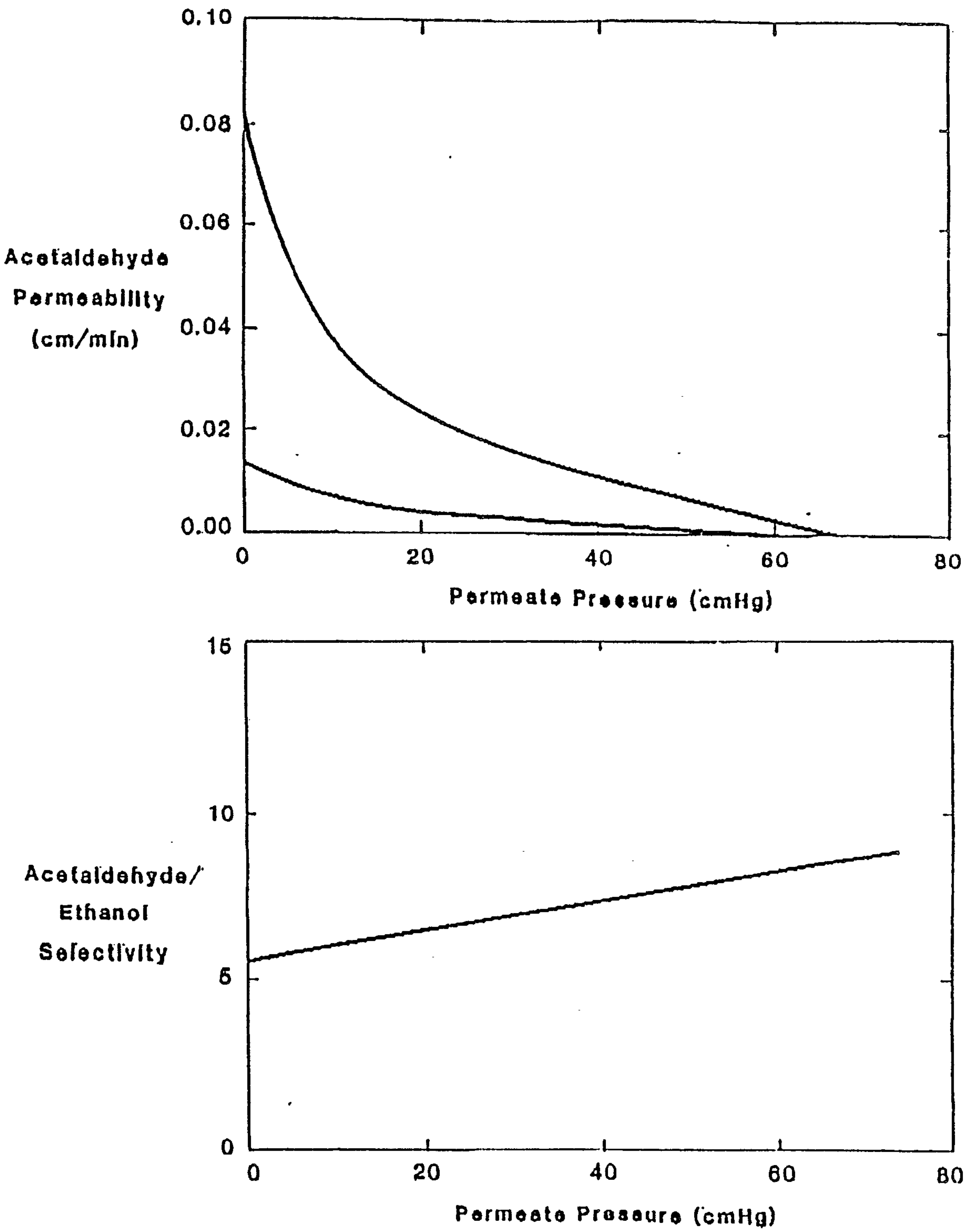
0.10
0.08
0.06
0.04
0.02
0.00
Acetaldehyde
Permeability
(cm/min)
0
20
40
60
80
Permeate Pressure (cmHg)
15
10
5
0
Acetaldehyde/
Ethanol
Selectivity
0
20
40
60
80
Permeate Pressure (cmHg)